# EUROPEAN PATENT APPLICATION

(11) **EP 1 559 428 A1**
(43) Date of publication of application: **03.08.2005**
(21) Application number: 03810601.9
(22) Date of filing: 05.11.2003
(51) Int. Cl.: A61K 31/4468, A61K 31/454, A61K 31/4545, A61P 1/04, A61P 1/14, A61P 3/10, A61P 9/06, A61P 9/10, A61P 9/12, A61P 11/00, A61P 11/06, A61P 13/12, A61P 15/12, A61P 19/02, A61P 25/04, A61P 29/00, A61P 37/04, A61P 43/00, C07D 211/58, C07D 211/66

(54) **RECEPTOR REGULATOR**

(30) Priority: 06.11.2002 JP 2002322534
(71) Applicant: Takeda Pharmaceutical Company Limited, Osaka-shi, Osaka 541-0045 (JP)
(72) Inventor: KAJINO, M.#c/o Takeda Pharmaceutical Company ltd., Osaka-shi, Osaka (JP); HINUMA, S. c/o Takeda Pharmaceutical Company ltd., Ibaraki (JP); TARUI, N. c/o Takeda Pharmaceutical Company ltd., Osaka-shi, Osaka (JP); YAMASHITA, T. Takeda Pharmaceutical Company ltd., Osaka-shi, Osaka (JP); NAKAYAMA, Yutaka, Chuo-ku, Sapporo-shi, Hokkaido (JP)
(74) Representative: Rickard, Timothy Mark Adrian
(86) International application number: PCT/JP2003/014101
(87) International publication number: WO 2004/041282

(57) **Abstract**

A compound having a partial structure represented by the formula (A) 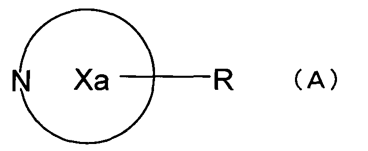 (wherein ring Xa represents a nitrogen-containing ring and R represents optionally substituted amino) or a salt thereof. The compound or salt is highly effective in regulating neuromedin U receptors and is useful as a preventive/therapeutic agent for hypertension, etc.

SEQUENCE LISTING
<110> TAKEDA PHARMACEUTICAL COMPANY LIMITED
<120> RECEPTOR REGULATOR
<130> 3116WOOP
<150> JP 2002-322534
<151> 2002-11-06
<160> 3
<210> 1
<211> 403
<212> PRT
<213> Homo sapiens
<400> 1
Met Ala Cys Asn Gly Ser Ala Ala Arg Gly His Phe Asp Pro Glu Asp
1               5                   10                  15
Leu Asn Leu Thr Asp Glu Ala Leu Arg Leu Lys Tyr Leu Gly Pro Gln
            20                  25                  30
Gln Thr Glu Leu Phe Met Pro Ile Cys Ala Thr Tyr Leu Leu Ile Phe
        35                  40                  45
Val Val Gly Ala Val Gly Asn Gly Leu Thr Cys Leu Val Ile Leu Arg
    50                  55                  60
His Lys Ala Met Arg Thr Pro Thr Asn Tyr Tyr Leu Phe Ser Leu Ala
65                  70                  75                  80
Val Ser Asp Leu Leu Val Leu Leu Val Gly Leu Pro Leu Glu Leu Tyr
                85                  90                  95
Glu Met Trp His Asn Tyr Pro Phe Leu Leu Gly Val Gly Gly Cys Tyr
            100                 105                 110
Phe Arg Thr Leu Leu Phe Glu Met Val Cys Leu Ala Ser Val Leu Asn
        115                 120                 125
Val Thr Ala Leu Ser Val Glu Arg Tyr Val Ala Val Val His Pro Leu
    130                 135                 140
Gln Ala Arg Ser Met Val Thr Arg Ala His Val Arg Arg Val Leu Gly
145                 150                 155                 160
Ala Val Trp Gly Leu Ala Met Leu Cys Ser Leu Pro Asn Thr Ser Leu
                165                 170                 175
His Gly Ile Arg Gln Leu His Val Pro Cys Arg Gly Pro Val Pro Asp
            180                 185                 190
Ser Ala Val Cys Met Leu Val Arg Pro Arg Ala Leu Tyr Asn Met Val
        195                 200                 205
Val Gln Thr Thr Ala Leu Leu Phe Phe Cys Leu Pro Met Ala Ile Met
    210                 215                 220
Ser Val Leu Tyr Leu Leu Ile Gly Leu Arg Leu Arg Arg Glu Arg Leu
225                 230                 235                 240
Leu Leu Met Gln Glu Ala Lys Gly Arg Gly Ser Ala Ala Ala Arg Ser
                245                 250                 255
Arg Tyr Thr Cys Arg Leu Gln Gln His Asp Arg Gly Arg Arg Gln Val
            260                 265                 270
Thr Lys Met Leu Phe Val Leu Val Val Val Phe Gly Ile Cys Trp Ala
        275                 280                 285
Pro Phe His Ala Asp Arg Val Met Trp Ser Val Val Ser Gln Trp Thr
    290                 295                 300
Asp Gly Leu His Leu Ala Phe Gln His Val His Val Ile Ser Gly Ile
305                 310                 315                 320
Phe Phe Tyr Leu Gly Ser Ala Ala Asn Pro Val Leu Tyr Ser Leu Met
                325                 330                 335
Ser Ser Arg Phe Arg Glu Thr Phe Gln Glu Ala Leu Cys Leu Gly Ala
            340                 345                 350
Cys Cys His Arg Leu Arg Pro Arg His Ser Ser His Ser Leu Ser Arg
        355                 360                 365
Met Thr Thr Gly Ser Thr Leu Cys Asp Val Gly Ser Leu Gly Ser Trp
    370                 375                 380
Val His Pro Leu Ala Gly Asn Asp Gly Pro Glu Ala Gln Gln Glu Thr
385                 390                 395                 400
Asp Pro Ser
        403
<210> 2
<211> 405
<212> PRT
<213> Mus spp.

<400> 2
Met Val Cys Asn Ile Ser Glu Phe Lys Trp Pro Tyr Gln Pro Glu Asp
                5                   10                  15
Leu Asn Leu Thr Asp Glu Ala Leu Arg Leu Lys Tyr Leu Gly Pro Gln
            20                  25                  30
Gln Met Lys Gln Phe Val Pro Ile Cys Val Thr Tyr Leu Leu Ile Phe
        35                  40                  45
Val Val Gly Thr Leu Gly Asn Gly Leu Thr Cys Thr Val Ile Leu Arg
    50                  55                  60
Asn Lys Thr Met Arg Thr Pro Thr Asn Phe Tyr Leu Phe Ser Leu Ala
65                  70                  75                  80
Val Ser Asp Met Leu Val Leu Leu Val Gly Leu Pro Leu Glu Leu Tyr
                85                  90                  95
Glu Met Gln Gln Asn Tyr Pro Phe Gln Leu Gly Ala Ser Ala Cys Tyr
            100                 105                 110
Phe Arg Ile Leu Leu Leu Glu Thr Val Cys Leu Ala Ser Val Leu Asn
        115                 120                 125
Val Thr Ala Leu Ser Val Glu Arg Tyr Val Ala Val Val Arg Pro Leu
    130                 135                 140
Gln Ala Lys Ser Val Met Thr Arg Ala His Val Arg Arg Met Val Gly
145                 150                 155                 160
Ala Ile Trp Val Leu Ala Thr Leu Phe Ser Leu Pro Asn Thr Ser Leu
                165                 170                 175
His Gly Leu Ser Gln Leu Thr Val Pro Cys Arg Gly Pro Val Pro Asp
            180                 185                 190
Ser Ala Ile Cys Ser Leu Val Gly Pro Met Asp Phe Tyr Lys Leu Val
        195                 200                 205
Val Leu Thr Thr Ala Leu Leu Phe Phe Cys Leu Pro Met Val Thr Ile
    210                 215                 220
Ser Val Leu Tyr Leu Leu Ile Gly Leu Arg Leu Arg Arg Glu Arg Met
225                 230                 235                 240
Leu Leu Gln Val Glu Val Lys Gly Arg Lys Thr Ala Ala Thr Gln Glu
                245                 250                 255
Thr Ser His Arg Arg Ile Gln Leu Gln Asp Arg Gly Arg Arg Gln Val
            260                 265                 270
Thr Lys Met Leu Phe Ala Leu Val Val Val Phe Gly Ile Cys Trp Ala
        275                 280                 285
Pro Phe His Ala Asp Arg Ile Met Trp Ser Leu Val Tyr Gly His Ser
    290                 295                 300
Thr Glu Gly Leu His Leu Ala Tyr Gln Cys Val His Ile Ala Ser Gly
305                 310                 315                 320
Ile Phe Phe Tyr Leu Gly Ser Ala Ala Asn Pro Val Leu Tyr Ser Leu
                325                 330                 335
Met Ser Thr Arg Phe Arg Glu Thr Phe Leu Gln Ala Leu Gly Leu Gly
            340                 345                 350
Thr Gln Cys Cys His Arg Arg Gln Pro Tyr His Gly Ser His Asn His
        355                 360                 365
Ile Arg Leu Thr Thr Gly Ser Thr Leu Cys Asp Val Gly His Arg Asn
    370                 375                 380
Ser Arg Asp Glu Pro Leu Ala Val Asn Glu Asp Pro Gly Cys Gln Gln
385                 390                 395                 400
Glu Thr Asp Pro Ser
                405
<210> 3
<211> 415
<212> PRT
<213> Homo sapiens
<400> 3

## Description

### Technical Field

The present invention relates to a composition for regulating neuromedin U receptor function containing a nitrogen-containing ring derivative having an amino group.

### Background Art

Neuromedin U is a peptide which was isolated and purified from a pig spine using rat uterine smooth muscle constricting activity as an index, two kinds of neuromedin U-8 consisting of 8 amino acid residues and neuromedin U-25 consisting of 25 amino acid residues were first reported (Minamino, N. et al., Biochem. Biophys. Res. Commun. 130, 1078-1085, 1985) and, thereafter, neuromedin U was identified from many animal species including a human and a rat. Since a sequence of neuromedin U-8 is consistent with a C-terminal part of neuromedin U-25, a basic amino acid pair which is frequently seen at a site undergoing cleavage by processing is present at its upstream, both are thought to be derived from a common precursor. As physiological action of neuromedin U, in addition to smooth muscle constricting action, many actions have been widely known and, for example, increase in a blood pressure (Minamino, N. et al., Biochem. Biophys. Res. Commun. 130, 1078-1085, 1985), reduction in a blood stream amount of viscus (Sumi, S. et al., Life Sci. 41, 1585-1590, 1987), regulation of ion transport in an intestine tract (Brown, D.R. and Quito, F.L., Eur. J. Pharmacol. 155, 159-162, 1988), and increase in ACTH after subcutaneous administration and subsequent corticosterone (Malendowicz, L.K. et al., In Vivo, 7, 419-422, 1993) have been reported.

A neuromedin U receptor has been unknown for a long time, but recently, FM-3 (WO 00/02919, WO 01/40797) and TGR-1 (WO 01/57524), which are an orphan receptor, have been found to be neuromedin U receptors. FM-3 is also called as GPR66, NmU-R1, NMU1 or SNORF62, and TGR-1 is also called as FM-4, NmU-R2, NMU2 or SNORF72. FM-3 and TGR-1 are a subtype of a neuromedin U receptor, and FM-3 is distributed mainly in lung, and an intestine tract, and TGR-1 is expressed much in hypothalamus, medulla oblongata, spine and ovary. However, a selective agonist or antagonist of these neuromedin U receptors has not been known.

It is reported that a compound of the formula: 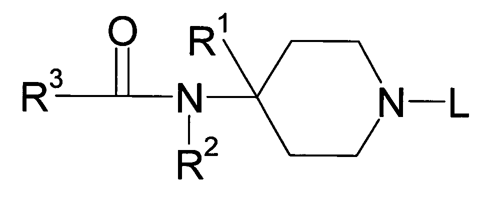 wherein R¹ is a pyridyl group, R² is a phenyl group optionally substituted with a halogen atom, R³ is a lower alkyl group, and L is a phenyl lower alkyl group, a pyrazolyl lower alkyl group or a (4, 5-di-hydro-5-oxy-1H-tetrazolyl) lower alkyl group optionally substituted with lower alkyl at a 4-position, has anesthetic, antagonistic or analgesic action (JP 1-213279 A).

It is reported that a compound of the formula: 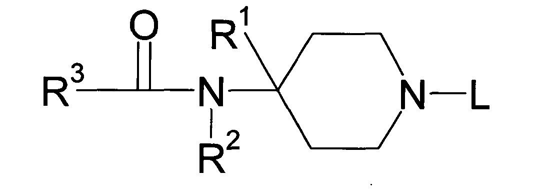 wherein R¹ is an unsubstituted or substituted unsaturated heterocyclic 5-membered ring, R² is an unsubstituted or substituted phenyl group, R³ is a lower alkyl group or a lower alkoxy group, and L is selected from various groups, has anesthetic, antagonistic or analgesic action (USP4791120).

Since neuromedin U or a salt thereof is involved in smooth muscle constricting action, blood pressure rising action, regulation of ion transport in an intestine tract, and increase in ACTH after subcutaneous administration and subsequent corticosterone, it is utilized as a preventive/therapeutic agent for hypotension, a local vessel constricting agent, or a therapeutic agent for pituitary gland hormone secretion dysfunction, therefore, a neuromedin U receptor agonist can be used as apreventive/therapeutic agent for hypotension, alocal vessel constricting agent, or atherapeutic agent for pituitary gland hormone dysfunction, and aneuromedin U receptor antagonist can be used as apreventive/therapeutic agent for hypertension, cardiac infarct, acute renal dysfunction, or stress disease (e.g. (i) cardiac vascular disease (angina, cardiac infarct, arrhythmia etc.), (ii) respiratory disease (bronchial asthma, hyperpnea syndrome etc.), (iii) muscular skeletal disease (rheumatoid arthritis, lumbago, migraine, tension headache etc.), (iv) others (diabetes, climacteric disorder, chronic pain, immune activity reduction etc.)), or a digestive tract disease (stomach ulcer, ulcerative colitis etc.).

In addition, since neuromedin U or a salt thereof has appetite regulating action and, based on the appetite regulating action, aneuromedin receptor agonist can be used as aappetite inhibiting agent, ananti-obesity agent, atherapeutic agent for bulimia, or atherapeutic agent for polyphagia, and aneuromedin U receptor antagonist can be used as anappetite promoting agent.

A selective non-peptidic low-molecular agonist or antagonist of two receptors TGR-1 and FM-3 of neuromedin U has previously not been known. Then, development of a neuromedin U receptor function regulator excellent as a preventive/therapeutic agent for hypertension is desired.

The present invention provides, as a preventive/therapeutic agent for neuromedin U-associated morbid state or disease, a composition-- for regulating neuromedin U receptor functionwhich has a nitrogen-containing ring (amino piperidine skeleton etc.) having an amino group, and is useful and safe.

### Disclosure of Invention

The present inventors have studied intensively and, as a result, found out that a compound having a nitrogen-containing ring having an amino group (aminopiperidine skeleton etc.) or a salt thereof has unexpectedly excellent neuromedin U receptor function regulating action based on its peculiar chemical structure, further has excellent nature also in physical properties as a medicine such as stability, and is a medicine which is safe and useful as a preventive/therapeutic agent for mammal neuromedin U-associated morbid state or disease. The present invention has been completed based on these findings.

That is, the present invention provides:
[1] a composition for regulatingneuromedin U receptor, which comprises a compound having a partial structure represented by the formula: 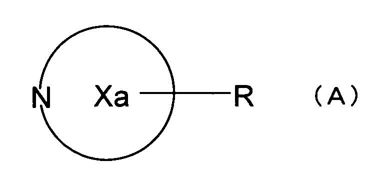 wherein ring Xa represents a nitrogen-containing ring, and R represents an optionally substituted amino group, or a salt thereof,
[2] the composition according to [1], which comprises a compound having an amino piperidine skeleton or a salt thereof,
[3] the composition according to [1], which comprises a compound represented by the formula: 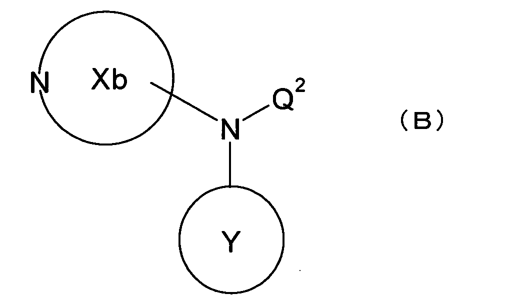 wherein ring Xb represents an optionally further substituted 5 to 8-membered nitrogen-containing ring, Y represents an optionally substituted ring group, and Q² represents an acyl group, or a salt thereof or a prodrug thereof,
[4] the composition according to [1], which comprises a compound represented by the formula: 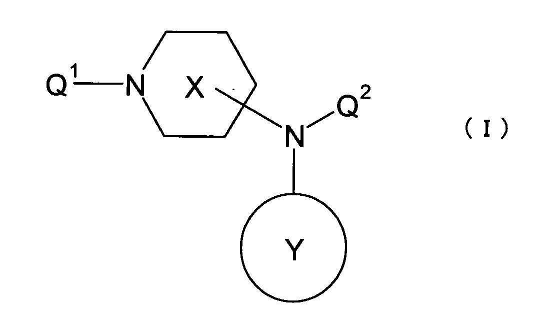 wherein ring X represents an optionally further substituted piperidine ring, Y represents an optionally substituted ring group, Q¹ represents a hydrogen atom or a substituent, and Q² represents an acyl group, or a salt thereof or a prodrug thereof,
[5] the composition according to [1], which comprises a compound represented by the formula: 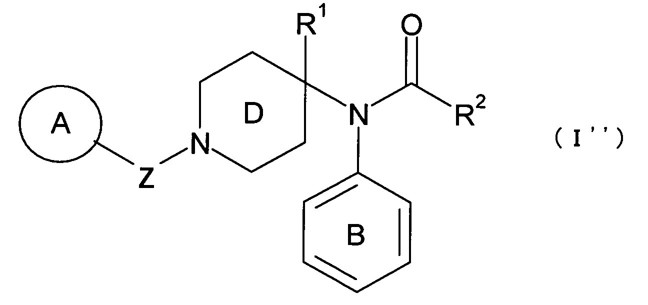 wherein A represents an optionally substituted ring group, B represents an optionally substituted phenyl group, ring D represents an optionally further substituted piperidine ring, Z represents an optionally substituted methylene group, -COCH₂-, -CH₂CO- or -SO₂-, R¹ represents a hydrogen atom, a cyano group, an optionally substituted lower alkyl group, an optionally substituted phenyl group, an optionally substituted aromatic heterocyclic group, an optionally esterified carboxyl group or an optionally substituted carbamoyl group, and R² represents an optionally substituted lower alkyl group, an optionally substituted lower alkenyl group, an optionally substituted lower alkoxy group, an optionally substituted aralkyloxy group or an optionally substituted phenyl group, or a salt thereof or a prodrug thereof,
[6] the composition according to [1], which is an antagonist of a neuromedin U receptor FM-3,
[7] the composition according to [1], which is a composition for physiological function in which neuromedin U is involved, or a preventive/therapeutic agent for morbid state or disease in which neuromedin U in involved,
[8] the composition according to [1], which is a preventive/therapeutic agent for hypertension, cardiac infarct, acute renal dysfunction, angina, cardiac infarct, arrhythmia, bronchial asthma, hyperpnea syndrome, rheumatoid arthritis, diabetes, climacteric disorder, immune activity reduction, stomach ulcer or ulcerative colitis, or a composition for regulating an appetite,
[9] a method of regulating function of a neuromedin U receptor, which comprises administering an effective amount of a compound having a partial structure represented by the formula: 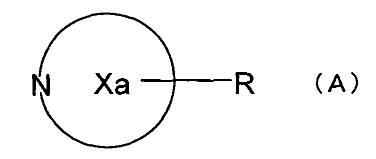 wherein ring Xa represents a nitrogen-containing ring, and R represents an optionally substituted amino group, or a salt thereof to a mammal,
[10] use of a compound having a partial structure represented by the formula: 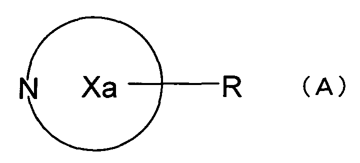 wherein ring Xa represents a nitrogen-containing ring, and R represents an optionally substituted amino group, or a salt thereof for preparing a composition for regulatingneuromedin U receptor,
[11] a compound represented by the formula: 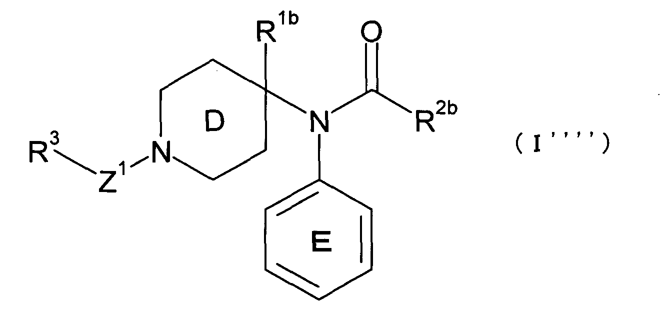 wherein ring D represents an optionally further substituted piperidine ring, E represents an optionally substituted phenyl group, Z¹ represents a methylene group optionally substituted with a substituent selected from the group consisting of lower alkyl, lower alkoxycarbonyl, oxo and phenyl, -COCH₂-, -CH₂CO- or -SO₂-, R^{1b} represents an optionally substituted 2-thiazolyl group, an optionally substituted 2-imidazolyl group or an optionally substituted 2-pyridyl group, R^{2b} represents an optionally halogenated lower alkyl group, and R³ represents an optionally substituted phenyl group, an optionally substituted aromatic heterocyclic group or an optionally substituted cycloalkyl group, provided that N-[1-benzyl-4-(4-methylthiazol-2-yl)-4-piperidinyl]-N-phenylpropionamide and N-[1-benzyl-4-(2-pyridinyl)-4-piperidinyl]-N-phenylpropionamide are excluded, or a salt thereof,
[12] the compound according to [11], wherein R³ is an optionally substituted phenyl group or an optionally substituted thienyl group,
[13] the compound according to [11], wherein R³ is a phenyl group,
[14] the compound according to [11], wherein E is a phenyl group optionally having a substituent at an ortho position or a meta position,
[15] the compound according to [11], wherein E is an unsubstituted phenyl group,
[16] the compound according to [11], wherein R^{1b} is a 2-thiazolyl group optionally substituted with a lower alkyl group,
[17] the compound according to [11], wherein R^{1b} is a 4-methyl-2-thiazolyl group,
[18] the compound according to [11], wherein R^{1b} is a 2-pyridyl group optionally substituted with a substituent selected from the group consisting of a lower alkyl group, a lower alkylthio group, a halogen atom, a C₆₋₁₄aryl group and an aromatic heterocyclic group,
[19] the compound according to [11], wherein R^{1b} is a 6-methyl-2-pyridyl group,
[20] the compound according to [11], wherein Z¹ is a methylene group optionally substituted with a lower alkyl group,
[21] the compound according to [11], wherein Z¹ is a methylene group,
[22] the compound according to [11], wherein R^{2b} is an optionally halogenated methyl group or ethyl group,
[23] the compound according to [11], wherein R^{2b} is a methyl group or a trifluoromethyl group,
[24] the compound according to [11], wherein ring D is a piperidine ring optionally further substituted with a lower alkyl,
[25] the compound according to [11], wherein ring D is a piperidine ring optionally further substituted with C₁₋₆alkyl, E is a phenyl group optionally substituted with a substituent selected from the group consisting of a halogen atom and C₁₋₆alkyl, Z¹ is a methylene group optionally substituted with a substituent selected from the group consisting of C₁₋₆alkyl, C₁₋₆alkoxycarbonyl, oxo and phenyl, -COCH₂- or -SO₂-, R^{1b} is (i) a 2-thiazolyl group optionally substituted with C₁₋₆alkyl, (ii) a 2-imidazolyl group optionally substituted with C₁₋₆alkyl, or (iii) a 2-pyridyl group optionally substituted with a substituent selected from the group consisting of C₁₋₆alkyl, a halogen atom, C₁₋₆alkylthio, phenyl and thienyl, R^{2b} is an optionally halogenated C₁₋₆alkyl group, R³ is (i) a C₃₋₈cycloalkyl group, (ii) a phenyl group or (iii) a 5- to 10-membered aromatic heterocyclic group containing one or two kinds of 1 to 4 hetero atoms selected from a nitrogen atom, a sulfur atom and an oxygen atom in addition to carbon atoms, which may be substituted with a substituent selected from the group consisting of a halogen atom, cyano, C₁₋₆alkyl optionally substituted with a halogen atom, C₁₋₆alkoxy optionally substituted with a halogen atom, C₁₋₆alkyl-carbonylamino, a 5- or 6-membered aromatic heterocyclic group and C₁₋₆alkylthio,
[26] N-[1-benzyl-4-(4-methylthiazol-2-yl)-4-piperidinyl]-N-phenylacetamide, N-[1-benzyl-4-(4-methylthiazol-2-yl)-4-piperidinyl]-2,2,2-trifluoro-N-phenylacetamide, N-[1-benzyl-4-(6-methyl-2-pyridinyl)-4-piperidinyl]-N-phenylacetamide, N-[1-benzyl-4-(6-methyl-2-pyridinyl)-4-piperidinyl]-2,2,2-trifluoro-N-phenylacetamide, N-[1-(4-fluorobenzyl)-4-(4-methylthiazol-2-yl)-4-piperidinyl]-N-phenylacetamide, N-[1-benzyl-4-(4-methylthiazol-2-yl)-4-piperidinyl]-N-(2-methylphenyl)acetamide, N-[1-benzyl-4-(4-methylthiazol-2-yl)-4-piperidinyl]-N-(3-chlorophenyl)acetamide, N-[4-(4-methylthiazol-1-(2-thienylmethyl)-2-yl)-4-piperidinyl]-N-phenylacetamide, N-[1-benzyl-4-(1-methyl-1H-imidazol-2-yl)-4-piperidinyl]-N-phenylacetamide, or a salt thereof,
[27] a prodrug of the compound according to [11] or [26] or a salt thereof, and
[28] a medicine comprising the compound according to [11] or [26] or a salt thereof or a prodrug thereof.

Further, the present invention provides:
[29] the composition according to [5], wherein A is an optionally substituted phenyl group or an optionally substituted thienyl group,
[30] the composition according to [5], wherein A is a phenyl group,
[31] the composition according to [5], wherein B is a phenyl group optionally having a substituent at an ortho or meta position,
[32] the composition according to [5], wherein B is an unsubstituted phenyl group,
[33] the composition according to [5], wherein R¹ is an optionally substituted phenyl group, an optionally substituted aromatic heterocyclic group or an optionally esterified carboxyl group,
[34] the composition according to [5], wherein R¹ is an optionally substituted 5-membered aromatic heterocyclic group,
[35] the composition according to [5], wherein R¹ is an optionally substituted thiazolyl group,
[36] the composition according to [5], wherein R¹ is a 2-thiazolyl group optionally substituted with a lower alkyl group,
[37] the composition according to [5], wherein R¹ is a 4-methyl-2-thiazolyl group,
[38] the composition according to [5], wherein R¹ is a pyridyl group optionally substituted with (a substituent selected from the group consisting of C₁₋₆ alkyl, a halogen atom, C₁₋₆ alkylthio, phenyl and thienyl),
[39] the composition according to [5], wherein R¹ is a 2-pyridyl group optionally substituted with (a substituent selected from the group consisting of C₁₋₆ alkyl, a halogen atom, C₁₋₆ alkylthio, phenyl and thienyl),
[40] the composition according to [5], wherein R¹ is an optionally esterified carboxyl group,
[41] the composition according to [5], wherein R² is an optionally halogenated methyl group or ethyl group,
[42] the composition according to [5], wherein A is an optionally substituted phenyl group, and R¹ is an optionally substituted aromatic heterocyclic group, or an optionally esterified carboxyl group,
[43] the composition according to [5], wherein A is an optionally substituted phenyl group, and R¹ is an optionally substituted 5- or 6-membered nitrogen-containing aromatic heterocyclic group,
[44] the composition according to [5], wherein A is an optionally substituted phenyl group, and R¹ is a 2-thiazolyl group optionally substituted with a lower alkyl group, or an optionally substituted 2-pyridyl group,
[45] the composition according to [5], wherein A is an optionally substituted phenyl group, and R¹ is a 4-methyl-2-thiazolyl group, and
[46] the composition according to [5], wherein A is (i) a C₃₋₈ cycloalkyl group, (ii) a C₆₋₁₄ aryl group or (iii) a 5- to 10-membered aromatic heterocyclic group containing one or two kinds of 1 to 4 hetero atoms selected from a nitrogen atom, a sulfur atom and an oxygen atom in addition to carbon atoms, which may be substituted with a substituent selected from the group consisting of halogen atom, cyano, C₁₋₆ alkyl optionally substituted with a halogen atom, C₁₋₆ alkoxy optionally substituted with a halogen atom, C₁₋₆ alkyl-carbonylamino, a 5- or 6-membered aromatic heterocyclic group and C₁₋₆ alkylthio, B is a phenyl group optionally substituted with a substituent selected from a halogen atom and C₁₋₆ alkyl, a ring D is a piperidine ring optionally further substituted with C₁₋₆ alkyl, Z is a methylene group optionally substituted with a substituent selected from the group consisting of C₁₋₆ alkyl, C₁₋₆ alkoxycarbonyl, oxo and phenyl, -COCH₂-, -CH₂CO- or - SO₂-, R¹ is (i) a hydrogen atom, (ii) a cyano group, (iii) a C₁₋₆ alkyl group optionally substituted with a 5- or 6-membered aromatic heterocyclic group, (iv) a phenyl group, (v) a thiazolyl group optionally substituted with C₁₋₆ alkyl, (vi) an imidazolyl group optionally substituted with C₁₋₆ alkyl, (vii) a pyridyl group optionally substituted with a substituent selected from the group consisting of C₁₋₆ alkyl, a halogen atom, C₁₋₆ alkylthio, phenyl and thienyl, (viii) a carboxyl group, (ix) a C₁₋₆ alkoxy-carbonyl group, (x) a C₇₋₁₆ aralkyloxy-carbonyl group, (xi) a carbamoyl group, (xii) a mono-C₁₋₆ alkyl-carbamoyl group, (xiii) a C₁₋₆ alkyl (C₁₋₆ alkoxy)-carbamoyl group, (xiv) a 5- to 7-membered cyclic carbamoyl group and R² is an optionally halogenated C₁₋₆ alkyl group, a C₂₋₆ alkenyl group, a C₁₋₆ alkoxy group, a C₇₋₁₁ aralkyloxy group or a phenyl group.

### Best Mode for Carrying Out the Invention

The compound used in the present invention (hereinafter, abbreviated as the compound of the present invention in some cases) is a compound having a partial structure represented by the formula: 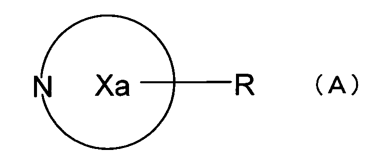 wherein ring Xa represents a nitrogen-containing ring, and R represents an optionally substituted amino group, preferably a compound having an aminopiperidine skeleton (further preferably 4-aminopiperidine skeleton).

Specifically, the compound of the present invention is a compound represented by the formula: 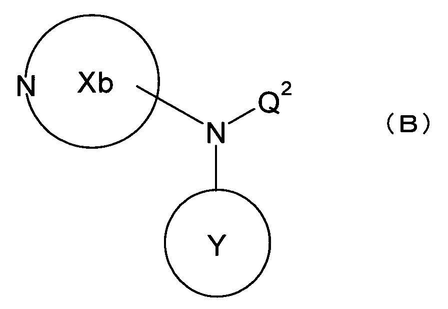 wherein ring Xb represents an optionally further substituted 5- to 8-membered nitrogen-containing ring, Y represents an optionally substituted ring group, and Q² represents an acyl group, preferably a compound represented by the formula: 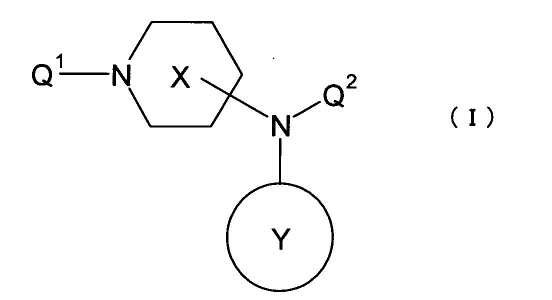 wherein ring X represents an optionally further substituted piperidine ring, Y represents an optionally substituted ring group, Q¹ represents a hydrogen atom or a substituent, and Q² represents an acyl group, further preferably a compound represented by the formula: 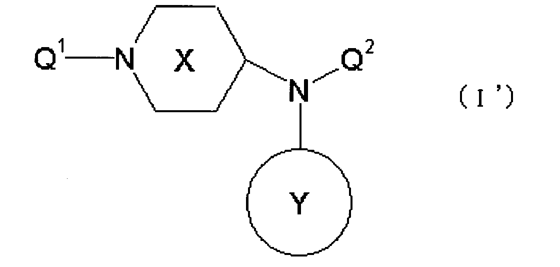 wherein respective symbols are as defined above, more preferably a compound represented by the formula: 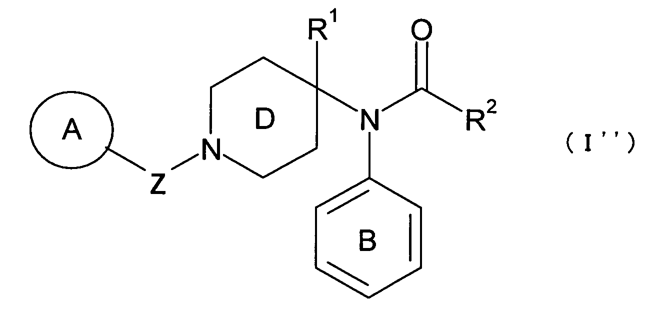 wherein A represents an optionally substituted cyclic group, B represents an optionally substituted phenyl group, ring D represents an optionally substituted piperidine ring, Z represents an optionally substituted methylene group, - COCH₂-, -CH₂CO- or -SO₂-, R¹ represents a hydrogen atom, a cyano group, an optionally substituted lower alkyl group, an optionally substituted phenyl group, an optionally substituted aromatic heterocyclic group, an optionally esterified carboxyl group or an optionally substituted carbamoyl group, and R² represents an optionally substituted lower alkyl group, an optionally substituted lower alkenyl, an optionally substituted lower alkoxy group, an optionally substituted aralkyloxy group or an optionally substituted phenyl group, particularly preferably a compound represented by the formula: 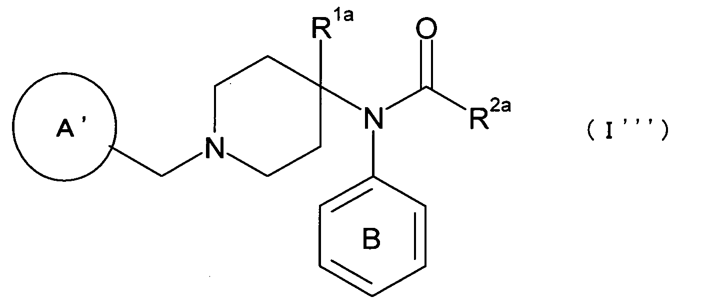 wherein A' represents an optionally substituted phenyl group or an optionally substituted aromatic heterocyclic group, B represents an optionally substituted phenyl group, R^{1a} represents a lower alkyl group optionally substituted with a lower alkoxy group, an optionally substituted phenyl group, an optionally substituted 5-membered aromatic heterocyclic group, a pyridyl group, an optionally esterified carboxyl group or an optionally substituted carbamoyl group, and R^{2a} represents an optionally halogenated lower alkyl group, or a salt thereof or a prodrug thereof.

Ring Xa represents a nitrogen-containing ring, for example, a 5- to 8-membered nitrogen-containing ring.

As the 5- to 8-membered nitrogen-containing ring, for example, a saturated or unsaturated 5- to 8-membered nitrogen-containing ring comprising one nitrogen atom and carbon atoms is used, specifically, for example, a 5- to 8-membered saturated nitrogen-containing ring such as a pyrrolidine ring, a piperidine ring, an azepane ring, an azokane ring, and a 5- to 8-membered unsaturated nitrogen-containing ring such as a dihydropyrrole ring, a pyridine ring, a dihydropyridine ring, a tetrahydropyridine ring, a dihydroazepine ring, a tetrahydroazepine ring, a dihydroazocine ring, a tetrahydroazocine ring, and a hexahydroazocine ring are used.

As the substituent for the "optionally substituted amino group" represented by R, for example, an optionally substituted ring group (Y), an acyl group (Q²) and an optionally substituted hydrocarbon group (exemplified as Q¹) mentioned below are used.

The position of R substituting on a ring Xa is not particularly limited, but particularly preferably on a carbon atom.

As the "5- to 8-membered nitrogen-containing ring" of the "optionally further substituted 5- to 8-membered nitrogen-containing ring" represented by ring Xb, the same groups as those exemplified for ring Xa are used.

The position of a group represented by the formula: 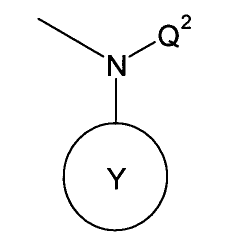 wherein respective symbols are as defined above, on ring Xb is not particularly limited, but preferably on a carbon atom, particularly preferably on a carbon atom separated from the nitrogen atom of ring Xb by 2 or 3 atoms, is preferable.

The position of a group represented by the formula: 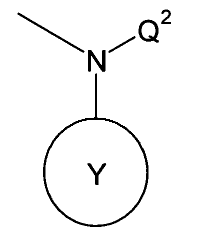 wherein respective symbols are as defined above, substituting on the piperidine ring X is not particularly limited, but is preferably the 4-position.

Ring Xb or ring X may further have a substituent, in addition to: 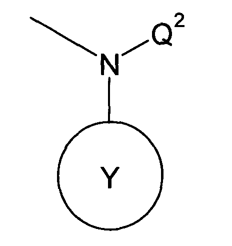 wherein respective symbols are as defined above, and, as the substituent, a substituent (substituent A group) selected from oxo, a halogen atom (e.g. fluorine, chlorine, bromine, iodine etc.), C₁₋₃ alkylenedioxy (e.g. methylenedioxy, ethylenedioxy etc.), nitro, cyano, an optionally substituted hydrocarbon group [e.g. optionally substituted lower (C₁₋₆)alkyl, an optionally substituted lower (C₂₋₆)alkenyl, optionally substituted lower (C₂₋₆)alkynyl, optionally substituted C₃₋₈ cycloalkyl, optionally substituted C₆₋₁₄ aryl (preferably optionally substituted phenyl), or optionally substituted C₇₋₁₉ aralkyl etc.], optionally substituted lower (C₁₋₆)alkoxy, hydroxy, optionally substituted C₆₋₁₄aryloxy (e.g. phenyloxy, 1-naphthyloxy, 2-naphthyloxy etc.), optionally substituted C₇₋₁₆aralkyloxy (e.g. benzyloxy, phenethyloxy etc.), mercapto, optionally substituted lower (C₁₋₆)alkylthio, optionally substituted C₆₋₁₄ arylthio, optionally substituted C₇₋₁₄ aralkylthio, formyl, carboxy, optionally substituted lower (C₁₋₆)alkyl-carbonyl (e.g. acetyl, propionyl, pivaloyl etc.), optionally substituted C₃₋₈ cycloalkyl-carbonyl (e.g. cyclopropylcarbonyl, cyclopentylcarbonyl, cyclohexylcarbonyl, 1-methylcyclohexyl-carbonyl etc.), optionally substituted C₆₋₁₄ aryl-carbonyl (e.g. benzoyl, 1-naphthoyl, 2-naphthoyl etc.), optionally substituted C₇₋₁₆ aralkyl-carbonyl (e.g. phenylacetyl, 3-phenylpropionyl etc.), optionally substituted 5- to 7-membered heterocyclic carbonyl containing one or two kinds of 1 to 4 hetero atoms selected from a nitrogen atom, a sulfur atom and an oxygen atom in addition to carbon atoms (e.g. nicotinoyl, isonicotinoyl, thenoyl, furoyl, morpholinocarboyl, thiomorpholinocarbonyl, piperazin-1-ylcarbonyl, pyrrolidin-1-ylcarbonyl etc.), optionally esterified carboxyl, optionally substituted carbamoyl group, optionally substituted lower (C₁₋₆)alkylsulfonyl (e.g. methylsulfonyl, ethylsulfonyl etc.), optionally substituted lower (C₁₋₆)alkylsulfinyl (e.g. methylsulfinyl, ethylsulfinyl etc.), optionally substituted C₆₋₁₄ arylsulfonyl (e.g. phenylsulfonyl, 1-naphthylsulfonyl, 2-naphthylsulfonyl etc.), optionally substituted C₆₋₁₄ arylsulfinyl (e.g. phenylsulfinyl, 1-naphthylsulfinyl, 2-naphthylsulfinyl etc.), optionally substituted amino, optionally substituted lower (C₁₋₆)alkyl-carbonyloxy (e.g. acetoxy, propionyloxy etc.), optionally substituted C₆₋₁₄ aryl-carbonyloxy (e.g. benzoyloxy, naphthylcarbonyloxy etc.), optionally substituted lower (C₁₋₆)alkoxy-carbonyloxy (e.g. methoxycarbonyloxy, ethoxycarbonyloxy, propoxycarbonyloxy, butoxycarbonyloxy etc.), optionally substituted mono-lower (C₁₋₆)alkyl-carbamoyloxy (e.g. methylcarbamoyloxy, ethylcarbamoyloxy etc.), optionally substituted di-lower (C₁₋₆)alkyl-carbamoyloxy (e.g. dimethylcarbamoyloxy, diethylcarbamoyloxy etc.), optionally substituted mono- or di-C₆₋₁₄ aryl-carbamoyloxy (e.g. phenylcarbamoyloxy, naphthylcarbamoyloxy etc.), optionally substituted heterocyclic group, sulfo, sulfamoyl, sulfinamoyl, sulfenamoyl, or a group in which two or more (e.g. 2 to 3) of these substituents are bound is used. Ring Xb or ring X may have 1 to 5, preferably 1 to 3 of the aforementioned substituents at a replaceable position and, when the number of replaceable groups is 2 or more, respective substituents may be the same or different.

As the "optionally esterified carboxyl group" of the substituent A group, for example, C₁₋₆ alkoxy-carbonyl (e.g. methoxycarbonyl, ethoxycarbonyl, propoxycarbonyl, tert-butoxycarbonyl etc.), C₆₋₁₄ aryloxy-carbonyl (e.g. phenoxycarbonyl etc.), and C₇₋₁₆ aralkyloxy-carbonyl (e.g. benzyloxycarbonyl, phenethyloxycarbonyl etc.) are used.

As the "lower (C₁₋₆)alkyl" of the "optionally substituted lower (C₁₋₆)alkyl" of the substituent A group, for example, methyl, ethyl, propyl, isopropyl, butyl, isobutyl, sec-butyl, tert-butyl, pentyl, isopentyl, neopentyl, and hexyl are used.

As the "lower (C₂₋₆)alkenyl" of the "optionally substituted lower (C₂₋₆)alkenyl" of the substituent A group, for example, vinyl, allyl, 1-propenyl, isopropenyl, 2-buten-1-yl, 4-penten-1-yl, and 5-hexen-1-yl are used.

As the "lower (C₂₋₆)alkynyl" of the "optionally substituted lower (C₂₋₆)alkynyl" of the substituent A group, for example, ethynyl, propargyl, 1-propynyl, 2-butyn-1-yl, 4-pentyn-1-yl, and 5-hexyn-1-yl are used.

As the "C₃₋₈ cycloalkyl" of the "optionally substituted C₃₋₈ cycloalkyl" of the substituent A group, for example, cyclopropyl, cyclobutyl, cyclopentyl, and cyclohexyl are used.

As the "C₆₋₁₄ aryl" of the "optionally substituted C₆₋₁₄ aryl" of the substituent A group, for example, phenyl, 1-naphthyl, 2-naphthyl, 2-biphenylyl, 3-biphenylyl, 4-biphenylyl, and 2-anthryl are used.

As the "C₇₋₁₉ aralkyl" of the "optionally substituted C₇₋₁₉ aralkyl" of the substituent A group, for example, benzyl, phenethyl, diphenylmethyl, 1-naphthylmethyl, 2-naphthylmethyl, 2, 2-diphenylethyl, 3-phenylpropyl, 4-phenylbutyl, 5-phenylpentyl, 2-biphenylylmethyl, 3-biphenylylmethyl, 4-biphenylylmethyl, and trityl are used, and C₇₋₁₆ aralkyl is preferable.

As the "lower (C₁₋₆)alkoxy" of the "optionally substituted lower (C₁₋₆)alkoxy" of the substituent A group, for example, methoxy, ethoxy, propoxy, isopropoxy, butoxy, isobutoxy, sec-butoxy, pentyloxy, and hexyloxy are used.

As the "lower (C₁₋₆)alkylthio" of the "optionally substituted lower (C₁₋₆)alkylthio" of the substituent A group, for example, methylthio, ethylthio, propylthio, isopropylthio, butylthio, sec-butylthio, and tert-butylthio are used.

As the "C₆₋₁₄ arylthio" of the "optionally substituted C₆₋₁₄ arylthio" of the substituent A group, for example, phenylthio, 1-naphthylthio, and 2-naphthylthio are used.

As the "C₇₋₁₆ aralkylthio" of the "optionally substituted C₇₋₁₆ aralkylthio" of the substituent A group, for example, benzylthio, and phenethylthio are used.

As the substituent of these "lower alkyl", "lower alkenyl", "lower alkynyl", "C₃₋₈ cycloalkyl", "lower alkoxy", "lower alkylthio", "lower alkyl-carbonyl", "C₃₋₈cycloalkyl-carbonyl", "lower alkylsulfonyl", "lower alkylsulfinyl", "lower alkyl-carbonylamino", "C₃₋₈ cycloalkyl-carbonylamino", "lower alkoxy-carbonylamino", "lower alkylsulfonylamino", "lower alkyl-carbonyloxy", "lower alkoxy-carbonyloxy", "mono-lower alkyl-carbamoyloxy" and, "di-lower (C₁₋₆)alkyl-carbamoyloxy", 1 to 5 substituents selected from a halogen atom (e.g. fluorine atom, chlorine atom, bromine atom, iodine atom), carboxy, hydroxy, amino, mono- or di-lower (C₁₋₆)alkylamino, mono- or di-C₆₋₁₄ arylamino, mono- or di-C₇₋₁₆ aralkylamino, formylamino, lower (C₁₋₆)alkyl-carbonylamino, C₃₋₈ cycloalkyl-carbonylamino, C₆₋₁₄ aryl-carbonylamino, lower (C₁₋₆)alkoxy-carbonylamino, lower (C₁₋₆)alkylsulfonylamino, C₆₋₁₄ arylsulfonylamino, C₃₋₈ cycloalkyl, lower (C₁₋₆)alkoxy, lower (C₁₋₆)alkoxy-carbonyl, lower (C₁₋₆)alkylthio, lower (C₁₋₆)alkylsulfinyl, lower (C₁₋₆)alkylsulfonyl, the aforementioned optionally esterified carboxyl, carbamoyl, thiocarbamoyl, mono-lower (C₁₋₆)alkyl-carbamoyl (e.g. methylcarbamoyl, ethylcarbamoyl etc.), di-lower (C₁₋₆)alkyl-carbamoyl (e.g. dimethylcarbamoyl, diethylcarbamoyl, ethylmethylcarbamoyl etc.), mono- or di-C₆₋₁₄ aryl-carbamoyl (e.g. phenylcarbamoyl, 1-naphthylcarbamoyl, 2-naphthylcarbamoyl etc.), mono- or di-5 to 7-membered heterocyclic carbamoyl containing one or two kinds of 1 to 4 hetero atoms selected from a nitrogen atom, a sulfur atom and an oxygen atom in addition to carbon atoms (e.g. 2-pyridylcarbamoyl, 3-pyridylcarbamoyl, 4-pyridylcarbamoyl, 2-thienylcarbamoyl, 3-thienylcarbamoyl etc.), 5 to 14-membered (monocyclic, dicyclic or tricyclic) heterocyclic group containing one or two kinds of 1 to 4 metal atoms selected from a nitrogen atom, a sulfur atom and an oxygen atom in addition to carbon atoms (e.g. 5 to 8-membered aromatic heterocyclic group such as thienyl, furyl, thiazolyl, oxazolyl, triazolyl, tetrazolyl and pyridyl, preferably 5 or 6-membered aromatic heterocyclic group; the heterocyclic group may be substituted with halogen atom, carboxy, hydroxy, amino, mono- or di-lower (C₁₋₆)alkylamino, mono- or di-C₆₋₁₄ arylamino, C₃₋₈ cycloalkyl, lower (C₁₋₆)alkoxy, lower (C₁₋₆)alkoxy-carbonyl, lower (C₁₋₆)alkylthio, lower (C₁₋₆)alkylsulfinyl, lower (C₁₋₆)alkylsulfonyl, optionally esterified carboxyl, carbamoyl, thiocarbamoyl, mono-lower (C₁₋₆)alkyl-carbamoyl, di-lower (C₁₋₆)alkyl-carbamoyl, mono- or di-C₆₋₁₄ aryl-carbamoyl, or mono- or di-5 to 7-membered heterocyclic carbamoyl containing one or two kinds of 1 to 4 hetero atoms selected from a nitrogen atom, a sulfur atom and an oxygen atom in addition to carbon atoms) which may be substituted with mono- or di-5 to 7-membered heterocyclic carbamoyl containing one or two kinds of 1 to 4 hetero atoms selected from a nitrogen atom, a sulfur atom and an oxygen atom in addition to carbon atoms are used.

As the substituent of the "C₆₋₁₄ aryl", the "C₇₋₁₉ aralkyl", the "C₆₋₁₄ aryloxy", the "C₇₋₁₆ aralkyloxy", the "C₆₋₁₄ arylthio", the "C₇₋₁₆ aralkylthio", the "C₆₋₁₄ arylcarbonyl", the "C₇₋₁₆ aralkyl-carbonyl", the "5- to 7-membered heterocyclic carbonyl", the "C₆₋₁₄ arylsulfonyl", the "C₆₋₁₄ arylsulfinyl", the "C₆₋₁₄ aryl-carbonylamino", the "C₆₋₁₄ arylsulfonylamino", the "C₆₋₁₄ aryl-carbonyloxy" and the "mono-or di-C₆₋₁₄ aryl-carbamoyloxy" of the substituent A group, 1 to 5 substituents selected from a halogen atom, hydroxyl, carboxy, nitro, cyano, amino, mono-or di-lower (C₁₋₆)alkylamino, mono-or di-C₆₋₁₄ arylamino, mono- or di- C₇₋₁₆ aralkylamino, formylamino, lower (C₁₋₆)alkyl-carbonylamino, C₃₋₈ cycloalkyl-carbonylamino, C₆₋₁₄ aryl-carbonylamino, lower (C₁₋₆)alkoxy-carbonylamino, lower (C₁₋₆)alkylsulfonylamino, C₆₋₁₄ arylsulfonylamino, the aforementioned optionally substituted lower alkyl, the aforementioned optionally substituted lower alkenyl, the aforementioned optionally substituted lower alkynyl, the aforementioned optionally substituted C₃₋₈ cycloalkyl, the aforementioned optionally substituted lower alkoxy, the aforementioned optionally substituted lower alkylthio, the aforementioned optionally substituted lower alkylsulfinyl, the aforementioned optionally substituted C₁₋₆ alkylsulfonyl, the aforementioned optionally esterified carboxyl, carbamoyl, thiocarbamoyl, mono-lower alkyl-carbamoyl, di-lower alkyl-carbamoyl, mono-or di-C₆₋₁₄ aryl-carbamoyl, mono-or di-5- to 7-membered heterocyclic carbamoyl containing one or two kinds of 1 to 4 hetero atoms selected from a nitrogen atom, a sulfur atom and an oxygen atom in addition to carbon atoms, 5- to 14-membered (monocyclic, dicyclic or tricyclic)heterocyclic group containing one or two kinds of 1 to 4 hetero atoms selected from a nitrogen atom, a sulfur atom and an oxygen atom in addition to carbon atoms (e.g. 5- to 8- membered aromatic heterocyclic group, such as thienyl, furyl, thiazolyl, oxazolyl, triazolyl, tetrazolyl, imidazolyl, pyrrolyl, thiadiazolyl, oxadiazolyl, pyridyl, pyrazyl, pyrimidyl, pyridazinyl, or triazinyl, preferably 5- or 6- membered aromatic heterocyclic group; the heterocyclic group may be substituted with halogen atom, carboxy, hydroxyl, amino, mono- or di- lower (C₁₋₆)alkylamino, mono- or di- C₆₋₁₄ arylamino, C₃₋₈ cycloalkyl, lower (C₁₋₆)alkoxy, lower (C₁₋₆)alkoxy-carbonyl, lower (C₁₋₆)alkylthio, lower (C₁₋₆)alkylsulfinyl, lower (C₁₋₆)alkylsulfonyl, optionally esterified, carboxyl, carbamoyl, thiocarbamoyl, mono-lower (C₁₋₆)alkyl-carbamoyl, di-lower (C₁₋₆)alkyl-carbamoyl, mono-or di-C₆₋₁₄ aryl-carbamoyl, or mono- or di- 5- to 7-membered heterocyclic carbamoyl containing one or two kinds of 1 to 4 hetero atoms selected from a nitrogen atom, a sulfur atom and an oxygen atom in addition to carbon atoms) which may be substituted with mono- or di- 5- to 7-membered heterocyclic carbamoyl containing one or two kinds of 1 to 4 hetero atoms selected from a nitrogen atom, a sulfur atom and an oxygen atom in addition to carbon atoms are used.

As the "heterocyclic group" of the "optionally substituted heterocyclic group" of the substituent A group, for example, a 5- to 14-membered (monocyclic, dicyclic or tricyclic) heterocyclic group containing one or two kinds of 1 to 4 hetero atoms selected from a nitrogen atom, a sulfur atom and an oxygen atom in addition to carbon atoms, preferably (i) a 5- to 14-membered (preferably 5- to 10-membered) aromatic heterocyclic group, (ii) a 5- to 10-membered non-aromatic heterocyclic group or (iii) a monovalent group obtained by removing one arbitrary hydrogen atom from a 7- to 10-membered heterocyclic crosslinked ring is used and, inter alia, a 5-membered aromatic heterocyclic group is preferably used. Specifically, aromatic heterocyclic groups such as thienyl (e.g. 2-thienyl, 3-thienyl), furyl (e.g. 2-furyl, 3-furyl), pyridyl (e.g. 2-pyridyl, 3-pyridyl, 4-pyridyl), thiazolyl (e.g. 2-thiazolyl, 4-thiazolyl, 5-thiazolyl), oxazolyl (e.g. 2-oxazolyl, 4-oxazolyl, 5-oxazolyl), 1, 2, 4-triazolyl, 1, 2, 3-triazolyl, 1, 3, 4-oxadiazolyl, 1, 3, 4-thiadiazolyl, tetrazolyl (e.g. 1-tetrazolyl, 2-tetrazolyl, 5-tetrazolyl), quinolyl (e.g. 2-quinolyl, 3-quinolyl, 4-quinolyl, 5-quinolyl, 8-quinolyl), isoquinolyl (e.g. 1-isoquinolyl, 3-isoquinolyl, 4-isoquinolyl, 5-isoquinolyl), pyrazinyl, pyrimidinyl (e.g. 2-pyrimidinyl, 4-pyrimidinyl), pyrrolyl (e.g. 1-pyrrolyl, 2-pyrrolyl, 3-pyrrolyl), imidazolyl (e.g. 1-imidazolyl, 2-imidazolyl, 4-imidazolyl), pyrazolyl (e.g. 1-pyrazolyl, 3-pyrazolyl, 4-pyrazolyl), pyridazinyl (e.g. 3-pyridazinyl, 4-pyridazinyl), isothiazolyl (e.g. 3-isothiazolyl), isooxazolyl (e.g. 3-isooxazolyl), indolyl (e.g. 1-indolyl, 2-indolyl, 3-indolyl), 2-benzothiazolyl, benzo[b]thienyl (e.g. 2-benzo[b]thienyl, 3-benzo[b]thienyl), and benzo[b]furanyl (e.g. 2-benzo[b]furanyl, 3-benzo[b]furanyl), and non-aromatic heterocyclic groups such as pyrrolidinyl (e.g. 1-pyrrolidinyl, 2-pyrrolidinyl, 3-pyrrolidinyl), oxazolydinyl (e.g. 2-oxazolidinyl), imidazolinyl (e.g. 1-imidazolinyl, 2-imidazolinyl, 4-imidazolinyl), piperidinyl (e.g. 1-piperidinyl, 2-piperidinyl, 3-piperidinyl, 4-pipiridinyl), piperazinyl (e.g. 1-piperazinyl, 2-piperazinyl), morpholino, thiomorpholino, and azepanyl are used.

As the substituent of the heterocyclic group, for example, a halogen atom, hydroxyl, carboxy, nitro, cyano, amino, mono-or di-lower (C₁₋₆)alkylamino, mono- or di-C₆₋₁₄ arylamino, mono- or di-C₇₋₁₆ aralkylamino, formylamino, lower (C₁₋₆)alkyl-carbonylamino, C₃₋₈ cycloalkyl-carbonylamino, C₆₋₁₄ aryl-carbonylamino, lower (C₁₋₆)alkoxy-carbonylamino, lower (C₁₋₆)alkylsulfonylamino, C₆₋₁₄ arylsulfonylamino, the aforementioned optionally substituted lower alkyl, the aforementioned optionally substituted lower alkenyl, the aforementioned optionally substituted lower alkynyl, the aforementioned optionally substituted C₃₋₈ cycloalkyl, the aforementioned optionally substituted C₆₋₁₄ aryl, the aforementioned optionally substituted lower alkoxy, the aforementioned optionally substituted lower alkylthio, the aforementioned optionally substituted C₆₋₁₄ arylthio, the aforementioned optionally substituted C₇₋₁₆ aralkylthio, the aforementioned optionally substituted lower alkylsulfinyl, the aforementioned optionally substituted C₆₋₁₄ arylsulfinyl, the aforementioned optionally substituted C₁₋₆ alkylsulfonyl, the aforementioned optionally substituted C₆₋₁₄ arylsulfonyl, the aforementioned optionally esterified carboxyl, carbamoyl, thiocarbamoyl, mono-lower alkyl-carbamoyl, di-lower alkyl-carbamoyl, mono- or di-C₆₋₁₄ aryl-carbamoyl, mono- or di- 5- to 7-membered heterocyclic carbamoyl containing one or two kinds of 1 to 4 hetero atoms selected from a nitrogen atom, a sulfur atom and an oxygen atom in addition to carbon atoms, a 5- to 14-membered (monocyclic, dicyclic or tricyclic) heterocyclic group containing one or two kinds of 1 to 4 hetero atoms selected from a nitrogen atom, a sulfur atom and an oxygen atom in addition to carbon atoms (e.g. 5- to 8-membered aromatic heterocyclic group such as thienyl, furyl, thiazolyl, oxazolyl, triazolyl, tetrazolyl, imidazolyl, pyrrolyl, thiadiazolyl, oxadiazolyl, pyridyl, pyrazyl, pyrimidyl, pyridazinyl, and triazinyl; the heterocyclic group may be substituted with halogen atom, carboxy, hydroxyl, amino, mono- or di-lower (C₁₋₆)alkylamino, mono- or di- C₆₋₁₄ arylamino, C₃₋₈ cycloalkyl, lower (C₁₋₆)alkoxy, lower (C₁₋₆)alkoxy-carbonyl, lower (C₁₋₆)alkylthio, lower (C₁₋₆)alkylsulfinyl, lower (C₁₋₆)alkylsulfonyl, optionally esterified carboxyl, carbamoyl, thiocarbamoyl, mono-lower (C₁₋₆)alkyl-carbamoyl, di-lower (C₁₋₆)alkyl-carbamoyl, mono-di-C₆₋₁₄ arylcarbamoyl, or mono-or di- 5- to 7- membered heterocyclic carbamoyl containing one or two kinds of 1 to 4 hetero atoms selected from a nitrogen atom, a sulfur atom and an oxygen atom in addition to carbon atoms) which may be substituted with mono- or di-5- to 7-membered heterocyclic carbamoyl containing one or two kinds of 1 to 4 hetero atoms selected from a nitrogen atom, a sulfur atom and an oxygen atom in addition to carbon atoms are used.

As the "optionally substituted carbamoyl group" of the substituent A group, the aforementioned optionally substituted lower alkyl, optionally substituted C₂₋₆ alkenyl, optionally substituted C₂₋₆ alkynyl, optionally substituted C₃₋₈ cycloalkyl, optionally substituted C₆₋₁₄ aryl, optionally substituted heterocyclic group, and a carbamoyl group optionally substituted with optionally substituted lower alkoxy are used, specifically, for example, carbamoyl, thiocarbamoyl, mono-C₁₋₇ alkyl-carbamoyl (e.g. methylcarbamoyl, ethylcarbamoyl etc.), di-C₁₋₆ alkyl-carbamoyl (e.g. dimethylcarbamoyl, diethylcarbamoyl, ethylmethylcarbamoyl etc.), C₁₋₆ alkyl(C₁₋₆ alkoxy)carbamoyl (e.g. methyl(methoxy)carbamoyl, ethyl(methoxy)carbamoyl), mono-or di-C₆₋₁₄ aryl-carbamoyl (e.g. phenylcarbamoyl, 1-naphthylcarbamoyl, 2-naphthylcarbamoyl etc.), mono- or di-5- to 7-membered heterocyclic carbamoyl containing one or two kinds of 1 to 4 hetero atoms selected from a nitrogen atom, a sulfur atom and an oxygen atom in addition to carbon atoms (e.g. 2-pyridiylcarbamoyl, 3-pyridiylcarbamoyl, 4-pyridylcarbamoyl, 2-thienylcarbamoyl, 3-thienylcarbamoyl etc.), and 5- to 7-membered carbamoyl (e.g. 1-pyrrolidinylcarbonyl, 1-piperidinylcarbonyl, hexamethyleneiminocarbonyl) are used.

As the "optionally substituted amino" of the substituent A group, amino optionally substituted with one or two groups selected from the aforementioned optionally substituted lower alkyl, the aforementioned optionally substituted lower alkenyl, the aforementioned optionally substituted lower alkynyl, the aforementioned optionally substituted C₃₋₈ cycloalkyl, the aforementioned optionally substituted C₆₋₁₄ aryl, the aforementioned optionally substituted C₇₋₁₆ aralkyl, the aforementioned optionally substituted lower alkoxy, formyl, optionally substituted lower alkyl-carbonyl, optionally substituted C₃₋₈ cycloalkyl-carbonyl, optionally substituted C₆₋₁₄ aryl-carbonyl, optionally substituted C₇₋₁₆ aralkyl-carbonyl, optionally substituted 5- to 7-membered heterocyclic carbonyl containing one or two kinds of 1 to 4 hetero atoms selected from a nitrogen atom, a sulfur atom and an oxygen atom in addition to carbon atoms, optionally substituted lower alkylsulfonyl, optionally substituted lower alkylsulfinyl, optionally substituted C₆₋₁₄ arylsulfonyl, and optionally substituted C₆₋₁₄ arylsulfinyl is used. Specifically, for example, amino, optionally substituted mono- or di-lower (C₁₋₆)alkylamino (e.g. methylamino, ethylamino, propylamino, dimethylamino, diethylamino etc.), optionally substituted mono- or di-C₆₋₁₄ arylamino (e.g. phenylamino, diphenylamino etc.), optionally substituted mono- or di-C₇₋₁₆ aralkylamino (e.g. benzylamino etc.), formylamino, optionally substituted lower (C₁₋₆)alkyl-carbonylamino (e.g. acetylamino, propionylamino, pivaloyl amino etc.), optionally substituted C₃₋₈ cycloalkyl-carbonylamino (e.g. cyclopropylcarbonylamino, cyclopentylcarbonylamino, cyclohexylcarbonylamino etc.), optionally substituted C₆₋₁₄ aryl-carbonylamino (e.g. benzoylamino, naphthoylamino etc.), optionally substituted lower (C₁₋₆)alkoxy-carbonylamino (e.g. methoxycarbonylamino, ethoxycarbonylamino, propoxycarbonylamino, butoxycarbonylamino etc.), optionally substituted lower (C₁₋₆)alkylsulfonylamino (e.g. methylsulfonylamino, ethylsulfonylamino etc.), and optionally substituted C₆₋₁₄ arylsulfonylamino (e.g. phenylsulfonylamino, 2-naphthylsulfonylamino, 1-naphthylsulfonylamino etc.) are preferably used.

As the "optionally substituted cyclic group" represented by Y, for example, an "optionally substituted cyclic hydrocarbon group" or an "optionally substituted heterocyclic group" is used.

As the "heterocyclic hydrocarbon group" of the "optionally substituted heterocyclic hydrocarbon group", a cyclic hydrocarbon group of a carbon number of 3 to 16 is used and, for example, a cycloalkyl group, an aryl group, and an aralkyl group are used.

As the "cycloalkyl group", for example, a C₃₋₈ cycloalkyl group (e.g. cyclopropyl, cyclobutyl, cyclopentyl, cyclohexyl) is used.

As the "aryl group", for example, a C₆₋₁₄ aryl group (e.g. phenyl, 1-naphthyl, 2-naphthyl, 2-anthryl) is used.

As the "aralkyl group", for example, a C₇₋₁₉ aralkyl group (e.g. benzyl, phenethyl, diphenylmethyl, 1-naphthylmethyl, 2-naphthylmethyl, 2, 2-diphenylethyl, 3-phenylpropyl, 4-phenylbutyl, 5-phenylpentyl, tolyl) is used and, inter alia, a C₇₋₁₆ aralkyl group is preferable.

Examples of the "heterocyclic group" of the "optionally substituted heterocyclic group" include a 5- to 14-membered (monocyclic, dicyclic or tricyclic) heterocyclic group containing one or two kinds of 1 to 4 hetero atoms selected from a nitrogen atom, a sulfur atom and an oxygen atom in addition to carbon atoms, preferably, (i) a 5- to 14-membered (preferably 5- to 10-membered) aromatic heterocyclic group, (ii) a 5- to 10-membered non-aromatic heterocyclic group and (iii) a monovalent group obtained by removing one arbitrary hydrogen atom from a 7-to 10-membered heterocyclic crosslinked ring. Specifically, aromatic heterocyclic groups such as thienyl (e.g. 2-thienyl, 3-thienyl), furyl (e.g. 2-furyl, 3-furyl), pyridyl (e.g. 2-pyridyl. 3-pyridyl, 4-pyridyl), thiazolyl (e.g. 2-thiazolyl, 4-thiazolyl, 5-thiazolyl), oxazolyl (e.g. 2-oxazolyl, 4-oxazolyl, 5-oxazolyl), 1,2,4-triazolyl, 1,2,3-triazolyl, 1,3,4-oxadiazolyl, 1,3,4-thiadiazolyl, tetrazolyl (e.g. 1-tetrazolyl, 2-tetrazolyl, 5-tetrazolyl), quinolyl (e.g. 2-quinolyl, 3-quinolyl, 4-quinolyl, 5-quinolyl, 8-quinolyl), isoquinolyl (e.g. 1-isoquinolyl, 3-isoquinolyl, 4-isoquinolyl, 5-isoquinolyl), pyrazinyl, pyrimidinyl (e.g. 2-pyrimidinyl, 4-pyrimidinyl), pyrrolyl (e.g. 1-pyrrolyl, 2-pyrrolyl, 3-pyrrolyl), imidazolyl (e.g. 1-imidazolyl, 2-imidazolyl, 4-imidazolyl), pyrazolyl (e.g. 1-pyrazolyl, 3-pyrazolyl, 4-pyrazolyl), pyridazinyl (e.g. 3-pyridazinyl, 4-pyridazinyl), isothiazolyl (e.g. 3-isothiazolyl), isooxazolyl (e.g. 3-isooxazolyl), indolyl (e.g. 1-indolyl, 2-indolyl, 3-indolyl), 2-benzothiazolyl, benzo[b]thienyl (e.g. 2-benzo[b]thienyl, 3-benzo[b]thienyl), and benzo[b]furanyl (e.g. 2-benzo[b]furanyl, 3-benzo[b]furanyl), and non-aromatic heterocyclic groups such as pyrrolidinyl (e.g. 1-pyrrolidinyl, 2-pyrrolidinyl, 3-pyrrolidinyl), oxazolidinyl (e.g. 2-oxazolidinyl), imidazolinyl (e.g. 1-imidazolinyl, 2-imidazolinyl, 4-imidazolinyl), piperidinyl (e.g. 1-piperidinyl, 2-piperidinyl, 3-piperidinyl, 4-piperidinyl), piperazinyl (e.g. 1-piperazinyl, 2-piperazinyl), morpholino, thiomorpholino, and azepanyl are used.

As the substituent of the "optionally substituted cyclic group", the "optionally substituted cyclic hydrocarbon group" or the "optionally substituted heterocyclic group", the same substituent as the substituent A group of ring Xb or ring X is used.

As the substituent represented by Q¹, for example, an "optionally substituted hydrocarbon group", an "optionally substituted heterocyclic group" and an "acyl group" are used.

As the "hydrocarbon group" of the "optionally substituted hydrocarbon group", for example, a chain or cyclic hydrocarbon group (e.g. alkyl, alkenyl, alkynyl, cycloalkyl, aryl, aralkyl) is used. Inter alia, a chain or cyclic hydrocarbon group of a carbon number of 1 to 19 is preferable.

As the "alkyl", for example, a C₁₋₆ alkyl group (e.g. methyl, ethyl, propyl, isopropyl, butyl, isobutyl, sec-butyl, tert-butyl, pentyl, hexyl) is preferable.

As the "alkenyl", for example, a C₂₋₆ alkenyl group (e.g. vinyl, allyl, isopropenyl, 1-butenyl, 2-butenyl, 3-butenyl, 2-methyl-2-propenyl, 1-methyl-2-propenyl, 2-methyl-1-propenyl) is preferable.

As the "alkynyl", for example, C₂₋₆ alkynyl (e.g. ethynyl, propargyl, 1-butynyl, 2-butynyl, 3-butynyl, 1-hexynyl) is preferable.

As the "cycloalkyl group", for example, a C₃₋₈ cycloalkyl group (e.g. cyclopropyl, cyclobutyl, cyclopentyl, cyclohexyl) is used.

As the "aryl group", for example, a C₆₋₁₄ aryl group (e.g. phenyl, 1-naphtyl, 2-naphthyl, 2-anthryl) is used.

As the "aralkyl group", for example, a C₇₋₁₉ aralkyl group (e.g. benzyl, phenethyl, diphenylmethyl, 1-naphthylmethyl, 2-naphthylmethyl, 2, 2-diphenylethyl, 3-phenylpropyl, 4-phenylbutyl, 5-phenylpentyl, 2-biphenylylmethyl, 3-biphenylylmethyl, 4-biphenylylmethyl, trityl) is used.

As the substituent of the "hydrocarbon group", the same substituents as those of the substituent A group of ring Xb or ring X are used.

As the "optionally substituted heterocyclic group", the same group as the "optionally substituted heterocyclic group" of the substituent A group is used.

As the "acyl group", the same group as the acyl group represented by Q² described later is used.

As the acyl group represented by Q², for example, an acyl group represented by the formula: -(C=O)-R⁴, -(C=O)-OR⁴, -(C=O)-NR⁴R⁵, -(C=S)-NHR⁴, -(C=O)-N(OR⁴)R⁵, -(C=S)-NHOR⁴ or -SO₂-R⁶ (wherein R⁴ represents a hydrogen atom, an optionally substituted hydrocarbon group or an optionally substituted heterocyclic group, R⁵ represents a hydrogen atom or a lower (C₁₋₆)alkyl group, and R⁶ represents an optionally substituted hydrocarbon group or an optionally substituted heterocyclic group) is used.

As the "optionally substituted hydrocarbon group" represented by R⁴ and R⁶, the same group as the "optionally substituted hydrocarbon group" represented by Q¹ is used.

As the "optionally substituted heterocyclic group" represented by R⁴ and R⁶, the same group as the "optionally substituted heterocyclic group" represented by Q¹ is used.

As the "lower (C₁₋₆)alkyl group" represented by R⁵, for example, methyl, ethyl, propyl, isopropyl, butyl, isobutyl, sec-butyl, tert-butyl, pentyl, isopentyl, neopentyl, and hexyl are used.

As the "optionally substituted cyclic group" represented by A, the same group as the "optionally substituted cyclic group" represented by Y is used.

Specifically, as the "optionally substituted cyclic group" represented by A, (i) a C₃₋₈ cycloalkyl group (e.g. cyclopropyl, cyclohexyl), (ii) a C₆₋₁₄ aryl group (e.g. phenyl) or (iii) a 5- to 10-membered aromatic heterocyclic group containing one or two kinds of 1 to 4 hetero atoms selected from a nitrogen atom, a sulfur atom and an oxygen atom in addition to carbon atoms (e.g. pyridinyl, pyrimidinyl, pyrazinyl, pyridazinyl, furyl, thienyl, thiazolyl, oxazolyl, pyrrolyl, pyrazolyl, imidazolyl), which may be substituted with a substituent selected from the group consisting of a halogen atom, cyano, C₁₋₆ alkyl optionally substituted with a halogen atom (e.g. methyl, ethyl, trifluoromethyl etc.), C₁₋₆alkoxy optionally substituted with a halogen atom (e.g. methoxy, ethoxy, trifluoromethoxy etc.), C₁₋₆ alkyl-carbonylamino (e.g. acetylamino, ethylcarbonylamino etc.), a 5- to 6-membered aromatic heterocyclic group (e.g. 5- or 6-membered aromatic heterocyclic group containing one or two kinds of 1 to 4 hetero atoms selected from a nitrogen atom, a sulfur atom and an oxygen atom in addition to carbon atoms, such as pyridinyl, pyrimidinyl, pyrazinyl, pyridazinyl, furyl, thienyl, thiazolyl, oxazolyl, pyrrolyl, pyrazolyl, and imidazolyl) and C₁₋₆ alkylthio is used. Inter alia, a C₆₋₁₄ aryl group (e.g. phenyl) optionally substituted with a substituent selected from the group consisting of a halogen atom, a cyano, C₁₋₆ alkyl optionally substituted with a halogen atom, C₁₋₆ alkoxy optionally substituted with a halogen atom, C₁₋₆ alkyl-carbonylamino, a 5- or 6-membered aromatic heterocyclic group and C₁₋₆alkylthio is preferable, and a C₆₋₁₄ aryl group (e.g. phenyl) optionally substituted with a halogen atom is particularly preferable.

Examples of the "aromatic heterocyclic group" of the "optionally substituted aromatic heterocyclic group" represented by A' include a 5- to 14-membered (monocyclic, dicyclic or tricyclic) aromatic heterocyclic group containing one or two kinds of 1 to 4 hetero atoms selected from a nitrogen atom, a sulfur atom and an oxygen atom in addition to carbon atoms, preferably, (i) a 5- to 14-membered (preferably 5- to 10-membered) aromatic heterocyclic group, and (ii) a monovalent group obtained by removing one arbitrary hydrogen atom from a 7- to 10-membered aromatic heterocyclic crosslinked ring. Inter alia, a monocyclic aromatic heterocyclic group is preferable. Specifically, for example, thienyl (e.g. 2-thienyl, 3-thienyl), furyl (e.g. 2-furyl, 3-furyl), pyridyl (e.g. 2-pyridyl, 3-pyridyl, 4-pyridyl), thiazolyl (e.g. 2-thiazolyl, 4-thiazolyl, 5-thiazolyl), oxazolyl (e.g. 2-oxazolyl, 4-oxazolyl, 5-oxazolyl), 1,2,4-triazolyl, 1,2,3-triazolyl, 1,3,4-oxadiazolyl, 1,3,4-thiadiazolyl, tetrazolyl (e.g. 1-tetrazolyl, 2-tetrazolyl, 5-tetrazolyl), pyrazinyl, pyrimidinyl, (e.g. 2-pyrimidinyl, 4-pyrimidinyl), pyrrolyl (e.g. 1-pyrrolyl, 2-pyrrolyl, 3-pyrrolyl), imidazolyl (e.g. 1-imidazolyl, 2-imidazolyl, 4-imidazolyl), pyrazolyl (e.g. 1-pyrazolyl, 3-pyrazolyl, 4-pyrazolyl), pyridazinyl (e.g. 3-pyridazinyl, 4-pyridazinyl), isothiazolyl (e.g. 3-isothiazolyl), and isooxazolyl (e.g. 3-isooxazolyl) are used. Inter alia, thienyl (e.g. 2-thienyl) and furyl (e.g. 2-furyl) are preferable.

As A', a phenyl group, a thienyl group (e.g. 2-thienyl, 3-thienyl), furyl (e.g. 2-furyl, 3-furyl) and pyridyl (e.g. 2-pyridyl, 4-pyridyl) are preferable, and a phenyl group is particularly suitable.

As the substituent of the "phenyl group" or the "aromatic heterocyclic group" represented by A', substituents selected from the substituent A group are used. Inter alia, a halogen atom, cyano, C₁₋₆ alkyl optionally substituted with a halogen atom, C₁₋₆ alkoxy optionally substituted with a halogen atom, C₁₋₆ alkyl-carbonylamino, a 5- or 6-membered aromatic heterocyclic group, and C₁₋₆ alkylthio are preferable, and a halogen atom (e.g. fluorine atom, chlorine atom, bromine atom) is particularly preferable.

A and A' are preferably unsubstituted. As A and A', an unsubstituted phenyl group is particularly preferable.

As the substituent of the "optionally substituted phenyl group" represented by B, substituents selected from the substituent A group are used. Inter alia, a halogen atom (e.g. fluorine atom, chlorine atom, bromine atom), and C₁₋₆ alkyl (e.g. methyl, ethyl, propyl) are preferable, and fluorine, chlorine, bromine and methyl are particularly suitable. As a position of a substituent, an ortho position or a meta position is preferable, and an ortho position is particularly preferable. As B, an unsubstituted phenyl group is preferable.

As the substituent of the piperidine ring represented by ring D, the same substituents as those of the substituent A group are used. Inter alia, a C₁₋₆ alkyl group (e.g. methyl) is preferable.

As an optionally substituted methylene group represented by Z, for example, a methylene group optionally substituted with the substituent A group is used. Inter alia, a methylene group optionally substituted with a substituent selected from the group consisting of C₁₋₆ alkyl (e.g. methyl), C₁₋₆ alkoxycarbonyl (e.g. methoxycarbonyl), oxo and phenyl is preferable, a methylene group optionally substituted with C₁₋₆ alkyl is particularly preferable, and an unsaturated methylene group is most preferable.

As Z¹, a methylene group optionally substituted with a substituent selected from the group consisting of C₁₋₆ alkyl (e.g. methyl), C₁₋₆ alkoxycarbonyl (e.g. methoxycarbonyl), oxo and phenyl is preferable. Inter alia, a methylene group optionally substituted with C₁₋₆ alkyl is preferable, and a methylene group is particularly preferable.

As the "optionally substituted lower alkyl group" represented by R¹, the same group as the "optionally substituted lower alkyl group" of the substituent A group is used.

As the "lower alkyl group optionally substituted with a lower alkoxy group" represented by R^{1a}, for example, a C₁₋₆ alkyl group (e.g. methyl, ethyl, propyl, isopropyl, butyl, isobutyl, sec-butyl, tert-butyl, pentyl, hexyl) optionally substituted with methoxy, ethoxy, propoxy, isopropoxy, butoxy, isobutoxy, sec-butoxy, pentyloxy or hexyloxy is used. Inter alia, a C₁₋₆ alkyl group is preferable.

As the "optionally substituted phenyl group" represented by R¹ or R^{1a}, a phenyl group optionally substituted with a substituent selected from a substituent A group is used and, inter alia, an unsubstituted phenyl group is preferable.

As the "optionally substituted aromatic heterocyclic group" represented by R¹, a 5- to 14-membered (monocyclic, dicyclic or tricyclic) aromatic heterocyclic group containing one or two kinds of 1 to 4 hetero atoms selected from a nitrogen atom, a sulfur atom and an oxygen atom in addition to carbon atoms, which may be substituted with a substituent selected from a substituent A group is used. Inter alia, a 5- or 6- membered aromatic heterocyclic group is preferably used. Specifically, for example, thienyl (e.g. 2-thienyl, 3-thienyl), furyl (e.g. 2-furyl, 3-furyl), pyridyl (e.g. 2-pyridyl, 3-pyridyl, 4-pyridyl), thiazolyl (e.g. 2-thiazolyl, 4-thiazolyl, 5-thiazolyl), oxazolyl (e.g. 2-oxazolyl, 4-oxazolyl, 5-oxazolyl), 1,2,4-triazolyl, 1,2,3-triazolyl, 1,3,4-oxadiazolyl, 1,3,4-thiadiazolyl, quinolyl (e.g. 2-quinolyl, 3-quinolyl, 4-quinolyl, 5-quinolyl, 8-quinolyl), isoquinolyl (e.g. 1-isoquinolyl, 3-isoquinolyl, 4-isoquinolyl, 5-isoquinolyl), pyrazinyl, pyrimidinyl (e.g. 2-pyrimidinyl, 4-pyrimidinyl), pyrrolyl (e.g. 1-pyrrolyl, 2-pyrrolyl, 3-pyrrolyl), imidazolyl (e.g. 1-imidazolyl, 2-imidazolyl, 4-imidazolyl), pyrazolyl (e.g. 1-pyrazolyl, 3-pyrazolyl, 4-pyrazolyl), pyridazinyl (e.g. 3-pyridazinyl, 4-pyridazinyl), isothiazolyl (e.g. 3-isothiazolyl), isooxazolyl (e.g. 3-isooxazolyl), indolyl (e.g. 1-indolyl, 2-indolyl, 3-indolyl), 2-benzothiazolyl, benzo[b]thienyl (e.g. 2-benzo[b]thienyl, 3-benzo[b]thienyl, and benzo[b]furanyl (e.g. 2-benzo[b]furanyl, 3-benzo[b]furanyl) are used. In particular, a thiazolyl group (e.g. 2-thiazolyl), an imidazolyl group (e.g. 2-imidazolyl) and a pyridyl group (e.g. 2-pyridyl) are preferable.

As the "optionally substituted 5-membered aromatic heterocyclic group" represented by R^{1a}, a 5-membered aromatic heterocyclic group containing one or two kinds of 1 to 4 hetero atoms selected from a nitrogen atom, a sulfur atom and an oxygen atom in addition to carbon atoms, which may be substituted with a substituent selected from the substituent A group is used. Specifically, for example, thienyl (e.g. 2-thienyl, 3-thienyl), furyl (e.g. 2-furyl, 3-furyl), thiazolyl (e.g. 2-thiazolyl), oxazolyl (e.g. 2-oxazolyl), 1,2,4-triazolyl-3-yl, 1,2,3-triazolyl-4-yl, 1,3,4-oxadiazolyl (e.g. 1,3,4-oxadiazol-2-yl), 1,3,4-thiadiazolyl (e.g. 1,3,4-thiadiazol-2-yl), pyrrolyl (e.g. 2-pyrrolyl, 3-pyrrolyl), pyrazolyl (e.g. 3-pyrazolyl), imidazolyl (e.g. 2-imidazolyl), isothiazolyl (e.g. 3-isothiazolyl), and isooxazolyl (e.g. 3-isooxazolyl) are used. As a substituent, C₁₋₆ alkyl (e.g. methyl, ethyl, propyl) is preferable.

Specifically, as the "optionally substituted aromatic heterocyclic group" represented by R¹, (i) a thiazolyl group optionally substituted with C₁₋₆ alkyl (e.g. methyl), (ii) an imidazolyl group optionally substituted with C₁₋₆ alkyl (e.g. methyl), and (iii) a pyridyl group optionally substituted with a substituent selected from the group consisting of C₁₋₆ alkyl (e.g. methyl), a halogen atom (e.g. chlorine atom), C₁₋₆ alkylthio (e.g. methylthio), phenyl and thienyl are preferable.

As the "optionally substituted 5-membered aromatic heterocyclic group" represented by R^{1a}, a thiazolyl group (e.g. 2-thiazolyl), an imidazolyl group (e.g. 2-imidazolyl) or a pyridyl group (e.g. 2-pyridyl), which may be substituted with C₁₋₆ alkyl (e.g. methyl, ethyl, propyl) are preferable. Inter alia, a thiazolyl group (e.g. 2-thiazolyl) optionally substituted with C₁₋₆ alkyl (e.g. methyl, ethyl, propyl) is preferable, and a 4-methyl-2-thiazolyl group is particularly preferable.

As the pyridyl group represented by R^{1a}, a 2-pyridyl group, a 3-pyridyl group, and a 4-pyridyl group are used. Inter alia, a 2-pyridyl group is preferable.

As the "optionally esterified carboxyl group" represented by R¹ or R^{1a}, the same group as the "optionally esterified carboxyl group" of the substituent A group is used. Inter alia, a carboxyl group, a C₁₋₆ alkoxy-carbonyl group (e.g. methoxycarbonyl, ethoxycarbonyl, propoxycarbonyl, tert-butoxycarbonyl etc.), and a C₇₋₁₆ aralkyloxy-carbonyl group (e.g. benzyloxycarbonyl, phenethyloxycarbonyl etc.) are preferable.

As the "optionally substituted carbamoyl group" represented by R¹ or R^{1a}, the same group as the "optionally substituted carbamoyl group" of the substituent A group is used. Inter alia, carbamoyl, mono-C₁₋₆ alkyl-carbamoyl (e.g. methylcarbamoyl, ethylcarbamoyl), C₁₋₆ alkyl(C₁₋₆ alkoxy)-carbamoyl (e.g. methyl(methoxy)carbamoyl, ethyl(methoxy)carbamoyl), and 5- to 7-membered cyclic carbamoyl (e.g. 1-pyrrolidinylcarbonyl, 1-piperidinylcarbonyl, hexamethyleneiminocarbonyl) are preferable.

As R¹, an optionally substituted phenyl group, an optionally substituted aromatic heterocyclic group or an optionally esterified carboxyl group is preferable. As the optionally substituted aromatic heterocyclic group, an optionally substituted 5-membered aromatic heterocyclic group (e.g. thiazolyl group), and an optionally substituted pyridyl group are preferable. A 2-thiazolyl group optionally substituted with lower (C₁₋₆)alkyl, and a 2-pyridyl group optionally substituted with lower (C₁₋₆)alkyl are particularly preferable. Specifically, as R¹, (i) a hydrogen atom (ii) a cyano group, (iii) a C₁₋₅ alkyl group optionally substituted with a 5- or 6-membered aromatic heterocyclic group (e.g. 5- or 6-membered aromatic heterocyclic group containing one or two kinds of 1 to 4 hetero atoms selected from a nitrogen atom, a sulfur atom and an oxygen atom, such as pyridyl), (iv) a phenyl group, (v) a thiazolyl group (e.g. 2-thiazolyl group) optionally substituted with C₁₋₆ alkyl, (vi) an imidazolyl group optionally substituted with C₁₋₆ alkyl, (vii) a pyridyl group (e.g. 2-pyridyl group) optionally substituted with a substituent selected from the group consisting of C₁₋₆ alkyl (e.g. methyl), a halogen atom (e.g. chlorine atom, bromine atom), C₁₋₆ alkylthio (e.g. methylthio), phenyl and thienyl, (viii) a carboxyl group, (ix) a C₁₋₆ alkoxy-carbonyl group (e.g. methoxycarbonyl, ethoxycarbonyl), (x) a C₇₋₁₆ aralkyloxy-carbonyl group (e.g. benzyloxycarbonyl), (xi) a carbamoyl group, (xii) a mono-C₁₋₆ alkyl-carbamoyl group (e.g. methylcarbamoyl, ethylcarbamoyl, propylcarbamoyl), (xiii) a C₁₋₆ alkyl (C₁₋₆ alkoxy)-carbamoyl group (e.g. methyl(methoxy)carbamoyl), and (xiv) a 5- to 7-membered cyclic carbamoyl group are preferable, and a 4-methyl-2-thiazolyl group is particularly preferable.

Specifically, as R^{1a}, a hydrogen atom, a cyano group, a C₁₋₆ alkyl group, a phenyl group, a thiazolyl group (e.g. 2-thiazolyl) optionally substituted with C₁₋₆ alkyl (e.g. methyl, ethyl, propyl), an optionally substituted imidazolyl group (e.g. 2-imidazolyl), an optionally substituted pyridyl group (e.g. 2-pyridyl), a carboxyl group, a C₁₋₆ alkoxy-carbonyl group (e.g. methoxycarbonyl, ethoxycarbonyl, propoxycarbonyl, tert-butoxycarbonyl), a C₇₋₁₆ aralkyloxy-carbonyl group (e.g. benzyloxycarbonyl, phenethyloxycarbonyl), a carbamoyl group, a mono-C₁₋₆ alkyl-carbamoyl group (e.g. methylcarbamoyl, ethylcarbamoyl), a C₁₋₆ alkyl(C₁₋₆ alkoxy)-carbamoyl group (e.g. methyl(methoxy)carbamoyl, ethyl(methoxy)carbamoyl), and a 5- to 7-membered cyclic carbamoyl group (e.g. 1-pyrrolidinylcarbonyl, 1-piperidinylcarbonyl, hexamethyleneiminocarbonyl) are used.

As R^{1a}, an optionally substituted phenyl group, an optionally substituted 5-membered aromatic heterocycle, an optionally substituted pyridyl group, and an optionally esterified carboxyl group are preferable. Specifically, a phenyl group, a thiazolyl group (e.g. 2-thiazolyl) optionally substituted with C₁₋₆ alkyl (e.g. methyl, ethyl, propyl), an imidazolyl group (e.g. 2-imidazolyl) optionally substituted with C₁₋₆ alkyl (e.g. methyl, ethyl, propyl), a pyridyl group (e.g. 2-pyridyl) optionally substituted with C₁₋₆ alkyl (e.g. methyl, ethyl, propyl), a carboxyl group, a C₁₋₆ alkoxy-carbonyl group (e.g. methoxycarbonyl, ethoxycarbonyl, propoxycarbonyl, tert-butoxycarbonyl), and a C₇₋₁₆ aralkyloxy-carbonyl group (e.g. benzyloxycarbonyl), phenethyloxycarbonyl) are preferable.

As "optionally substituted lower alkyl group", the "optionally substituted lower alkenyl group", the "optionally substituted lower alkoxy group" and the "optionally substituted phenyl group" represented by R², the same groups as the "optionally substituted lower alkyl group", the "optionally substituted lower alkenyl group", the "optionally substituted lower alkoxy group" and the "optionally substituted phenyl group" of the substituent A group, respectively, are used.

As the "optionally substituted aralkyloxy group" represented by R², the same group as the "optionally substituted C₇₋₁₆ aralkyloxy group" of the substituent A group is used.

As R², an optionally halogenated C₁₋₆ alkyl group, a C₂₋₆ alkenyl group, a C₁₋₆ alkoxy group, a C₇₋₁₁ aralkyloxy group, and a phenyl group are preferable. Inter alia, an optionally halogenated methyl group or ethyl group is preferable, and methyl and trifluoromethyl are particularly preferable.

As the lower alkyl group represented by R^{2a}, a C₁₋₆ alkyl group such as methyl, ethyl, propyl, isopropyl, butyl, isobutyl, sec-butyl, tert-butyl, pentyl, and hexyl is used. Inter alia, a C₁₋₃ alkyl group such as methyl, ethyl and propyl is preferable, and methyl and ethyl are particularly preferable.

Examples of the combination of A and R¹ include:
(1) A is an optionally substituted phenyl group, and R¹ is an optionally substituted aromatic heterocyclic group or an optionally esterified carboxyl group,
(2) A is an optionally substituted phenyl group, and R¹ is an optionally substituted 5- or 6-membered nitrogen-containing aromatic heterocyclic group (e.g. thiazolyl, imidazolyl, pyridyl)
(3) A is an optionally substituted phenyl group, and R¹ is an optionally substituted 5-membered aromatic heterocyclic group, a pyridyl group or an optionally esterified carboxyl group,
(4) A is an optionally substituted phenyl group, and R¹ is an optionally substituted 5-membered aromatic heterocyclic group,
(5) A is an optionally substituted phenyl group, and R¹ is a 2-thiazolyl group optionally substituted with a lower alkyl group,
(6) A is an optionally substituted phenyl group, and R¹ is a 2-pydiryl group optionally substituted with a lower alkyl group, and
(7) A is an optionally substituted phenyl group, and R¹ is a 4-methyl-2-thiazolyl group.

As Compound (I"), a compound in which A is (i) a C₃₋₈ cycloalkyl group, (ii) a C₆₋₁₄ aryl group or (iii) a 5- to 10-membered aromatic heterocyclic group containing one or two kinds of 1 to 4 hetero atoms selected from a nitrogen atom, a sulfur atom and an oxygen atom in addition to carbon atoms, which may be substituted with a substituent selected from the group consisting of a halogen atom, cyano, C₁₋₆ alkyl optionally substituted with a halogen atom, C₁₋₆ alkoxy optionally substituted with a halogen atom, C₁₋₆ alkyl-carbonylamino, a 5- to 6-membered aromatic heterocyclic group (e.g. 5- or 6-membered aromatic heterocyclic group containing one or two kinds of 1 to 4 hetero atoms selected from a nitrogen atom, a sulfur atom and an oxygen atom in addition to carbon atoms) and C₁₋₆ alkylthio, B is a phenyl group optionally substituted with a substituent selected from the group consisting of a halogen atom and C₁₋₆ alkyl, ring D is a piperidine ring optionally further substituted with C₁₋₆ alkyl, Z is a methylene group optionally substituted with a substituent selected from the group consisting of C₁₋₆ alkyl, C₁₋₆ alkoxycarbonyl, oxo and phenyl, -COCH₂-, -CH₂CO- or -SO₂-, R¹ is (i) a hydrogen atom, (ii) a cyano group, (iii) a C₁₋₆ alkyl group optionally substituted with a 5- or 6-membered aromatic heterocyclic group, (iv) a phenyl group, (v) a thiazolyl group optionally substituted with C₁₋₆ alkyl, (vi) an imidazolyl group optionally substituted with C₁₋₆ alkyl, (vii) a pyridyl group optionally substituted with a substituent selected from the group consisting of C₁₋₆ alkyl, a halogen atom C₁₋₆ alkylthio, phenyl and thienyl, (viii) a carboxyl group, (ix) C₁₋₆ alkoxy-carbonyl group, (x) a C₇₋₁₆ aralkyloxy-carbonyl group, (xi) a carbamoyl group, (xii) a mono-C₁₋₆ alkyl-carbamoyl group, (xiii) a C₁₋₆ alkyl(C₁₋₆ alkoxy)-carbamoyl group, or (xiv) a 5- to 7-membered cyclic carbamoyl group, and R² is an optionally halogenated C₁₋₆ alkyl group, a C₂₋₆ alkenyl group, a C₁₋₆ alkoxy group, a C₇₋₁₁ aralkyloxy group or a phenyl group is preferable. Specifically, compounds of Reference Examples 1 to 102 described hereinafter are used.

Among the compounds of the present invention, compound (I'''') represented by the formula: 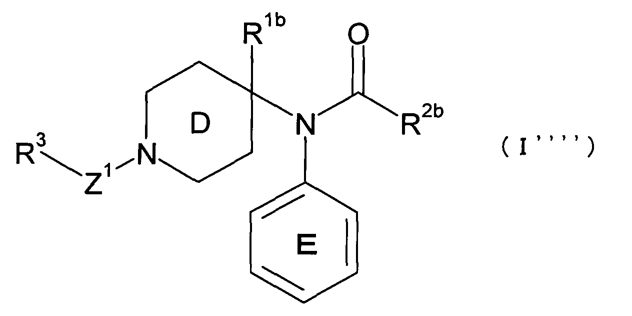 wherein R^{1b} represents an optionally substituted 2-thiazolyl group, an optionally substituted 2-imidazolyl group or an optionally substituted 2-pyridyl group, R^{2b} represents an optionally halogenated lower alkyl group, R³ represents an optionally substituted phenyl group, an optionally substituted aromatic heterocyclic group or an optionally substituted cycloalkyl group, ring D, ring E and Z¹ are as defined above, provided that N-[1-benzyl-4-(4-methylthiazol-2-yl)-4-piperidinyl]-N-phenylpropionamide and N-[1-benzyl-4-(2-pyridinyl)-4-piperidinyl]-N-phenylpropionamide are excluded, is a novel compound.

As the substituent of the "optionally substituted 2-thiazolyl group", the "optionally substituted 2-imidazolyl group" or the "optionally substituted 2-pyridyl group" represented by R^{1b}, the same substituents as those of the substituent A group are used. Inter alia, lower (C₁₋₆)alkyl (e.g. methyl), lower (C₁₋₆)alkylthio (e.g. methylthio), a halogen atom, C₆₋₁₄ aryl (e.g. phenyl), and an aromatic heterocyclic group (e.g. 5- or 6-membered aromatic heterocyclic group containing one or two kinds of 1 to 4 hetero atoms selected from a nitrogen atom, a sulfur atom and an oxygen atom in addition to carbon atoms, such as thienyl) are preferable, and lower (C₁₋₆)alkyl (e.g. methyl) is particularly preferable.

Specifically, as R^{1b},
(1) a 2-thiazolyl group optionally substituted with lower (C₁₋₆)alkyl (e.g. methyl) (particularly, 4-methyl-2-thiazolyl group),
(2) a 2-imidazolyl group optionally substituted with lower (C₁₋₆)alkyl (e.g. methyl),
(3) a 2-pyridyl group optionally substituted with a substituent selected from the group consisting of lower (C₁₋₆)alkyl (e.g. methyl), lower (C₁₋₆)alkylthio (e.g. methylthio), a halogen atom, C₆₋₁₄ aryl (e.g. phenyl) and an aromatic heterocyclic group (e.g. 5- or 6-membered aromatic heterocyclic group containing one or two kinds of 1 to 4 hetero atoms selected from a nitrogen atom, a sulfur atom and an oxygen atom in addition to carbon atoms, such as thienyl), are preferable.

As the lower (C₁₋₆)alkyl group of the "optionally halogenated lower alkyl group" represented by R^{2b}, for example, methyl, ethyl, propyl, isopropyl, butyl, isobutyl, sec-butyl, tert-butyl, pentyl, isopentyl, neopentyl and hexyl are used. As a halogen atom of a substituent, a fluorine atom, a chlorine atom, a bromine atom, and an iodine atom are used. Inter alia, a fluorine atom is preferable.

As R^{2b}, an optionally halogenated methyl group or ethyl group is preferable, and a methyl group or a trifluoromethyl group is particularly preferable.

As the "optionally substituted phenyl group" and the "optionally substituted aromatic heterocyclic group" represented by R³, the same groups as the "optionally substituted phenyl group" and the "optionally substituted aromatic heterocyclic group" represented by A' are used.

As the "optionally substituted cycloalkyl group" represented by R³, a C₃₋₈ cycloalkyl group (e.g. cyclopropyl, cyclobutyl, cyclopentyl, cyclohexyl) optionally substituted with a substituent selected from the substituent A group is used.

Specifically, as R³, (i) a C₃₋₈ cyloalkyl group, (ii) a phenyl group or (iii) a 5- to 10-membered aromatic heterocyclic group containing one or two kinds of 1 to 4 hetero atoms selected from the nitrogen atom, a sulfur atom and an oxygen atom in addition to carbon atoms, which may be substituted with a substituent selected from the group consisting of a halogen atom, cyano, C₁₋₆ alkyl optionally substituted with a halogen atom, C₁₋₆ alkoxy optionally substituted with a halogen atom, C₁₋₆ alkyl-carbonylamino, a 5- or 6-membered aromatic heterocyclic group and C₁₋₆ alkylthio is preferable. Inter alia, a phenyl group optionally substituted with a substituent selected from the group consisting of a halogen atom, cyano, C₁₋₆ alkyl optionally substituted with a halogen atom, C₁₋₆ alkoxy optionally substituted with a halogen atom, C₁₋₆ alkyl-carbonylamino, a 5- or 6-membered aromatic heterocyclic group and C₁₋₆ alkylthio is preferable.

In addition, as R³, an optionally substituted phenyl group or an optionally substituted thienyl group is preferable. Inter alia, a phenyl group is preferable.

As a substituent of ring D, a lower alkyl group (e.g. methyl) is used.

As the substituent of the "optionally substituted phenyl group" represented by E, substituents selected from the substituent A group are used. Inter alia, a halogen atom (e.g. fluorine atom, chlorine atom, bromine atom) and C₁₋₆ alkyl (e.g. methyl, ethyl, propyl) are preferable, and fluorine, chlorine, bromine and methyl are particularly preferable.

As E, a phenyl group optionally having a substituent at an ortho position or a meta position, particularly an ortho position is preferable, and an unsubstituted phenyl group is particularly preferable.

As Z¹, a methylene group optionally substituted with a lower alkyl group is preferable, and a methylene group is particularly preferable.

As Compound (I''''), a compound in which ring D is a piperidine ring optionally substituted with C₁₋₆ alkyl, E is a phenyl group optionally substituted with a substituent selected from the group consisting of a halogen atom and C₁₋₆ alkyl, Z¹ is a methylene group optionally substituted with a substituent selected from the group consisting of C₁₋₆ alkyl, C₁₋₆ alkoxycarbonyl, oxo and phenyl, -COCH₂- or - SO₂-, R^{1b} is (i) a 2-thiazolyl group optionally substituted with C₁₋₆ alkyl, (ii) a 2-imidazolyl group optionally substituted with C₁₋₆ alkyl, or (iii) a 2-pyridyl group optionally substituted with a substituent selected from the group consisting of C₁₋₆ alkyl, a halogen atom, C₁₋₆ alkylthio, phenyl and thienyl, R^{2b} is an optionally halogenated C₁₋₆ alkyl group, and R³ is (i) a C₃₋₈ cycloalkyl group, (ii) a phenyl group or (iii) a 5- to 10-membered aromatic heterocyclic group containing one or two kinds of 1 to 4 hetero atoms selected from a nitrogen atom, a sulfur atom and an oxygen atom in addition to carbon atoms, which may be substituted with a substituent selected from the group consisting of a halogen atom, cyano, C₁₋₆ alkyl optionally substituted with a halogen atom, C₁₋₆ alkoxy optionally substituted with a halogen atom, C₁₋₆ alkyl-carbonylamino, a 5- or 6-membered aromatic heterocyclic group and C₁₋₆ alkylthio is preferable. Specifically, compounds of Reference Examples 33 to 37, 39, 42 to 50, 53 to 62, and 63B to 101 described hereinafter are used. Inter alia, N-[1-benzyl-4-(4-methylthiazol-2-yl)-4-piperidinyl]-N-phenylacetamide (Reference Examples 39, 44, 101), N-[1-benzyl-4-(4-methylthiazol-2-yl)-4-piperidinyl]-2,2,2-trifluoro-N-phenylacetamide (Reference Examples 84A, B), N-[1-benzyl-4-(6-methyl-2-pyridinyl)-4-piperidinyl]-N-phenylacetamide (Reference Example 53), N-[1-benzyl-4-(6-methyl-2-pyridinyl)-4-piperidinyl]-2,2,2-trifluoro-N-phenylacetamide (Reference Example 54), N-[1-(4-fluorobenzyl)-4-(4-methylthiazol-2-yl)-4-piperidinyl]-N-phenylacetamide (Reference Example 77), N-[1-benzyl-4-(4-methylthiazol-2-yl)-4-piperidinyl]-N-[2-methylphenyl]acetamide (Reference Example 80A, B), N-[1-benzyl-4-(4-methylthiazol-2-yl)-4-piperidinyl]-N-(3-chlorophenyl)acetamide (Reference Example 82A, B), N-[4-(4-methylthiazol-1-(2-thienylmethyl)-2-yl)-4-piperidinyl]-N-phenylacetamide (Reference Example 48), and N-[1-benzyl-4-(1-methyl-1H-imidazol-2-yl)-4-piperidinyl]-N-phenylacetamide (Reference Example 50) or a salt thereof are preferable.

Examples of the salt of the compound of the present invention include a metal salt, an ammonium salt, a salt with an organic base, a salt with an inorganic acid, a salt with an organic acid, and a salt with a basic or acidic amino acid, Preferable examples of a metal salt include an alkali metal salt such as a sodium salt, and a potassium salt; an alkaline earth metal salt such as a calcium salt, a magnesium salt, and a barium salt; an aluminum salt. Preferable examples of a salt with an organic base include salts with trimethylamine, triethylamine, pyridine, picoline, 2,6-lutidine, ethanolamine, diethanolamine, triethanolamine, cyclohexylamine, dicyclohexylamine, and N, N'-dibenzylethylenediamine. Preferable examples of a salt with an inorganic acid include salts with hydrochloric acid, hydrobromic acid, nitric acid, sulfuric acid, and phosphoric acid. Preferable examples of a salt with an organic salt include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, and p-toluenesulfonic acid. Preferable examples of a salt with a basic amino acid include salts with arginine, lysine, and ornithine, and preferable examples of a salt with an acidic amino acid include salts with aspartic acid, and glutamic acid.

Among them, pharmaceutically acceptable salts are preferable. For example, when an acidic functional group is possessed by the compound, inorganic salts such as an alkali metal salt (e.g. sodium salt, potassium salt etc.), and an alkaline earth metal salt (e.g. calcium salt, magnesium salt, barium salt), and an ammonium salt are preferable. In addition, when a basic functional group is possessed in the compound, salts with an inorganic acid such as hydrochloric acid, hydrobromic acid, nitric acid, sulfuric acid and phosphoric acid, or salts with organic acids such as acetic acid, phthalic acid, fumaric acid, oxalic acid, tartaric acid, maleic acid, citric acid, succinic acid, methanesulfonic acid, and p-toluenesulfonic acid are preferable.

A prodrug of the compounds (B), (I), (I'), (I"), (I'") and (I"") of the present invention or a salt thereof (hereinafter, abbreviated as Compound (I) of the present invention) refers to a compound which is converted into Compound (I) of the present invention by a reaction with an enzyme or stomach acid under the physiological conditions in a living body, that is, a compound which is changed into the compound of the present invention by enzymatic oxidation, reduction or hydrolysis, or a compound which is changed into Compound (I) of the present invention by hydrolysis with stomach acid.

Examples of the prodrug of Compound (I) of the present invention include a compound in which an amino group of Compound (I) of the present invention is acylated, alkylated or phosphorylated (e.g. a compound in which an amino group of Compound (I) of the present invention is eicosanoylated, alanylated, pentylaminocarbonylated, (5-methyl-2-oxo-1, 3-dioxolen-4-yl)methoxycarbonylated, tetrahydrofuranylated, pyrrolidylmethylated, pivaloyloxymethylated, or tert-butylated); a compound in which a hydroxyl group of Compound (I) of the present invention is acylated, alkylated, or borylated (e.g. a compound in which a hydroxyl group of Compound (I) of the present invention is acetylated, palmitoylated, propanoylated, pivaloylated, succinylated, fumarylated, alanylated, or dimethylaminomethylcarbonylated); a compound in which a carboxyl group of Compound (I) of the present invention is esterified, or amidated (e.g. a compound in which a carboxy group of Compound (I) of the present invention is ethylesterified, phenylesterified, carboxymethylesterified, dimethylaminomethylesterified, pivaloyloxymethylesterified, ethoxycarbonyloxyethylesterified, phthalidylesterified, (5-methyl-2-oxo-1, 3-dioxolen-4-yl)methylesterified, cyclohexyloxycarbonylethylesterified, or methylamidated). These compounds can be prepared from Compound (I) of the present invention by the known per se method.

Alternatively, a prodrug of Compound (I) of the present invention may be a compound which is changed into Compound (I) of the present invention under the physiological conditions as described in "Development of Medicines", vol.7, Molecular Design, pp.163-198 published in 1990 by Hirokawashoten.

The compound of the present invention can be prepared by using a per se known method, and Compound (I') can be prepared, for example, using the following process.

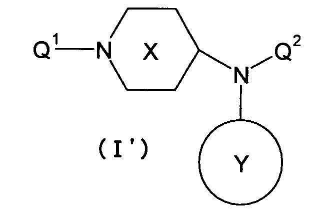

Compound (I') of the present invention can be easily prepared from the following known piperidone (II) by using a per se known reaction (e.g. reductive amination reaction, Strecker reaction, nucleophilic addition reaction of organic lithium agent to imine, acylating reaction of amine, reduction reaction, alkylation reaction to amine etc.) or the method described in a known publication (e.g. J. Med. Chem., vol.32, pp.2534-2542 published in 1989, Synlett, pp.1923-1924 published in 1999, USP 4,791,120 or USP 4,801,615).

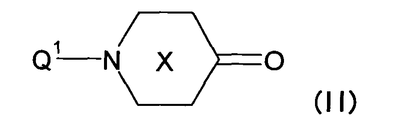

Compound (Ia) in which 4-position of piperidine is unsubstituted can be prepared, for example, by reacting cyclic amine with a known piperidone (II), reducing the resulting Schiff base (III), for example, using hydrogen catalytic reduction or a reducing agent (e.g. metal hydride reagent (e.g. sodium borohydride, sodium triacetoxyborohydride, sodium cyanoborohydride, lithium borohydride, aluminum lithium hydride etc.)) to obtain 4-aminopiperidine (IVa), and reacting the compound (IVa) with suitable acid halide or acid anhydride to introduce a suitable acyl group into the amino group.

In addition, Compound (Ib) in which 4-position of piperidine has a substituent R¹ of a phenyl group, an alkyl group or a heterocyclic group can be prepared, for example, by reacting Schiff base (III) with an organic nucleophilic agent [e.g. organic lithium agent R¹Li (e.g. phenyllithium, n-butyllithium, methyllithium, heterocycle lithium agent etc.), Grignard reagent (R¹MgBr or R¹MgCl etc.) etc.] to obtain 4-aminopiperidines (IVb), and acylating the compound (IVb). 4-Aminopiperidines (IVb) can be also prepared by subjecting the piperidone (II) to Strecker reaction using cyclic primary amine and a cyanating agent (e.g. alkali metal cyanide (e.g. sodium cyanide, potassium cyanide etc.), trimethylsilyl cyanide, prussic acid etc.), and reacting the resulting 4-cyano-4-aminopiperidines (V) with 2 equivalent of the aforementioned organic nucleophilic agent. In this case, Schiff base (III) as an intermediate is produced by the reaction of 4-cyano-4-aminopiperidines (V) and one equivalent of an organic nucleophilic agent.

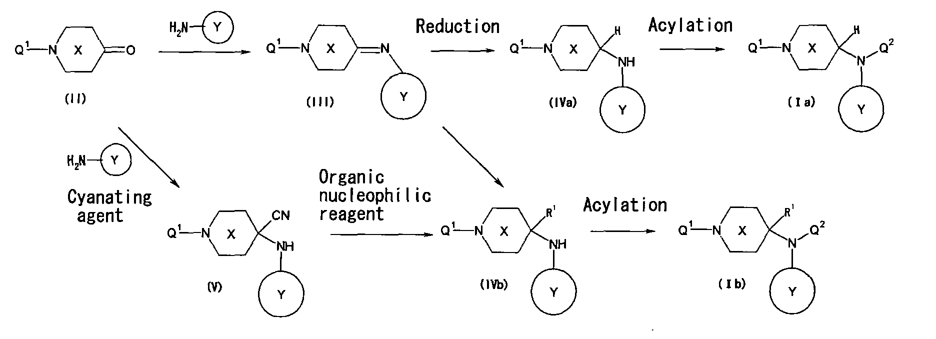

In addition, Compound (Ic-e) in which 4-position of piperidine has an optionally esterified carboxyl group or an optionally substituted carbamoyl group can be prepared by following methods. Hydrolyzing 4-cyano-4-aminopiperidines (V) using an acid such as sulfuric acid and hydrochloric acid and subsequent esterification of the resulting carboxylic acid (VI) by a per se known method (e.g. a method using an alcohol and an acid catalyst, a method of converting carboxylic acid into a sodium or potassium salt, and alkylating the salt with an alkylating reagent, a method of esterification with diazomethane) lead to Compound (IVc), which is acylated to obtain Compound (Ic). Alternatively, Compound (Id) can be prepared by reacting carboxylic acid (VI) with acid halide or acid anhydride to produce an intermediate (VII), and solvolyzing this using a suitable alcohol.

Compound (Ie) in which 4-position of piperidine as a carbamoyl group can be prepared by hydrolyzing an intermediate (VII) to obtain carboxylic acid (VIII), and reacting with various amides by the known per se amide bond forming reaction (e.g. a method using a condensing agent (e.g. carbodiimides such as DCC and WSC, phosphate reagents such as BOP-Cl, diethyl cyanophosphate, diphenyl phosphoryl azide (DPPA) etc.), and a method of converting carboxylic acid into a reactive derivative (e.g. acid halide, acid anhydride, mixed acid anhydride, active esters etc.), followed by reacting with amine, etc.).

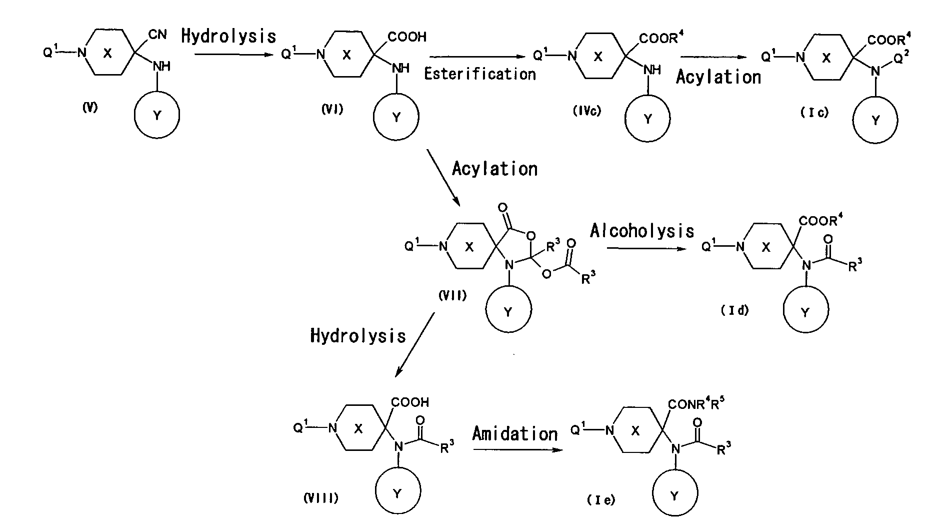

When it is difficult to obtain the piperidone (II) having desired Q¹ in the aforementioned reaction, Compound (I') can be prepared by preparing Compound (If) similarly using 1-benzyl-4-piperidone (IIa) in which Q¹ is a benzyl group, removing a benzyl group by a per se known debenzylating method (e.g. hydrogen catalytic reduction using a catalyst, a method of reacting with 1-chloroethyl chloroformate, followed by methanolysis), and reacting the resulting compound (IX) and a reactive compound Q¹-L [L represents a leaving group (e.g. a halogen atom such as chlorine, bromine, iodide etc.), a OSO₂R^{L} group (R^{L} represents an optionally halogenated alkyl group or an optionally substituted phenyl group) etc.] in the presence of a base (e.g. potassium carbonate, lithium carbonate, sodium carbonate, sodium bicarbonate, sodium hydroxide, potassium hydroxide, sodium hydride etc.).

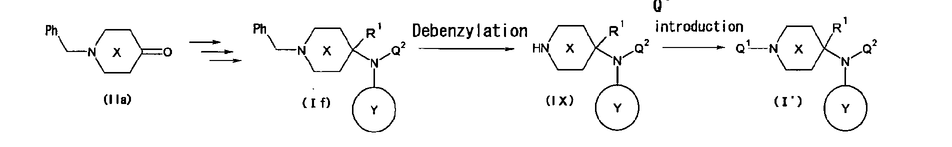

When an objective product is obtained in the free state by the aforementioned reaction, the product may be converted into a salt by a conventional method. In addition, when an objective product is obtained as a salt, the salt may be converted into a free compound or another salt according to a conventional method. The thus obtained compound of the present invention or a salt thereof can be isolated and purified from a reaction mixture by a known means such as transference dissolution, concentration, solvent extraction, fractional distillation, crystallization, recrystallization and chromatography.

When the compound of the present invention is present as a configurational isomer (arrangement isomer), a diastereomer or a conformer, each can be isolated by the aforementioned isolation and purification means, if desired. In addition, when the compound of the present invention is racemic modification, it can be separated into S-compound and R-compound by a normal optical resolution means.

When there are steric isomers in the compound of the present invention, this isomer alone, and a mixture of those isomers are also included in the present invention.

The compound of the present invention may be a hydrate or a non-hydrate.

The compound of the present invention may be labeled with an isotope element (e.g. ³H, ¹⁴C, ³⁵S).

Neuromedin U receptor function regulating action of the compound of the present invention can be measured using a method of screening a compound by changing binding of neuromedin U and FM-3 described in WO 00/02919 and WO 01/40797, or a method of screening a compound by changing binding of neuromedin U and TGR1 described in WO 01/57524.

Since the compound of the present invention has action of changing binding of neuromedin U and a neuromedin U receptor (e.g. FM-3, TGR-1, particularly FM-3), particularly, neuromedin U receptor antagonist activity, has low toxicity, and has little side effect, it is useful as a safe composition for regulating neuromedin U receptor function, preferably, as a neuromedin U receptor antagonist.

Since a pharmaceutical composition containing the compound of the present invention has excellent neuromedin U receptor function regulating action on a mammal (e.g. mouse, rat, hamster, rabbit, cat, dog, cow, sheep, monkey, human etc.), it is useful as a composition for regulating physiological function in which neuromedin U is involved, or as a preventive/therapeutic agent for morbid state or disease in which neuromedin U is involved. Specifically, a pharmaceutical composition containing the compound of the present invention can be used as a preventive/therapeutic agent for hypertension, cardiac infarct, acute renal dysfunction, or stress disease (e.g. (i) cardiac vascular disease (angina, cardiac infarct, arrhythmia etc.), (ii) respiratory disease (bronchial asthma, hyperpnea syndrome etc.), (iii) muscular skeletal disease (rheumatoid arthritis, lumbago, migraine, tension headache etc.), (iv) others (diabetes, climacteric disorder, chronic pain, immune activity reduction etc.)), or a digestive tract disease (stomach ulcer, ulcerative colitis etc.).

Further, since neuromedin U or a salt thereof has appetite regulating action, a pharmaceutical composition containing the compound of the present invention can be used as a composition for regulating an appetite, in particular, as a composition for promoting an appetite.

A pharmaceutical composition containing the compound of the present invention has low toxicity, and the compound of the present invention is formulated into pharmaceutical preparations such as tables (including sugar-coated tablets, film coating tablets), powders, granules, capsules (including soft capsules), solutions, injectables, suppositories and sustained-release agents by using the compound of the present invention as it is, or by mixing with a pharmacologically acceptable carrier according to a per se known means which is generally used for formulating pharmaceutical preparations, which can be safely administered orally or parenterally (e.g. local, rectal, intravenous administration etc.).

A content of the compound of the present invention in the preparation of the present invention is about 0.01 to about 100% by weight based on the whole preparation. A dose is different depending on an administration subject, an administration route, a disease and symptom and, for example, an active ingredient [the compound of the present invention] may be administered orally to a patient with hypertension (weight about 60 kg) at about 0.01 to about 30 mg/kg weight, preferably about 0.1 to about 20 mg/kg weight, further preferably about 1 to about 20 mg/kg weight per day once or a few times a day.

Examples of the pharmacologically acceptable carrier which may be used in preparation of the pharmaceutical composition of the present invention include various organic or inorganic carrier substances which are conventional as a preparation material, for example, excipents, lubricants, binders and disintegrating agents in a solid preparation, or solvents, solubilizers, suspending agents, isotonics, buffers and soothing agents in a liquid preparation. Further, if necessary, normal additives such as antiseptics, antioxidants, coloring agents, sweeteners, adsorbing agents and wetting agents may be appropriately used at an appropriate amount.

Examples of excipients include lactose, white sugar, D-mannitol, starch, corn starch, crystalline cellulose, and light silicic anhydride.

Examples of lubricants include magnesium stearate, potassium stearate, talc, and colloidal silica.

Examples of binders include crystalline cellulose, white sugar, D-mannitol, dextrin, hydroxypropylcellulose, hydroxypropylmethylcellulose, polyvinylpyrrolidone, starch, sucrose, gelatin, methylcellulose, sodium carboxymethylcellulose.

Examples of disintegrating agents include starch, carboxymethylcellulose, potassium carboxymethylcellulose, sodium carboxymethylstarch, and L-hydroxypropylcellulose.

Examples of solvents include water for injection, alcohol, propylene glycol, macrogol, sesame oil, corn oil, and olive oil.

Examples of solubilizers include polyethylene glycol, propylene glycol, D-mannitol, benzyl benzoate, ethanol, trisaminomethane, cholesterol, triethanolamine, sodium carbonate, and sodium citrate.

Examples of suspending agents include surfactants such as stearyltriethanolamine, sodium laurylsulfate, lauryl aminopropionate, lecithin, benzalkonium chloride, benzethonium chloride, and monostearic acid glycerin; hydrophilic polymers such as polyvinyl alcohol, polyvinylpyrrolidone sodium carboxymethylcellulose, methylcellulose, hydroxymethylcellulose, hydroxyethylcellulose, and hydroxypropylcellulose.

Examples of isotonics include glucose, D-sorbitol, sodium chloride, glycerin, and D-mannitol.

Examples of buffers include buffers such as phosphate, acetate, carbonate, and citrate.

Examples of soothing agents include benzyl alcohol.

Examples of antiseptics include parahydroxybenzoic acid esters, chlorobutanol, benzyl alcohol, phenethyl alcohol, dehydroacetic acid, and sorbic acid.

Examples of antioxidants include sulfite, ascorbic acid, and α-tocopherol.

Further, the compound of the present invention can be used together with drugs other than the compound of the present invention.

Examples of drugs which can be used with the compound of the present invention jointly (hereinafter, abbreviated as joint use drug) include other drugs for the aforementioned diseases, immune regulating drugs, anti-inflammatory drugs, antibacterial agents, anti-fungus drugs, anti-protozoan drugs, antibiotics, antitussive/expectorants, sedatives, anesthetics, anti-ulcer drugs, arrhythmia treating drugs, hypotensive diuretics, anti-coagulation drugs, tranquilizers, anti-psychosis drugs, anti-tumor drugs, anti-hyperlipemia drugs, muscular relaxants, anti-epilepsy drugs, antidepressants, anti-allergy drugs, cardiacs, arrhythmia treating drugs, vasodilators, angiotonics, hypotensive diuretics, diabetes treating drugs, narcotic antagonists, vitamin drugs, vitamin derivatives, anti-asthma drugs, pollakiuria/urine incontinence treating drugs, atopic dermatis treating drugs, allergic rhinitis treating drugs, pressors, endotoxin antagonists or antibiotics, signal transmission inhibitors, inflammatory mediator action inhibitors, inflammatory mediator action inhibiting antibiotics, anti-inflammatory mediator action inhibitors, and anti-inflammatory mediator action inhibiting antibiotics. Specifically, examples include the following.

By combining the compound of the present invention and the joint use drug, the following excellent effects can be obtained:
(1) A dose can be decreased as compared with administration of the compound of the present invention or the joint use drug alone.
(2) A drug which is used with the compound of the present invention jointly can be selected depending on symptom (slight, severe) of a patient.
(3) By selecting the joint use drug having different action mechanism from that of the compound of the present invention, a treating term can be set long.
(4) By selecting the joint use drug having the different action mechanism from that of the compound of the present invention, duration of treating effect can be realized.
(5) By using the compound of the present invention together with the joint use drug, synergistic effect can be obtained.

Hereinafter, joint use of Compound (I) of the present invention and the joint use drug is referred to as "joint use agent of the present invention".

Upon use of the joint use agent of the present invention, an administration time of the compound of the present invention and the joint use drug is not limited, but the compound of the present invention or a pharmaceutical composition thereof and the joint use drug or a pharmaceutical composition thereof may be administered to an administration subject simultaneously, or may be administered at different times. A dose of the joint use drug may be according to a dose which is clinically used, and may be appropriately selected depending on an administration subject, an administration route, a disease, and a combination.

An administration aspect of the joint use agent of the present invention is not particularly limited, and it is enough that the compound of the present invention and the joint use drug are combined at administration. Examples of such the administration aspect include (1) administration of a single preparation obtained by formulating the compound of the present invention and the joint use drug into a preparation at the same time, (2) simultaneous administration by the same administration route of two kinds of preparations obtained by formulating the compound of the present invention and the joint use drug separately, (3) administration by the same administration route at different times, of two kinds of preparations obtained by formulating the compound of the present invention and the joint use drug into a preparation separately, (4) simultaneous administration by different administration routes of two kinds of preparations obtained by formulating the compound of the present invention and the joint use drug into a preparation separately, and (5) administration by different administration routes at different times of two kinds of preparations obtained by formulating the compound of the present invention and the joint use drug into a preparation separately (e.g. administration in an order of the compound of the present invention; the joint use drug, or administration in a reverse order).

The joint use agent of the present invention has low toxicity and, for example, the compound of the present invention or (and) the joint use drug are mixed with a pharmacologically acceptable carrier by a per se known method into a pharmaceutical composition such as tablets (including sugar-coated tablets, film coating tablets), powders, granules, capsules (including soft capsules), solutions, injectables, suppositories, and sustained-release preparations, which can be safely administered orally or parenterally (e.g. local, rectal, intravenous administration etc.). Injectables can be administered intravenously, intramuscularly, subcutaneously or into an organ, or directly into a lesion.

Examples of a pharmacologically acceptable carrier which may be used in preparation of the joint use agent of the present invention include various organic or inorganic carrier substances which are conventionally used as a preparation material, such as excipients, lubricants, binders and disintegrating agents in a solid preparation, or solvents, solubilizers, suspending agents, isotonics, buffers and soothing agents in a liquid preparation. Further, if necessary, normal additives such as antiseptics, antioxidants, coloring agents, sweeteners, adsorbing agents, and wetting agents may be appropriately used at an appropriate amount.

Examples of excipients include lactose, white sugar, D-mannitol, starch, corn starch, crystalline cellulose, and light silicic anhydride.

Examples of lubricants include magnesium stearate, potassium stearate, talc, and colloidal silica.

Examples of binders include crystalline cellulose, white sugar, D-mannitol, dextrin, hydroxypropylcellulose, hydroxypropylmethylcellulose, polyvinylpyrrolidone, starch, sucrose, gelatin, methylcellulose, and sodium carboxymethylcellulose.

Examples of disintegrating agents include starch, carboxymethylcellulose, potassium carboxymethylcellulose, sodium carboxymethylstarch, and L-hydroxypropylcellulose.

Examples of solvents include water for injection, alcohol, propylene glycol, macrogol, sesame oil, corn oil, and olive oil.

Examples of solubilizers include polyethylene glycol, propylene glycol, D-mannitol, benzyl benzoate, ethanol, trisaminomethane, cholesterol, triethanolamine, sodium carbonate, and sodium citrate.

Examples of suspending agents include surfactants such as stearyltriethanolamine, sodium laurylsulfate, lauryl aminopropionate, lecithin, benzalkonium chloride, benzethonium chloride, and monostearic acid glycerin; hydrophilic polymers such as polyvinyl alcohol, polyvinylpyrrolidone, sodium carboxymethylcellulose, methylcellulose, hydroxymethylcellulose, hydroxyethylcellulose, and hydroxypropylcellulose.

Examples of isotonics include glucose, D-sorbitol, sodium chloride, glycerin, and D-mannitol.

Examples of buffers include buffers such as phosphate, acetate, carbonate, and citrate.

Examples of soothing agents include benzyl alcohol.

Examples of antiseptics include paraoxybenzoic acid esters, chlorobutanol, benzyl alcohol, phenethyl alcohol, dehydroacetic acid, and sorbic acid.

Examples of antioxidants include sulfite, ascorbic acid, and α-tocopherol.

A ratio of blending the compound of the present invention and the joint use drug in the joint use agent of the present invention can be appropriately selected depending on an administration subject, an administration route, and a disease.

For example, a content of the compound of the present invention in the joint use agent of the present invention varies depending on a form of a preparation, and is usually about 0.01 to 100% by weight, preferably about 0.1 to 50% by weight, further preferably about 0.5 to 20% by weight based on the whole preparation.

The content of the joint use drug in the joint use agent of the present invention varies depending on a form of a preparation, and is usually about 0.01 to 100% by weight, preferably about 0.1 to 50% by weight, further preferably about 0.5 to 20% by weight based on the whole preparation.

The content of an additive such as a carrier in the joint use agent of the present invention varies depending on the form of a preparation, and is usually about 1 to 99.99% by weight, preferably about 10 to 90% by weight based on the whole preparation.

In addition, when the compound of the present invention and the joint use drug are formulated into a preparation separately, the similar content may be used.

These preparations can be prepared by a per se known method which is normally used in a pharmacy step.

For example, the compound of the present invention or the joint use drug together with dispersants (e.g. Tween 80 (manufactured by Atlas Powder, USA), HCO 60 (manufactured by Nikko Chemicals Co., Ltd.), polyethylene glycol, carboxymethylcellulose, sodium alginate, hydroxypropylmethylcellulose, dextrin etc.), stabilizers (e.g. ascorbic acid, sodium pyrosulfite etc.), surfactants (e.g. Polysorbate 80, macrogol etc.), solubilizing agents (e.g. glycerin, ethanol etc.), buffers (e.g. phosphoric acid and alkali metal salt thereof, citric acid and alkali metal salt thereof etc.), isotonics (e.g. sodium chloride, potassium chloride, mannitol, sorbitol, glucose etc.), pH adjusting agents (e.g. hydrochloric acid, sodium hydroxide etc.), preservatives (e.g. ethyl paraoxybenzoate, benzoic acid, methylparaben, propylparaben, benzyl alcohol etc.), dissolving agents (e.g. concentrated glycerin, meglumine etc.), solubilizers (e.g. propylene glycol, white sugar etc.), or soothing agents (e.g. glucose, benzyl alcohol etc.) can be prepared into an aqueous injectable, or can be dissolved, suspended or emulsified in a vegetable oil such as an olive oil, a sesame oil, a cottonseed oil and a corn oil, or a solubilizer such as propylene glycol into an oil-soluble injectable preparation, thus, an injectable preparation is obtained.

For obtaining an oral preparation, according to a per se known method, excipients (e.g. lactose, white sugar, starch etc.), disintegrating agents (e.g. starch, calcium carbonate etc.), binders (e.g. starch, gum arabic, carboxymethylcellulose, polyvinylpyrrolidone, hydroxypropylcellulose etc.) or lubricants (e.g. talc, magnesium stearate, polyethylene glycol 6000 etc.) are added to the compound of the present invention or the joint use drug, the mixture is compressed and molded and, if necessary, this can be coated by the known per se method for the purpose of taste masking, enteric solubility or durability, to obtain an oral preparation. As the coating agent, for example, hydroxypropylmethylcellulose, ethylcellulose, hydroxymethylcellulose, hydroxypropylcellulose, polyoxyethylene glycol, Tween 80, Pluronic F68, cellulose acetate phthalate, hydroxypropylmethylcellulose phthalate, hydroxymethylcellulose acetate succinate, Eudragit (manufactured by Rohm, Germany, methacrylic acid/acrylic acid copolymer) and a pigment (e.g. bengala, titanium dioxide etc.) are used. An oral preparation may be a rapid-releasing preparation or a sustained-release preparation.

For example, for formulating into a suppository, according to the known per se method, the compound of the present invention or the joint use drug can be formulated into an oily or aqueous solid, semisolid or liquid suppository. Examples of an oily base used in the composition include glyceride of higher fatty acid [e.g. cacao butter, witepsols (manufactured by Dynamite Nobel, Germany) etc.], medium fatty acid [e.g. mygliols (manufactured by Dynamite Nobel, Germany) etc.], and vegetable oil (e.g. sesame oil, soybean oil, cottonseed oil etc.). In addition, examples of an aqueous base include polyethylene glycols, and propylene glycol, and examples of an aqueous gel base include natural gums, cellulose derivative, vinyl polymer, and acrylic acid polymer.

Examples of the sustained-release preparation include a sustained-release microcapsule agent.

For formulating into sustained-release microcapsules, a per se known method may be adopted, but it is preferable to administer by molding, for example, into a sustained-release preparation shown in the following [2].

It is preferable that the compound of the present invention is molded into an oral preparation such as solid preparations (e.g. powders, granules, tablets, capsules), or molded into a rectal preparation such as a suppository. Oral preparation is particularly preferable.

The joint use drug can be formulated into the aforementioned dosage forms depending on a kind of a drug.

Hereinafter, [1] an injectable of the compound of the present invention or the joint use drug and preparation thereof, [2] a sustained-release preparation or a rapid-releasing preparation of the compound of the present invention or the joint use drug and preparation thereof, and [3] a sublingual tablet, a buccal or an oral cavity rapid-disintegrating agent of the compound of the present invention or the joint use drug and preparation thereof will be shown specifically.

### [1] Injectable and preparation thereof

An injectable in which the compound of the present invention or the joint use drug is dissolved in water is preferable. Benzoate and/or salicylate may be contained in the injectable.

The injectable is obtained by dissolving the compound of the present invention or the joint use drug and, optionally, benzoate and/or salicylate in water.

Examples of the above salt of benzoic acid or salicylic acid include an alkali metal salt such as sodium and potassium, an alkaline earth metal salt such as calcium, and magnesium, an ammonium salt, a meglumine salt, and an organic acid salt such as trometamol.

The concentration of the compound of the present invention or the joint use drug in an injectable is 0.5 to 50 w/v%, preferably around 3 to 20 w/v%. The concentration of benzoate and/or salicylate is 0.5 to 50 w/v%, preferably 3 to 20 w/v%.

In addition, additives which are generally used in an injectable, such as stabilizers (ascorbic acid, sodium pyrosulfite etc.), surfactants (Polysorbate 80, macrogol etc.), solubilizing agents (glycerin, ethanol etc.), buffers (phosphoric acid and alkali metal salt thereof, citric acid and alkali metal salt thereof etc.), isotonics (sodium chloride, potassium chloride etc.), dispersants (hydroxypropylmethylcellulose, dextrin), pH adjusting agents (hydrochloric acid, sodium hydroxide etc.), preservatives (ethyl paraoxybenzoate, benzoic acid etc.), dissolving agents (concentrated glycerin, meglumine etc.), solubilizers (propylene glycol, white sugar etc.), and soothing agents (glucose, benzyl alcohol etc.) can be appropriately blended in the present agent. These additives are blended at a ratio which is normally used in an injectable.

Suitably, the injectable is adjusted to a pH of 2 to 12, preferably 2.5 to 8.0 by addition of a pH adjusting agent.

The injectable is obtained by dissolving the compound of the present invention or the joint use drug and, optionally, benzoate and/or salicylate and, if necessary, the aforementioned additive in water. These dissolutions may be performed in any order, and may be performed appropriately as in the preparation of a conventional injectable preparation.

Suitably, an aqueous injectable solution is warmed, and an aqueous injectable solution can be provided as the injectable by performing filtration sterilization or high pressure heating sterilization as in a conventional injectable preparation.

Suitably, an aqueous injectable solution is high pressure heating-sterilized, for example, under conditions of 100°C to 121°C for 5 minutes to 30 minutes.

Further, the injectable may be prepared into a preparation to which antibacterial property of a solution is imparted so that it can be used as a multiple divided administration preparation.

### [2] Sustained-release preparation or rapid-releasing preparation and preparation thereof

A sustained-release preparation in which a core containing the compound of the present invention or the joint use drug is covered with a covering agent such as a water-insoluble substance or a swelling polymer if necessary, is preferable. For example, an oral sustained-release preparation which is a one day one time administration type is preferable.

Examples of the water-insoluble substance used in the covering agent include cellulose ethers such as ethylcellulose, and butylcellulose, cellulose esters such as cellulose acetate, and cellulose propionate, polyvinyl esters such as polyvinyl acetate, and polyvinyl butyrate, acrylic acid-based polymers such as an acrylic acid/methacrylic acid copolymer, a methyl methacrylate copolymer, an ethoxyethyl methacrylate/cinnamoethyl methacrylate/aminoalkyl methacrylate copolymer, polyacrylic acid, polymethacrylic acid, a methacrylic acid alkylamide copolymer, poly(methyl methacrylate), polymethacrylate, polymethacrylamide, aminoalkyl methacrylate copolymer, poly(methacrylic acid anhydride), a glycidyl methacrylate copolymer, inter alia, Eudragits (Rohm Farma) such as Eudragit RS-100, RL-100, RS-30D, RL-30D, RL-PO, RS-PO (ethyl acrylate/methyl methacrylate/trimethyl chloride methacrylate/ethyl ammonium copolymer), and Eudragit NE-30D (methyl methacrylate/ethyl acrylate copolymer), a hardened oil such as hardened castor oil (e.g. Lovely Wax (Freund Corporation) etc.), waxes such as carnauba wax, fatty acid glycerin ester, and paraffin, and polyglycerin fatty acid ester.

As the swelling polymer, a polymer having an acidic dissociating group, and exhibiting pH dependent swelling is preferable, and a polymer having an acidic dissociating group so that swelling is small in an acidic region such as in stomach, and swelling becomes great in a neutral region such as the small intestine and the large intestine is preferable.

Examples of such the polymer having an acidic dissociating group, and exhibiting pH dependent swelling include crosslinking polyacrylic acid polymers such Carbomer 934P, 940, 941, 974P, 980, and 1342, polycarbophil, and calcium polycarbophil (both manufactured by BF Goodrich), and Highviswako 103, 104, 105, and 304 (all manufactured by Wako Pure Chemical Industries, Ltd.).

The covering agent used in the sustained-release preparation may further contain a hydrophilic substance.

Examples of the hydrophilic substance include polysaccharides optionally having a sulfate group such as pullulan, dextrin, and alginic acid alkali metal salt, polysaccharides having a hydroxyalkyl group or a carboxyalkyl group such as hydroxypropylcellulose, hydroxypropylmethylcellulose, and sodium carboxymethylcellulose, methylcellulose, polyvinylpyrrolidone, polyvinyl alcohol, and polyethylene glycol.

The content of the water-insoluble substance in the covering agent for the sustained-release preparation is about 30 to about 90% (w/w), preferably about 35 to about 80% (w/w), further preferably about 40 to 75% (w/w), and the content of the swelling polymer is about 3 to about 30% (w/w), preferably about 3 to about 15% (w/w).

The covering agent may further contain a hydrophilic substance and, in this case, the content of the hydrophilic substance in the covering agent is about 50% (w/w) or smaller, preferably about 5 to about 40% (w/w), further preferably about 5 to about 35% (w/w). Herein, the % (w/w) indicates % by weight based on the composition of the covering agent in which a solvent (e.g. water, lower alcohol such as methanol and ethanol etc.) is removed from a solution of the covering agent.

The sustained-release preparation is prepared by preparing a core containing a drug, and covering the resulting core with a covering agent solution in which the water-insoluble substance or the swelling polymer is dissolved by heating or dissolved or dispersed in a solvent, as exemplified below.

### I. Preparation of core containing drug

The form of a core containing a drug covered with a covering agent (hereinafter, simply referred to as core) is not particularly limited, but the core is preferably formed into a particle shape such as a granule and a fine particle.

When the core is a granule or a fine particle, an average particle diameter thereof is preferably about 150 to 2,000 µm, more preferably about 500 to about 1,400 µm.

The core can be prepared by a conventional process. For example, the core is prepared by mixing an appropriate excipient, binder, disintegrating agent, lubricant and stabilizer with a drug, and formulating the mixture into a core by a wet extrusion granulating method, or a fluidizing layer granulating method.

The content of a drug in a core is about 0.5 to about 95% (w/w), preferably about 5.0 to about 80% (w/w), further preferably about 30 to about 70% (w/w).

As the excipient to be contained in the core, sugars such as white sugar, lactose, mannitol, and glucose, starch, crystalline cellulose, calcium phosphate, and corn starch are used. Inter alia, crystalline cellulose and corn starch are preferable.

As the binder, for example, polyvinyl alcohol, hydroxypropylcellulose, polyethylene glycol, polyvinylpyrrolidone, Pluronic F68, gum arabic, gelatin, and starch are used. As the disintegrating agent, for example, potassium carboxymethylcellulose (ECG505), sodium croscarmellose (Ac-Di-Sol), crosslinking polyvinylpyrrolidone (crospovidone), and low-substituted hydroxypropylcellulose (L-HPC) are used. Inter alia, hydroxypropylcellulose, polyvinylpyrrolidone, and low-substituted hydroxypropylcellulose are preferable. As the lubricant or aggregation preventing agent, for example, talc, magnesium stearate and an inorganic salt thereof are used. As the lubricant, polyethylene glycol is used. As the stabilizer, acids such as tartaric acid, citric acid, succinic acid, fumaric acid, maleic acid are used.

The core may be also prepared by a rolling granulating method, a pan coating method, a fluidizing layer coating method or a melting granulating method, which is performed by adding a drug or a mixture of this and an excipient or a lubricant in portions while spraying a binder dissolved in an appropriate solvent such as a lower alcohol (e.g. methanol, ethanol etc.) on an inert carrier particle which is to be a center of a core, in addition to the aforementioned process. As the inert carrier particle, a particle made of white sugar, lactose, starch, crystalline cellulose, or waxes may be used, and an average particle diameter thereof is preferably about 100 µm to about 1,500 µm.

For separating the drug contained in the core and the covering agent, the surface of the core may be covered with a protecting agent. As the protecting agent, for example, the aforementioned hydrophilic substance, or water-insoluble substance is used. As the protecting agent, preferably, polyethylene glycol, and polysaccharides having a hydroxyalkyl group or a carboxyalkyl group, more preferably, hydroxypropylmethylcellulose, or hydroxypropylcellulose is used. The protecting agent may contain an acid such as tartaric acid, citric acid, succinic acid, fumaric acid, or maleic acid as a stabilizer, and a lubricant such as talc. When the protecting agent is used, the covering amount thereof is about 1 to about 15% (w/w), preferably about 1 to about 10% (w/w), further preferably about 2 to 8% (w/w) based on the core.

The protecting agent can be covered by a normal coating method and, specifically, the protecting agent can be covered by spray-coating a core by a fluidizing layer coating method, or a pan coating method.

### II. Covering of core with covering agent

A sustained-release preparation is prepared by covering the core obtained in the above I with the aforementioned water-insoluble substance and pH dependent swelling polymer, and a covering agent solution in which a hydrophilic substance is dissolved by heating, or dissolved or dispersed in a solvent.

Examples of a method for covering the core with the covering agent solution include a method by spray-coating.

A composition ratio of the water-insoluble substance, the swelling polymer and the hydrophilic substance in the covering agent solution is appropriately selected so that a content of each component in a covering film becomes the aforementioned content, respectively.

The covering amount of the covering agent is about 1 to about 90% (w/w), preferably about 5 to about 50% (w/w), further preferably about 5 to 35% (w/w) based on the core (not containing the covering amount of the protecting agent).

As the solvent of the covering agent solution, water or an organic solvent alone, or a mixture of both of them can be used. The ratio of mixing water and the organic solvent (water/organic solvent: weight ratio) upon use of a mixture can vary in a range of 1 to 100%, preferably 1 to about 30%. The organic solvent is not particularly limited as far as it dissolves a water-insoluble substance, but lower alcohol such as methyl alcohol, ethyl alcohol, isopropyl alcohol, and n-butyl alcohol, lower alkanone such as acetone, acetonitrile, chloroform, and methylene chloride are used. Among them, lower alcohol is preferable, and ethyl alcohol, and isopropyl alcohol are particularly preferable. Water and a mixture of water and the organic solvent are preferably used as the solvent of the covering agent. Thereupon, if necessary, for stabilizing the covering agent, an acid such as tartaric acid, citric acid, succinic acid, fumaric acid, or maleic acid may be added to the covering agent solution.

The procedure for covering by spray coating can be conducted by a conventional coating method. Specifically, the procedure can be conducted by spray-coating the covering agent solution on the core by a fluidizing layer coating method or a pan coating method. Thereupon, if necessary, talc, titanium oxide, magnesium stearate, calcium stearate, or light silicic anhydride as a lubricant, and glycerin fatty acid ester, hardened castor oil, triethyl citrate, cetyl alcohol, or stearyl alcohol as a plasticizer may be added.

After covering with the covering agent, an antistatic agent such as talc may be mixed, if necessary.

A rapid-releasing preparation may be liquid (solution, suspension, emulsion etc.) or solid (particle, pill, tablet etc.). An oral preparation, and a parental preparation such as an injectable are used, and an oral preparation is preferable.

A rapid-releasing preparation may usually contain a carrier, an additive, and an excipient (hereinafter, abbreviated as excipient in some cases) which are conventionally used in the pharmacy field, in addition to a drug which is an active ingredient. The preparation excipient to be used is not particularly limited as far as it is an excipient which is conventionally used as a preparation excipient. Examples of the excipient for an oral solid preparation include lactose, starch, corn starch, crystalline cellulose (Avicel PH101 manufactured by Asahi Chemical Industry Co., Ltd.), powdery sugar, granulated sugar, mannitol, light silicic anhydride, magnesium carbonate, calcium carbonate, and L-cysteine, preferably corn starch and mannitol. These excipients can be used alone or in a combination of two or more. The content of the excipient is, for example, about 4.5 to about 99.4 w/w%, preferably about 20 to about 98.5 w/w%, further preferably about 30 to about 97 w/w% based on a total amount of a rapid-releasing preparation.

The content of a drug in a rapid-releasing preparation can be appropriately selected from a range of about 0.5 to about 95%, preferably about 1 to about 60% based on a total amount of a rapid-releasing preparation.

When a rapid-releasing preparation is an oral solid preparation, the preparation usually contains a disintegrating agent in addition to the aforementioned components. As such the disintegrating agent, for example, calcium carboxymethylcellulose (ECG-505 manufactured by Gotoku Chemical Company Ltd.), sodium croscarmellose (e.g. Acdisol manufactured by Asahi Chemical Industry Co., Ltd.), crospovidone (e.g. Coridone CL manufactured by BASF), low-substituted hydroxypropylcellulose (manufactured by Shin-Etsu Chemical Co., Ltd.), carboxymethylstarch (manufactured by Matsutani Chemical Industry Co., Ltd.), sodium carboxymethylstarch (Exprotab manufactured by Kimura Industry), and partially gelatinized starch (PCS manufactured by Asahi Chemical Industry Co., Ltd.) are used. For example, a disintegrating agent which disintegrates granules by contacting with water to absorb water, or swell, or form a channel between an active ingredient and an excipient which constitute a core, can be used. These disintegrating agents can be used alone or in combination of two or more. The blending amount of a disintegrating agent is appropriately selected depending on a kind and a blending amount of a drug to be used, and preparation design of releasability, and is, for example, about 0.05 to about 30 w/w%, preferably about 0.5 to about 15 w/w% based on a total amount of a rapid-releasing preparation.

When a rapid-releasing preparation is an oral solid preparation, the oral solid preparation may further contain additives which are conventionally used in a solid preparation, if desired, in addition to the aforementioned composition. As such additives, for example, binders (e.g. sucrose, gelatin, gum arabic powder, methylcellulose, hydroxypropylcellulose, hydroxypropylmethylcellulose, carboxymethylcellulose, polyvinylpyrrolidone, plullulan, dextrin etc.), lubricants (e.g. polyethylene glycol, magnesium stearate, talc, and light silicic anhydride (e.g. Aerosil Nippon Aerosil), surfactants (e.g. anionic surfactants such as sodium alkylsulfate, nonionic surfactants such as polyoxyethylene fatty acid ester and polyoxyethylene sorbitan fatty acid ester, polyoxyethylene castor oil derivative etc.), coloring agents (e.g. tar-based pigment, caramel, bengala, titanium oxide, riboflavins) and, if necessary, flavoring agents (e.g. sweetener, perfume etc.), adhering agents, preservatives, wetting agents, and antistatic agents are used. In addition, as a stabilizer, an organic acid such as tartaric acid, citric acid, succinic acid, or fumaric acid may be added.

As the binder, hydroxypropylcellulose, polyethylene glycol, and polyvinylpyrrolidone are preferably used.

A rapid-releasing preparation can be prepared by mixing the aforementioned respective components and, if necessary, kneading and molding the mixture based on technique for preparing a conventional preparation. The mixing is performed by a generally employed method such as mixing and kneading. Specifically, for example, a rapid-releasing preparation is formed into a particle, the particle can be prepared by mixing components using a vertical granulator, a universal kneader (manufactured by Hata Iron Works Co., Ltd.), or a fluidizing layer granulator (FD-5S (manufactured by Powrex Corporation), and granulating the mixture by a wet extrusion granulating method, or a fluidizing layer granulating method, according to the similar procedure to the method of preparing the core of the sustained-release preparation.

The thus obtained rapid-releasing preparation and sustained-release preparation are formulated into a preparation as they are, or appropriately formulated into a preparation separately together with a preparation excipient and, thereafter, may be formulated into preparations which are administered by a combination at the same time or at arbitrary administration intervals, or both may be appropriately formulated into one oral preparation (e.g. granules, fine granules, tablets, capsules etc.) as they are, or together with a preparation excipient. Both preparations are prepared into granules or fine granules, and may be filled into the same capsule to obtain an oral preparation.

### [3] Sublingual tablet, buccal or oral cavity rapid-disintegrating agent and preparation thereof

A sublingual tablet, a buccal preparation, and oral cavity rapid-disintegrating agent may be a solid preparation such as a tablet, or may be an oral cavity mucous applying tablet (film).

As the sublingual tablet, the buccal or the oral cavity rapid-disintegrating agent, a preparation containing the compound of the present invention or the joint use drug and an excipient is preferable. In addition, the preparation may contain assistant agents such as lubricants, isotonics, hydrophilic carriers, water-dispersible polymers, and stabilizers. In addition, for facilitating absorption, and enhancing biological availability, β-cyclodextrin or β-cyclodextrin derivative (e.g. hydroxypropyl-β-cyclodextrin) may be contained.

Examples of the excipient include lactose, white sugar, D-mannitol, starch, crystalline cellulose, and light silicic anhydride. Examples of the lubricant include magnesium stearate, calcium stearate, talc, and colloidal silica. Magnesium stearate and colloidal silica are particularly preferable. Examples of the isotonic include sodium chloride, glucose, fructose, mannitol, sorbitol, lactose, saccharose, glycerin, and urea. Mannitol is particularly preferable. Examples of the hydrophilic carrier include swelling hydrophilic carriers such as crystalline cellulose, ethylcellulose, crosslinking polyvinylpyrrolidone, light silicic anhydride, silicic acid, dipotassium phosphate, and calcium carbonate. Crystalline cellulose (e.g. microcrystalline cellulose etc.) is particularly preferable. Examples of the water-dispersible polymer include gum (e.g. tragacanth gum, gum arabic, guar gum), alginate (e.g. sodium alginate), cellulose derivative (e.g. methylcellulose, carboxymethylcellulose, hydroxymethylcellulose, hydroxypropylcellulose, hydroxypropylmethylcellulose), gelatin, water-soluble starch, polyacrylic acid (e.g. Carbomer), polymethacrylic acid, polyvinyl alcohol, polyethylene glycol, polyvinylpyrrolidone, polycarbophil, and ascorbate palmitate. Hydroxypropylmethylcellulose, polyacrylic acid, alginate, gelatin, carboxymethylcellulose, polyvinylpyrrolidone, and polyethylene glycol are preferable. Hydroxypropylmethylcellulose is particularly preferable. Examples of the stabilizer include cysteine, thiosorbitol, tartaric acid, citric acid, sodium carbonate, ascorbic acid, glycine, and sodium sulfite. Citric acid and ascorbic acid are particularly preferable.

The sublingual tablet, the buccal or the oral cavity rapid-disintegrating agent can be prepared by mixing the compound of the present invention or the joint use drug and an excipient by a per se known method. Further, if desired, the aforementioned assisting agents such as lubricants, isotonics, hydrophilic carriers, water-dispersible polymers, stabilizers, coloring agents, sweeteners, and preservatives may be mixed therein. After the aforementioned components are mixed simultaneously or at different times, the mixture is compressed and molded under pressure to obtain the sublingual tablet, the buccal tablet or the oral cavity rapid-disintegrating agent. In order to obtain an appropriate hardness, before or after a process of compressing and molding, if necessary, the mixture may be wetted and swollen using a solvent such as water and an alcohol and, after molding, this may be dried to prepare a tablet.

When molded into a mucous applying tablet (film), the compound of the present invention or the joint use drug and the aforementioned water-dispersible polymer (preferably, hydroxypropylcellulose, hydroxypropylmethylcellulose) and an excipient are dissolved in a solvent such as water, and the resulting solution is cast into a film. Further, additives such as plasticizers, stabilizers, antioxidants, preservatives, coloring agents, buffers and sweeteners may be added. For imparting appropriate elasticity to a film, glycols such as polyethylene glycol and propylene glycol may be contained, or for enhancing adhesion of a film to a mucous lining in an oral cavity, a biological adhesive polymer (e.g. Polycarbophil, Carbopol) may be contained. Casting is attained by pouring a solution on a non-adhesive surface, spreading this into a uniform thickness (preferably, around 10 to 1000 micron) with an application equipment such as a doctor blade, and drying the solution to form a film. The thus formed film may be dried at room temperature or under warming, and cut into the desired surface area.

Examples of the preferable oral cavity rapid-disintegrating agent include a solid rapid-diffusing administration agent comprising a net body of the compound of the present invention or the joint use drug, and a water-soluble or water-diffusing carrier which is inert to the compound of the present invention or the joint use drug. The net body is obtained by sublimating a solvent from the solid composition composed of a solution in which the compound of the present invention or the joint use drug is dissolved in an appropriate solvent.

It is preferable that a composition of the oral cavity rapid-disintegrating agent contains a matrix forming agent and a secondary composition, in addition to the compound of the present invention or the joint use drug.

Examples of the matrix forming agent include substances derived from animal proteins or vegetable proteins such as gelatins, dextrins as well as soybean, wheat and psyllium seed proteins; gummy substances such as gum arabic, guar gum, agar and xanthan; polysaccharides; alginic acids; carboxymethylcellulose; carrageenans; dextrans; pectins; synthetic polymers such as polyvinylpyrrolidone; gelatin-gum arabic complex. Further, sugars such as mannitol, dextrose, lactose, galactose, and trehalose; cyclic saccharides such as cyclodextrin; inorganic salts such as sodium chloride and aluminum silicate; amino acids of a carbon number of 2 to 12 such as glycine, L-alanine, L-aspartic acid, L-glutamic acid, L-hydroxyproline, L-isoleucine, L-leucine and L-phenylalanine are contained.

One or more kinds of matrix forming agents can be introduced in a solution or a suspension before solidification. Such the matrix forming agent may be present together with a surfactant, or may be present without a surfactant. In addition to formation of a matrix, a matrix forming agent can help maintain the diffusion state of the compound of the present invention or the joint use drug in a solution or a suspension.

The composition may contain secondary components such as preservatives, antioxidants, surfactants, thickeners, coloring agents, pH adjusting agents, flavoring agents, sweeteners and taste masking agents. Examples of the suitable coloring agents include red, black and yellow iron oxides, and FD&C dyes such as FD&C Blue No. 2 and FD&C Red No. 40 of Ellis & Everald. Examples of the suitable flavoring agents include mint, raspberry, glycyrrhiza, orange, lemon, grapefruit, caramel, vanilla, cherry and grape flavor and a combination thereof. Examples of the suitable pH of adjusting agents include citric acid, tartaric acid, phosphoric acid, hydrochloric acid and maleic acid. Examples of the suitable sweeteners include aspartame, acesulphame K and taumatin. Examples of the suitable taste masking agents include sodium bicarbonate, ion exchange resin, cyclodextrin inclusion compound, adsorbing substance and microcapsulated apomorphine.

The preparation contains the compound of the present invention or the joint use drug at usually about 0.1 to about 50% by weight, preferably about 0.1 to about 30% by weight. The preparation which can dissolve 90% or more of the compound of the present invention or the joint use drug (in water) in about 1 minute to about 60 minutes, preferably about 1 minute to about 15 minutes, more preferably about 2 minutes to about 5 minutes (the aforementioned sublingual tablet, and buccal), and the oral cavity rapid-disintegrating agent which is disintegrated in 1 to 60 seconds, preferably 1 to 30 seconds, further preferably 1 to 10 seconds after taken into an oral cavity are preferable.

The content of the excipient in the whole preparation is about 10 to about 99& by weight, preferably about 30 to about 90% by weight. The content of β-cyclodextrin or β-cyclodextrin derivative in a whole preparation is 0 to about 30% by weight. The content of a lubricant based on the whole preparation is about 0.01 to about 10% by weight, preferably about 1 to about 5% by weight. The content of an isotonic relative to a whole preparation is about 0.1 to about 90% by weight, preferably about 10 to about 70% by weight. The content of a hydrophilic carrier relative to a whole preparation is about 0.1 to about 50% by weight, preferably about 10 to about 30% by weight. The content of a water-dispersible polymer relative to a whole preparation is about 0.1 to about 30% by weight, preferably about 10 to about 25% by weight. The content of a stabilizer relative to a whole preparation is about 0.1 to about 10% by weight, preferably about 1 to about 5% by weight. The preparation may further contain additives such as coloring agents, sweeteners and preservatives as necessary.

The dose of the joint use agent of the present invention varies depending on a kind of the compound of the present invention, and an age, a weight, symptom, a dosage form, an administration method, and an administration term and, for example, usually, the joint use agent is intravenously administered at about 0.01 to about 1000 mg/kg preferably about 0.01 to about 100 mg/kg, more preferably about 0.1 to about 100 mg/kg, particularly about 0.1 to about 50 mg/kg, inter alia, about 1.5 to about 30 mg/kg in terms of the compound of the present invention and the joint use drug, respectively, per hypertension patient (adult, weight about 60 kg) daily once to a few times a day. Of course, since a dose varies under various conditions as described above, a dose smaller than the aforementioned dose is sufficient in some cases, and it is necessary to administer a dose exceeding the aforementioned range in a some cases.

Any amount of the joint use drug may be set in such a range that side effect is not problematic. The dosage amount per day as the joint use drug varies depending on a degree of symptom, an age, a sex, a weight, and a difference of sensitivity of an administration subject, an term and an interval of administration, nature, compounding, and a kind of a pharmaceutical preparation, and a kind of an active ingredient, being not limiting, and an amount of a drug is about 0.001 to 2000 mg, preferably about 0.01 to 500 mg, further preferably about 0.1 to 100 mg per kg weight of a mammal, for a example, in an oral administration, and this is usually administered by dividing into once to four times a day.

Upon administration of the medicine of the present invention, the compound of the present invention may be administered after administration of the joint use drug, or the joint use drug may be administered after administration of the compound of the present invention, although the compound of the present invention and the joint use drug may be administered at the same term. When they are administered at different times, the time difference varies depending on an active ingredient to be administered, a dosage form, and an administration method and, for example, when the joint use drug is administered first, there is a method of administering the compound of the present invention within 1 minute to 3 days, preferably 10 minutes to 1 day, more preferably 15 minutes to 1 hour after administration of the joint use drug. When the compound of the present invention is administered first, there is a method of administering the joint use drug within 1 minute to 1 day, preferably 10 minutes to 6 hours, more preferably 15 to 1 hour after administration of the compound of the present invention.

As a preferable administration method, for example, about 0.001 to 200 mg/kg of the joint use drug which has been formulated into an oral preparation is orally administered and, after about 15 minutes, about 0.005 to 100 mg/kg (as one day amount) of the compound of the present invention which has been formulated into an oral preparation is orally administered.

The present invention will be further explained in detail by way of the following Reference Examples, Examples, Preparation Examples and Test Examples, but these Examples are merely embodiments, and do not limit the present invention, and variation is possible in a range without departing the scope of the present invention.

In the following Reference Examples, and Examples, "room temperature" usually indicates about 10°C to about 35°C. "%" indicates weight percentage unless otherwise indicated. A yield is indicated in mol/mol%.

Other abbreviations used in the text have the following meanings.
s: singlet
d: doublet
t: triplet
q: quartet
dd: double doublet
ddd: double double doublet
dt: double triplet
br: broad
J: coupling constant
Hz: Hertz
CDCl₃: chloroform-d
DMSO-d₆: dimethyl sulfoxide-d₆
¹H-NMR: proton nuclear magnetic resonance
IR: infrared absorption
DMF: N,N-dimethylformamide
THF: tetrahydrofuran
mp: melting point

Herein, when a base and an amino acid are expressed by abbreviations, they are based on abbreviations by IUPAC-IUB Commission on Biochemical Nomenclature or conventional abbreviations in the art, and examples thereof are shown below. In addition, when there can be optical isomers regarding amino acids, L-compound is shown unless otherwise indicated.
Gly: glycine
Ala: alanine
Val: valine
Leu: leucine
Ile: isoleucine
Ser: serine
Thr: threonine
Cys: cysteine
Met: methionine
Glu: glutamic acid
Asp: aspartic acid
Lys: lysine
Arg: arginine
His: histidine
Phe: phenylalanine
Tyr: tyrosine
Trp: tryptophan
Pro: proline
Asn: asparagine
Gln: glutamine

Sequences of Sequence Listing in the present description show the following sequences.

### SEQ ID NO: 1

This indicates an amino acid sequence of human FM-3.

### SEQ ID NO: 2

This indicates an amino acid sequence of mouse FM-3.

### SEQ ID NO: 3

This indicated an amino acid sequence of human TGR-1.

### Examples

### Reference Example 1

### N-(1-Benzyl-4-piperidinyl)-N-phenylpropionamide oxalate

### Step 1

Sodium triacetoxyhydroborate (1.1 g, 5.3 mmol) was added to a solution of 1-benzyl-4-piperidone (1.0 g, 5.3 mmol) and aniline (0.5 g, 5.3 mmol) in a mixture of chloroform/ethyl acetate (10 mL/5 mL) by portions, and the mixture was stirred at room temperature for 40 minutes. To the reaction mixture was added an aqueous saturated sodium bicarbonate solution (50 ml), the mixture was extracted with ethyl acetate (3x50 ml), the organic layer was dried over anhydrous magnesium sulfate, and the solvent was distilled off. The residue was purified by silica gel column chromatography (solvent: hexane-ethyl acetate) to obtain (1-benzyl-4-piperidinyl)phenylamine (0.9 g, 64%).
¹H-NMR (CDCl₃) δ: 1.49 (2H, m), 2.04 (2H, m), 2.17 (2H, dt, J = 2.3 Hz, 10.8 Hz), 2.86 (2H, br d, J = 12.0 Hz), 3.30 (1H, m), 3.54 (2H, s), 6.50-7.35 (10H, m).

### Step 2

A chloroform solution (1 ml) of propionyl chloride (0.16 ml, 1.8 mmol) was added dropwise to a chloroform solution (4 ml) of (1-benzyl-4-piperidinyl)-phenyl-amine (0.4 g, 1.5 mmol) and triethylamine (0.4 ml, 2.9 mmol), and the mixture was stirred at room temperature for 40 minutes. The solvent was distilled off, and 10% hydrochloric acid (20 ml) and diethyl ether (20 ml) were added to the residue. A 8 N NaOH aqueous solution was added to make the aqueous layer basic, the mixture was extracted with dichloromethane (2×20 ml), the organic layer was washed with water (20 ml) and an aqueous saturated sodium chloride solution (20 ml), and dried over anhydrous sodium sulfate, and the solvent was distilled off. The residue was purified by silica gel column chromatography (solvent: hexane-ethyl acetate) to obtain oily N-(1-benzyl-4-piperidinyl)-N-phenylpropionamide (283 mg). A 2-propanol solution (4 ml) of citric acid (0.072 g, 0.80 mmol) was added to a 2-propanol solution (4 ml) of this compound, and precipitated crystals were washed with 2-propanol, and dried to obtain the title compound (0.31 g, 51%).
¹H-NMR (CDCl₃) δ: 1.00 (3H, t, J = 7.4Hz), 1.70-2.00 (6H, m), 2.74 (2H, br t, J = 12.0Hz), 3.55 (2H, br d, J = 11.7Hz), 4.12 (2H, s), 4.75 (1H, m), 6.95-7.45 (10H, m).

### Reference Example 2

### N-(1-Benzyl-4-piperidinyl)-N-phenylacetamide

(1-Benzyl-4-piperidinyl)-phenylamine obtained in Step1 of Reference Example 1 was heated in acetic anhydride at 70°C to obtain the oily title compound.

### Reference Example 3

### Ethyl 1-benzyl-4-[(3-methylbutanoyl) (phenyl)amino]piperidine-4-carboxylate ethyl ester oxalate

### Step 1

Trimethylsilylnitrile (7.1 ml, 53.0 mmol) was added dropwise to an acetic acid solution (50 mL) of 1-benzyl-4-piperidone (10.0 g, 53.0 mmol) and aniline (5.4 g, 58.0 mmol) over 10 minutes under ice-cooling with attention so that the reaction temperature did not exceed 40°C, and the mixture was stirred for 1 hour. The reaction mixture was poured into a cold aqueous ammonia solution (mixture of 50 ml of aqueous concentrated ammonium hydroxide solution and 50 g of crushed ice), and an aqueous concentrated ammonium hydroxide solution was slowly added until pH of the mixture became 10. The mixture was extracted with chloroform (3×100 ml), the organic layer was dried over anhydrous sodium sulfate, and the solvent was distilled off. To the resulting oily substance was added diethyl ether (20 ml), and precipitated white crystals were washed with diethyl ether, and dried to obtain 1-benzyl-4-(phenylamino)piperidine-4-carbonitril (13.6 g, 89%).
¹H-NMR(CDCl₃) δ: 1.93 (2H, dt, J = 3.0Hz, 10.0Hz), 2.34 (2H, dt, J = 2.2Hz, 12.0Hz), 2.47 (2H, dt, J = 2.2Hz, 10.0Hz), 2.82 (2H, br d, J = 11.0Hz), 3.56 (2H, s), 6.90-7.35 (10H, m) .

### Step 2

1-Benzyl-4-(phenylamino)piperidine-4-carbonitrile (19.0 g, 65.0 mmol) was added to concentrated sulfuric acid (70 ml) under ice-cooling with attention so that the reaction temperature did not exceed 25°C, and the mixture was stirred overnight. To the reaction mixture was added a cold aqueous ammonia solution (mixture of 170 ml of aqueous concentrated ammonium solution and 280 g of crushed ice), and the mixture was extracted with chloroform (4×200 ml). The organic layer was washed with water, and dried over anhydrous magnesium sulfate, and the solvent was distilled off. To the residue was added diisopropyl ether, the mixture was stirred, and precipitated crystals were dried to obtain 1-benzyl-4-(phenylamino)piperidine-4-carboxamide (14.6 g, 73%).
¹H-NMR(CDCl₃) δ: 1.92 (2H, d, J = 10.9Hz), 2.10 (2H, t, J = 10.9Hz), 2.34 (2H, dt, J = 3.5Hz, 11.7Hz), 2.75 (2H, br d, J = 10.9Hz), 3.48 (2H, s), 4.01 (1H, s), 5.27 (1H, br s), 6.60-7.30 (11H, m).

### Step 3

To 1,2-ethanediol (40 ml) were added 1-benzyl-4-(phenylamino)piperidine-4-carboxamide (14.6 g, 47.0 mmol) and potassium hydroxide (7.9 g, 141.0 mmol), and the mixture was heated under reflux for 20 hours. The mixture was allowed to stand until a temperature of the reaction mixture became room temperature, water (140 ml) was added, the mixture was filtered, and concentrated hydrochloric acid was added to the filtrate to make it acidic. Excessive 8 N sodium hydroxide was added to this aqueous solution to adjust pH to 8, and the resulting precipitates were recrystallized from water to obtain 1-benzyl-4-(phenylamino)piperidine-4-carboxylic acid sodium salt (6.6 g, 42%).
¹H-NMR(DMSO-d₆) δ: 1.84 (2H, m), 2.06 (2H, m), 2.38 (2H, m), 2.54 (2H, m), 3.49 (2H, s), 5.15 (1H, br s), 6.40-7.50 (10H, m) .

### Step 4

To an ethyl acetate suspension (2.5 ml) of 1-benzyl-4-(phenylamino)piperidine-4-carboxylic acid sodium salt (0.3 g, 0.9 mol) and isovaleic anhydride (1.25 ml, 6.3 mmol) was added slowly triethylamine (0.4 ml, 2.7 mmol), and the mixture was heated under reflux for 1 hour. The reaction mixture was cooled to 70°C, ethanol (0.7 ml) was added, and the mixture was heated under reflux again for 2 hours. The reaction mixture was allowed to stand to room temperature, an aqueous saturated sodium bicarbonate solution (20 ml) was added, the mixture was extracted with ethyl acetate (3×20 ml), the organic layer was dried over anhydrous magnesium sulfate, and the solvent was distilled off. To a 2-propanol solution (1 ml) of the resulting oily 1-benzyl-4-[(3-methylbutanoyl)(phenyl)amino]piperidine-4-carboxylic acid ethyl ester (0.074 g) was added a 2-propanol solution (2 ml) of citric acid (0.024 g, 0.27 mmol), and precipitated crystals were washed with 2-propanol, and dried to obtain the title compound (0.076 g, 20%).
¹H-NMR(CDCl₃) δ: 0.76 (6H, d, J = 6.7Hz), 1.30 (3H, t, J = 7.1Hz), 1.75 (2H, d, J = 7.0Hz), 1.95-2.15 (3H, m), 2.30-2.65 (4H, m), 3.15 (2H, br t, J = 12.3Hz), 3.35-3.50 (2H, m), 4.09 (2H, s), 4.25 (2H, q, J = 7.1Hz), 7.10-7.50 (10H, m) .

### Reference Example 4

### 4-[Benzoyl(phenyl)amino]-1-benzylpiperidine-4-carboxylic acid ethyl ester

According to the same manner as that of Step 4 of Reference Example 3, a sodium salt of 1-benzyl-4-(phenylamino)piperidine-4-carboxylic acid was reacted with benzoic anhydride to obtain the oily title compound (0.21 g, 52%).
¹H-NMR(CDCl₃) δ: 1.35 (3H, t, J = 7.1Hz), 1.90 (2H, m), 2.39 (2H, br d, J = 12.4Hz), 2.60 (2H, br t, J = 10.4Hz), 2.80 (2H, br d, J = 12.0Hz), 3.59 (2H, s), 4.31 (2H, q, J = 7.1Hz), 7.00-8.15 (15H, m).

### Reference Example 5

### 1-Benzyl-4-[phenyl(propionyl)amino]piperidine-4-carboxylic acid ethyl ester

According to the same manner as that of Step 4 of Reference Example 3, a sodium salt of 1-benzyl-4-(phenylamino)piperidine-4-carboxylic acid was reacted with propionic anhydride to obtain the oily title compound (1.0 g).

### Reference Example 6

### 1-Benzyl-4-[phenyl(propionyl)amino]piperidine-4-carboxylic acid ethyl ester maleate

To a solution of 1-benzyl-4-[phenyl(propionyl)amino]piperidine-4-carboxylic acid ethyl ester (760 mg, 1.92 mmol) obtained in Reference Example 5 in isopropanol (1.5 mM) was added a solution of maleic acid (222 mg, 1.92 mmol) in isopropanol (2 mL), and the mixture was allowed to stand at room temperature. The resulting white crystalline powder was filtered, washed with isopropanol, and dried to obtain the title compound (839 mg, 86%).
¹H-NMR (CDCl₃) δ: 0.95 (3H, t, J = 7.4Hz), 1.30 (3H, t, J = 7.1Hz), 1.88 (2H, q, J = 7.4Hz), 1.9-2.1 (2H, m), 2.41 (2H, d, J = 14.2Hz), 3.16 (2H, t, J = 11.7Hz), 3.35 (2H, d, J = 10.2Hz), 4.08 (2H, s), 4.26 (2H, q, J = 7.1Hz), 6.29 (2H, s), 7.19-7.26 (2H, m), 7.34-7.42 (8H, m).
Elementary Analysis: for C₂₄H₃₀N₂O₃·C₄H₄O₄
Calculated: C, 65.87; H, 6.71; N, 5.49. Found: C, 65.69; H, 6.76; N, 5.45.

### Reference Example 7

### 1-Benzyl-4-[phenyl(propionyl)amino]piperidine-4-carboxylic acid ethyl ester oxalate

To a solution of 1-benzyl-4-[phenyl(propionyl)amino]piperidine-4-carboxylic acid ethyl ester (1.0 g, 2.55 mmol) obtained in Reference Example 5 in isopropanol (3 mL) was added a solution of oxalic acid (341 mg, 2.70 mmol) in isopropanol (3 mL), and the mixture was allowed to stand at room temperature. The resulting white crystalline powder was filtered, washed with isopropanol, and dried to obtain the title compound.
¹H-NMR (CDCl₃) δ: 0.95 (3H, t, J = 7.2Hz), 1.30 (3H, t, J = 7.1Hz), 1.88 (2H, q, J = 7.5Hz), 2.00-2.15 (2H, br t), 2.40 (2H, d, J = 13.8Hz), 3.14 (2H, t, J = 11.6Hz), 3.30-3.45 (2H, br), 4.08 (2H, s), 4.24 (2H, q, J = 7.0Hz), 7.18-7.22 (2H, m), 7.28-7.42 (8H, m).

### Reference Example 8

### 4-[Acetyl(phenyl)amino]-1-benzylpiperidine-4-carboxylic acid ethyl ester oxalate

According to the same manner as that of Step 4 of Reference Example 3, a sodium salt of 1-benzyl-4-(phenylamino)piperidine-4-carboxylic acid was reacted with acetic anhydride to obtain the title compound (0.05 g, 7%). However, the salt is very scarcely soluble, and the following spectrum data are described as free amine.
¹H-NMR(CDCl₃, free amine) δ: 1.30 (3H, t, J = 7.1Hz), 1.69 (3H, s), 1.74 (2H, br d, J = 11.8Hz), 2.28 (2H, br d, J = 13.1Hz), 2.53 (2H, m), 2.71 (2H, m), 3.57 (2H, s), 4.24 (2H, q, J = 7.1Hz), 7.10-7.45 (10H, m).

### Reference Example 9

### 1-Benzyl-4-[butyryl(phenyl)amino]piperidine-4-carboxylic acid ethyl ester oxalate

According to the same manner as that of Step 4 of Reference Example 3, a sodium salt of 1-benzyl-4-(phenylamino)piperidine-4-carboxylic acid was reacted with butyric anhydride to obtain the title compound (0.30 g, 40%).
¹H-NMR(DMSO-d₆) δ: 0.80 (3H, t, J = 7.4Hz), 1.31 (3H, t, J = 7.0Hz), 1.46 (2H, m), 1.70-1.90 (4H, m), 2.32 (2H, br d, J = 13.6Hz), 2.80-3.15 (4H, m), 4.04 (2H, s), 4.22 (2H, q, J = 7.1Hz), 7.30-7.65 (10H, m).

### Reference Example 10

### 1-Benzyl-4-[pentanoyl(phenyl)amino]piperidine-4-carboxylic acid ethyl ester oxalate

According to the same manner as that of Step 4 of Reference Example 3, a sodium salt of 1-benzyl-4-(phenylamino)piperidine-4-carboxylic acid was reacted with valeric anhydride to obtain the title compound (0.59 g, 69%).
¹H-NMR(CDCl₃) δ: 0.76 (3H, t, J = 7.4Hz), 1.14 (2H, m), 1.29 (3H, t, J = 7.1Hz), 1.44 (2H, quintet, J = 7.5Hz), 1.86 (2H, t, J = 7.5Hz), 1.95-2.10 (2H, m), 2.38 (2H, br d, J = 14.6Hz), 3.30-3.40 (2H, m), 4.07 (2H, s), 4.24 (2H, q, J = 7.1Hz), 7.10-7.45 (10H, m).

### Reference Example 11

### 1-Benzyl-4-[hexanoyl(phenyl)amino]piperidine-4-carboxylic acid ethyl ester oxalate

According to the same manner as that of Step 4 of Reference Example 3, a sodium salt of 1-benzyl-4-(phenylamino)piperidine-4-carboxylic acid was reacted with hexanoic anhydride to obtain the title compound (0.38 g, 48%).
¹H-NMR(CDCl₃) δ: 0.80 (3H, t, J = 7.2Hz), 1.00-1.20 (4H, m), 1.29 (3H, t, J = 7.1Hz), 1.46 (2H, quintet, J = 7.3Hz), 1.85 (2H, t, J = 7.3Hz), 2.07 (2H, m), 2.38 (2H, br d, J = 14.6Hz), 3.15 (2H, br t, J = 12.6Hz), 3.39 (2H, br d, J = 11.5Hz), 4.10 (2H, s), 4.24 (2H, q, J = 7.1Hz), 7.15-7.50 (10H, m).

### Reference Example 12

### 1-Benzyl-4-[(2E)-2-butenoyl(phenyl)amino]piperidine-4-carboxylic acid ethyl ester oxalate

According to the same manner as that of Step 4 of Reference Example 3, a sodium salt of 1-benzyl-4-(phenylamino)piperidine-4-carboxylic acid was reacted with crotonic anhydride to obtain the title compound (0.33 g, 45%).
¹H-NMR(CDCl₃) δ: 1.28 (3H, t, J = 7.1Hz), 1.66 (3H, d, J = 6.9Hz), 2.05-2.15 (2H, m), 2.40 (2H, br d, J = 14.7Hz), 3.17 (2H, m), 3.41 (2H, br d, J = 5.6Hz), 4.11 (2H, s), 4.25 (2H, q, J = 7.1Hz), 5.39 (1H, d, J = 15.1Hz), 6.70-6.90 (1H, m), 7.15-7.45 (10H, m).

### Reference Example 13

### 1-Benzyl-4-[(4-chlorophenyl)(propionyl)amino]piperidine-4-carboxylic acid ethyl ester oxalate

According to the same manner as that of Reference Example 3, 4-chloroaniline was used in place of aniline to obtain the title compound (0.44 g, 66%).
¹H-NMR(DMSO-d₆) δ: 0.93 (3H, t, J = 7.4Hz), 1.30 (3H, t, J = 7.1Hz), 1.79 (2H, m), 1.90 (2H, q, 7.3Hz), 2.31 (2H, br d, J = 13.2Hz), 2.80-3.20 (4H, m), 4.03 (2H, s), 4.23 (2H, q, J = 7.0Hz), 7.40-7.70 (9H, m).

### Reference Example 14

### 1-Benzyl-4-[(3-chlorophenyl)(propionyl)amino]piperidine-4-carboxylic acid ethyl ester oxalate

According to the same manner as that of Reference Example 3, 3-chloroaniline was used in place of aniline to obtain the title compound (0.26 g, 33%).
¹H-NMR(DMSO-d₆) δ: 0.93 (3H, t, J = 7.4Hz), 1.30 (3H, t, J = 7.1Hz), 1.75-2.00 (4H, m), 2.25-2.45 (2H, m), 2.90-3.10 (4H, m), 4.07 (2H, s), 4.24 (2H, q, J = 7.1Hz), 7.40-7.70 (9H, m).

### Reference Example 15

### 1-Benzyl-4-[(2-chlorophenyl)(propionyl)amino]piperidine-4-carboxylic acid ethyl ester oxalate

According to the same manner as that of Reference Example 3, 2-chloroaniline was used in place of aniline to obtain the title compound (0.11 g, 13%).
¹H-NMR(DMSO-d₆) δ: 0.95 (3H, t, J = 6.9Hz), 1.31 (3H, t, J = 7.1Hz), 1.54 (1H, br t, J = 12.3Hz), 1.80-2.00 (3H, m), 2.00-2.15 (1H, m), 2.55-2.75 (1H, m), 2.85 (1H, br d, J = 13.5Hz), 2.90-3.30 (3H, m), 4.00 (2H, s), 4.15-4.35 (2H, m), 7.40-7.80 (9H, m).

### Reference Example 16

### 1-Benzyl-4-[(4-methylphenyl)(propionyl)amino]piperidine-4-carboxylic acid ethyl ester oxalate

According to the same manner as that of Reference Example 3, 4-methylaniline was used in place of aniline to obtain the title compound (0.42 g, 52%).
¹H-NMR(DMSO-d₆) δ: 0.92 (3H, t, J = 7.4Hz), 1.30 (3H, t, J = 7.1Hz), 1.79 (2H, m), 1.88 (2H, q, J = 7.4Hz), 2.30 (2H, bd, J = 13.4Hz), 2.45 (3H, s), 2.80-3.10 (4H, m), 4.02 (2H, br s), 4.22 (2H, q, J = 7.1Hz), 7.25-7.50 (9H, m).

### Reference Example 17

### 1-Benzyl-4-[(3-methylphenyl)(propionyl)amino]piperidine-4-carboxylic acid ethyl ester oxalate

According to the same manner as that of Reference Example 3, 3-methylaniline was used in place of aniline to obtain the title compound (0.072 g, 9%).
¹H-NMR(DMSO-d₆) δ: 0.92 (3H, t, J = 7.3Hz), 1.30 (3H, t, J = 7.1Hz), 1.79 (2H, m), 1.89 (2H, q, J = 7.3Hz), 2.33 (2H, br t, J = 15.2Hz), 2.44 (3H, s), 2.80-3.20 (4H, m), 4.03 (2H, br s), 4.23 (2H, q, J = 7.1Hz), 7.20-7.50 (9H, m).

### Reference Example 18

### 1-Benzyl-4-[(2-methylphenyl)(propionyl)amino]piperidine-carboxylic acid ethyl ester oxalate

According to the same manner as that of Reference Example 3, 2-methylaniline was used in place of aniline to obtain the title compound (0.047 g, 6%).
¹H-NMR(DMSO-d₆) δ: 0.93 (3H, t, J = 7.3Hz), 1.30 (3H, t, J = 7.1Hz), 1.65- 1.90 (4H, m), 2.36 (2H, br d, J = 12.8Hz), 2.43 (3H, s), 2.75-3.25 (4H, m), 4.06 (2H, br s), 4.24 (2H, q, J = 7.1Hz), 7.30-7.50 (9H, m).

### Reference Example 19

### 1-Benzyl-4-[(3-fluorophenyl)(propionyl)amino]piperidine-4-carboxylic acid ethyl ester maleate

According to the same manner as that of Reference Example 3, oily 1-benzyl-4-[(3-fluorophenyl)(propionyl)amino]piperidine-4-carboxylic acid ethyl ester was synthesized using 3-fluoroaniline in place of aniline, and this compound was converted into a salt using maleic acid to obtain the title compound (0.39 g, 49%).
¹H-NMR(DMSO-d₆) δ: 0.94 (3H, t, J = 7.3Hz), 1.31 (3H, t, J = 7.0Hz), 1.60- 2.05 (4H, m), 2.30 (1H, br d, J = 14.0Hz), 2.49 (1H, br d, J = 13.2Hz), 3.00-3.60 (4H, m), 4.15-4.30 (4H, m), 6.15 (2H, s), 7.25-7.75 (9H, m).

### Reference Example 20

### 1-Benzyl-4-[(3-bromophenyl)(propionyl)amino]piperidine-4-carboxylic acid ethyl ester maleate

According to the same manner as that of Reference Example 3, oily 1-benzyl-4-[(3-bromophenyl)(propionyl)amino]piperidine-4-carboxylic acid ethyl ester was synthesized using 3-bromoaniline in place of aniline, and this compound was converted into a salt using maleic acid to obtain the title compound (0.52 g, 61%).
¹H-NMR(DMSO-d₆) δ: 0.93 (3H, t, J = 7.3Hz), 1.31 (3H, t, J = 7.1Hz), 1.70- 2.00 (4H, m), 2.30 (1H, br d, J = 13.8Hz), 2.40-2.60 (1H, m), 3.00-3.70 (4H, m), 4.15-4.40 (4H, m), 6.15 (2H, s), 7.40-7.85 (9H, m).

### Reference Example 21

### 1-Benzyl-4-[(3-methoxyphenyl)(propionyl)amino]piperidine-4-carboxylic acid ethyl ester maleate

According to the same manner as that of Reference Example 3, oily 1-benzyl-4-[(3-methoxyphenyl)(propionyl)amino]piperidine-4-carboxylic acid ethyl ester was synthesized using 3-methoxyaniline in place of aniline, and this compound was converted into a salt using maleic acid to obtain the title compound (0.13 g, 5%).
¹H-NMR(DMSO-d₆) δ: 0.94 (3H, t, J = 7.3Hz), 1.31 (3H, t, J = 7.0Hz), 1.75- 2.00 (4H, m), 2.40 (1H, br d, J = 14.6Hz), 3.00-3.70 (4H, m), 3.89 (3H, s), 4.24 (2H, q, J = 7.0Hz), 6.15 (2H, s), 7.00 (2H, br d, J = 7.8Hz), 7.16 (1H, d, J = 7.7Hz), 7.40-7.60 (6H, m).

### Reference Example 22

### 1-Benzyl-4-[phenyl(propionyl)amino]piperidine-4-carboxylic acid

To a suspension of a sodium salt of 1-benzyl-4-(phenylamino)piperidine-4-carboxylic acid (9.0 g, 27.0 mmol) obtained in Step 3 of Reference Example 3 and water (10 ml) was added propionic anhydride (10 ml), and the mixture was heated under reflux overnight. The reaction mixture was cooled to 70°C, water (3 ml) was added thereto, and the mixture was heated under reflux again for 2 hours. The reaction mixture was allowed to stand to room temperature, and precipitates were filtered, washed with water, and dried to obtain the title compound (6.3 g, 75%).
¹H-NMR(DMSO-d₆) δ: 0.80 (3H, t, J = 7.3Hz), 1.40-1.60 (2H, m), 1.75 (2H, q, J = 7.3Hz), 2.08 (2H, br d, J = 13.0Hz), 2.20-2.60 (4H, m), 3.35 (2H, s), 7.10-7.70 (10H, m). Reference Example 23

### N-(1-Benzyl-4-butyl-4-piperidinyl)-N-phenylpropionamide oxalate

### Step 1

To a solution of 1.89 g (0.01 mol) of 1-benzyl-4-piperidone in 20 ml of toluene were added 1.02 g (0.011 mol) of aniline and 50 mg of p-toluenesulfonic acid monohydrate, and the mixture was heated under reflux for 16 hours under conditions of Molecular Sieve 4A dehydration. The reaction mixture was concentrated under reduced pressure, 10 ml of THF was added to the residue, and 12.5 ml (0.02 mol) of a 1.6 M n-butyllithium hexane solution was added under ice-cooling. After stirred at room temperature for 16 hours, ethyl acetate and water were added, followed by extraction. The organic layer was washed with water, and concentrated under reduced pressure, and the residue was purified by silica gel column chromatography (eluting solvent: n-hexane/ethyl acetate = 4/1) to obtain 720 mg (yield 22%) of (1-benzyl-4-butyl-4-piperidinyl)phenylamine as a colorless oil.

### Step 2

To a solution of 645 mg (2.0 mmol) of the resulting (1-benzyl-4-butyl-4-piperidinyl)phenylamine in 20 ml of chloroform was added 925 mg (10 mmol) of propionyl chloride, and the mixture was heated under reflux for 24 hours. The reaction mixture was concentrated, and ethyl acetate and a sodium bicarbonate solution were added to the residue, followed by extraction. The organic layer was washed with water, and concentrated under reduced pressure, the residue was purified by alumina column chromatography (eluting solvent: n-hexane/ethyl acetate = 95/5), and the eluate was recrystallized in a solvent of diisopropyl ether by addition of oxalic acid to obtain 180 mg (yield 19%) of the title compound as a white powder.
¹H-NMR(DMSO-d₆) δ: 0.74 (3H, t, J = 7.4Hz), 0.92 (3H, t, J = 6.9Hz), 1.20-1.38 (4H, m), 1.60-1.78 (4H, m), 2.00-2.15 (2H, m), 2.15-2.21 (2H, m), 2.80-2.95 (4H, m), 4.06 (2H, s), 7.10-7.20 (2H, m), 7.28-7.48 (8H, m).

### Reference Example 24

### N-(1-Benzyl-4-phenyl-4-piperidinyl)-N-phenylpropionamide oxalate

According to the same manner as that of Reference Example 23, phenyllithium was used in place of n-butyllithium to obtain 190 mg (yield 19%) of the title compound as a white powder.
¹H-NMR(DMSO-d₆) δ: 0.68 (3H, t, J = 7.3Hz), 1.65 (2H, q, J = 7.3Hz), 2.15-2.32 (2H, m), 2.52-2.73 (4H, m), 2.95-3.10 (2H, m), 3.98 (2H, s), 7.15-7.28 (4H, m), 7.28-7.50 (9H, m), 7.56 (2H, d, J = 7.7Hz) .

### Reference Example 25

### 1-Benzyl-4-[phenyl(propionyl)amino]-N-propyl-4-piperidinecarboxamide oxalate

To a solution of 310 mg (1.0 mmol) of 1-benzyl-4-(phenylamino)piperidine-4-carboxylic acid obtained by the method described in J. Org. Chem., vol.55, pp.4207 published in 1990 and 71 mg (1.2 mmol) of propylamine in 3 ml of DMF were added 196 mg (1.2 mmol) of diethyl cyanophosphate and 121 mg (1.2 mmol) of triethylamine under ice-cooling, and the mixture was stirred for 1 hour. To the reaction mixture were added ethyl acetate and a 2 N aqueous sodium hydroxide solution, followed by extraction. The organic layer was washed with water, and concentrated under reduced pressure, and the residue was purified by alumina column chromatography (eluting solvent: n-hexane/ethyl acetate = 4/1) to obtain 150 mg (yield 43%) of 1-benzyl-4-(phenylamine)-N-propyl-4-piperidinecarboxamide as a colorless oil. According to the same manner as that of Step 2 of Reference Example 3, 110 mg (yield 56%) of the title compound was obtained as a white powder, from the resulting 1-benzyl-4-(phenylamino)-N-propyl-4-piperidinecarboxamide.
¹H-NMR(DMSO-d6) δ: 0.75-0.85 (6H, m), 1.30-1.45 (2H, m), 1.52-1.78 (2H, m), 1.7 6 (2H, q, J = 7.4Hz), 2.20-2.28 (2H, m), 2.85-3.05 (6H, m), 3.97 (2H, s), 7.36 (5H, m), 7.38-7.50 (5H, m), 7.50-7.58 (1H, m).

### Reference Example 26

### 1-Benzyl-N-methoxy-N-methyl-4-[phenyl(propionyl)amino]-4-piperidinecarboxamide oxalate

To a solution of 500 mg (1.36 mmol) of 1-benzyl-4-[phenyl(propionyl)amino]piperidine-4-carboxylic acid obtained in Reference Example 22 and 399 mg (4.10 mmol) of N,O-dimethylhydroxylamine hydrochloride in 10 ml of DMF were added 266 mg (1.63 mmol) of diethyl cyanophosphate and 663 mg (6.55 mmol) of triethylamine under ice-cooling, and the mixture was stirred at room temperature for 16 hours. To the reaction mixture were added ethyl acetate and a 2 N aqueous sodium hydroxide solution, followed by extraction. The organic layer was washed with water and concentrated under reduced pressure, the residue was purified by alumina column chromatography (eluting solvent: n-hexane/ethyl acetate = 6/4), and the eluate was recrystallized in a solvent of diisopropyl ether by addition of oxalic acid to obtain 210 mg (yield 40%) of the title compound as a white powder.
¹H-NMR(DMSO-d₆) δ: 0.81 (3H, t, J = 7.4Hz), 1.55-1.70 (2H, m), 1.76 (2H, q, J = 7.4Hz), 2.20-2.32 (2H, m), 2.80-3.00 (2H, m), 3.00-3.12 (2H, m), 3.12 (3H, s), 3.66 (3H, s), 4.00 (2H, s), 7.21-7.26 (2H, m), 7.30-7.40 (5H, m), 7.40-7.50 (3H, m).

### Reference Example 27

### N-[1-Benzyl-4-(pyrrolidine-1-carbonyl)-4-piperidinyl]-N-phenylpropionamide oxalate

According to the same manner as that of Reference Example 26, pyrrolidine was used in place of N,O-dimethylhydroxylamine to obtain 210 mg (yield 40%) of the title compound as a white powder.
¹H-NMR(DMSO-d₆) δ: 0.81 (3H, t, J = 7.4Hz), 1.55-1.85 (6H, m), 1.81 (2H, q, J = 7.4Hz), 2.25-2.40 (2H, m), 3.10-3.28 (4H, m), 3.31-3.50 (4H, m), 4.15 (2H, s), 7.28-7.50 (10H, m) .

### Reference Example 28

### N-(1-Benzyl-4-cyano-4-piperidinyl)-N-phenylpropionamide

According to the same manner as that of Step 2 of Reference Example 23, from 1-benzyl-4-(phenylamino)piperidine-4-carbonitrile obtained by the method described in Synthetic Communications, vol.27, pp.923-937 published in 1997, 170 mg (yield 71%) of the title compound was obtained as a colorless oil.
¹H-NMR(CDCl₃) δ: 1. 02 (3H, t, J = 7.4Hz), 1. 50-1. 65 (2H, m), 1.93 (2H, q, J = 7.4Hz), 2.25-2.50 (4H, m), 2.75-2.90 (2H, m), 3.50 (2H, s), 7.16-7.35 (8H, m), 7.38-7.45 (2H, m).

### Reference Example 29

### 1-(2-Phenethyl)-4-[phenyl(propionyl)amino]piperidine-4-carboxylic acid ethyl ester oxalate

By the method described in Synthetic Communications, vol.27, pp.923-937 published in 1997, 180 mg (yield 44%) of the title compound was obtained as a white powder.
¹H-NMR(DMSO-d₆) δ: 0.83 (3H, t, J = 7.4Hz), 1.23 (3H, t, J = 7.0Hz), 1.70-1.95 (4H, m), 2.20-2.32 (2H, m), 2.75-2.90 (2H, m), 2.90-3.18 (4H, m), 3.20-3.35 (2H, m), 4.10-4.20 (2H, m), 7.15-7.40 (8H, m), 7.40-7.58 (2H, m).

### Reference Example 30

### 1-Benzoyl-4-[phenyl(propionyl)amino]piperidine-4-carboxylic acid ethyl ester

To a solution of 200 mg (0.66 mmol) of 4-[phenyl(propionyl)amino]piperidine-4-carboxylic acid ethyl ester obtained by the method described in J. Org. Chem., vol.55, pp.4207 published in 1990, and 1 ml of pyridine in 3 ml of chloroform was added 92 mg (0.66 mmol) of benzoyl chloride, and the mixture was stirred at room temperature for 16 hours. To the reaction mixture were added ethyl acetate and 2 N hydrochloric acid, followed by extraction. The organic layer was washed with water, and concentrated under reduced pressure, and the residue was purified by silica gel column chromatography (eluting solvent: n-hexane/ethyl acetate = 6/4) to obtain 210 mg (yield 86%) of the title compound as a colorless oil.
¹H-NMR(CDCl₃) δ: 0.95 (3H, t, J = 7.4Hz), 1.34 (3H, t, J = 7.1Hz), 1.30-1.70 (2H, m), 1.87 (2H, q, J = 7.4Hz), 2.15-2.25 (1H, m), 2.30-2.48 (1H, m), 3.10-3.25 (1H, m), 3.45-3.70 (2H, m), 4.29 (2H, q, J = 7.1Hz), 4.30-4.45 (1H, m), 7.20-7.45 (8H, m), 7.45-7.50 (2H, m).

### Reference Example 31

### 1-Phenyl-4-[phenyl(propionyl)amino]piperidine-4-carboxylic acid ethyl ester

To a solution of 250 mg (0.82 mmol) of 4-[phenyl(propionyl)amino]piperidine-4-carboxylic acid ethyl ester obtained by the method described in J. Org. Chem., vol.55, pp.4207 published in 1990 in 15 ml of toluene were added 836 mg (4.1 mmol) of iodobenzene, 150 mg (0.16 mmol) of tris(dibenzylideneacetone)dipalladium (0), 100 mg (0.49 mmol) of tri(tert-butyl)phosphine, and 1.34 g (4.1 mmol) of cesium carbonate, and the mixture was stirred at 80°C for 48 hours. The insolubles were filtered off, the filtrate was concentrated under reduced pressure, and the residue was purified by silica gel column chromatography (eluting solvent: n-hexane/ethyl acetate = 9/1) to obtain 68 mg (yield 22%) of the title compound as a colorless oil.
¹H-NMR(CDCl₃) δ: 0.98 (3H, t, J = 7.4Hz), 1.33 (3H, t, J = 7.1Hz), 1.70-1.82 (2H, m), 1.90 (2H, q, J = 7.4Hz), 2.35-2.45 (2H, m), 3.10-3.20 (2H, m), 3.30-3.41 (2H, m), 4.28 (2H, q, J = 7.1Hz), 6.75-6.85 (2H, m), 7.15-7.50 (8H, m).

### Reference Example 32

### 1-Cyclohexylmethyl-4-[phenyl(propionyl)amino]piperidine-4-carboxylic acid ethyl ester oxalate

To a solution of 210 mg (0.69 mmol) of 4-[phenyl(propionyl)amino]piperidine-4-carboxylic acid ethyl ester obtained by the method described in J. Org. Chem., vol.55, pp.4207 published in 1990, and 93 mg (0.83 mmol) of cyclohexylaldehyde in a mixture of 4 ml of chloroform and 1 ml of acetic acid was added 176 mg (0.83 mmol) of sodium triacetoxyborohydride, and the mixture was stirred at room temperature for 16 hours. To the reaction mixture were added ethyl acetate, and an aqueous sodium bicarbonate solution, followed by extraction. The organic layer was washed with water, and concentrated under reduced pressure, the residue was purified by silica gel column chromatography (eluting solvent: n-hexane/ethyl acetate = 6/4), and the eluate was recrystallized in a solvent of diisopropyl ether by addition of oxalic acid, to obtain 180 mg (yield 53%) of the title compound as a white powder. ¹H-NMR(DMSO-d₆) δ: 0.82 (3H, t, J = 7.4Hz), 1.00-1.20 (5H, m), 1.22 (3H, t, J = 7.1Hz), 1.50-1.72 (6H, m), 1.78 (2H, q, J = 7.4Hz), 1.80-1.90 (2H, m), 2.15-2.25 (2H, m), 2.65-2.75 (2H, m), 2.82-3.00 (2H, m), 3.10-3.26 (2H, m), 4.14 (2H, q, J = 7.1Hz), 7.25-7.35 (2H, m), 7.40-7.50 (3H, m).

### Reference Example 33

### N-[1-Benzyl-4-(4-methylthiazol-2-yl)-4-piperidinyl]-N-(3-chlorophenyl)propionamide

### Step 1

To a tetrahydrofuran solution (10 ml) of 4-methylthiazole (0.79 g, 8.0 mmol) was added dropwise 2-butyllithium (1.6 m hexane solution, 5.0 ml, 8.0 mmol) at - 78°C, and the mixture was stirred for 20 minutes. At the same temperature, a tetrahydrofuran solution (5 ml) of 1-benzyl-4-(3-chlorophenylamino)piperidine-4-carbonitrile (1.30 g, 4.0 mmol) was added dropwise, and a temperature was raised slowly to 0°C over 30 minutes. To the reaction mixture was added water (30 ml), the mixture was extracted with ethyl acetate (3×40 ml), the organic layer was washed with water (40 ml), and dried over anhydrous magnesium sulfate, and the solvent was distilled off. The residue was purified by silica gel column chromatography (solvent: hexane-ethyl acetate) to obtain [1-benzyl-4-(4-methylthiazol-2-yl)-4-piperidinyl]-(3-chlorophenyl)amine (1.56 g, 98%).
¹H-NMR(CDCl₃) δ: 2.10-2.30 (4H, m), 2.35-2.55 (2H, m), 2.45 (3H, s), 2.70-2.80 (2H, m), 3.52 (2H, s), 4.29 (1H, s), 6.31 (1H, d, J = 8.2Hz), 6.45-6.50 (1H, m), 6.60-6.70 (1H, m), 6.80 (1H, s), 6.96 (1H, t, J = 8.1Hz), 7.20-7.40 (5H, m) .

### Step 2

To a chloroform solution (15 ml) of [1-benzyl-4-(4-methylthiazol-2-yl)-4-piperidinyl]-(3-chlorophenyl)amine (0.4 g, 1.0 mmol) was added dropwise propionyl chloride (0.65 ml, 7.0 mmol) at room temperature, and the mixture was heated under reflux for 48 hours. The reaction solvent was distilled off, an aqueous saturated sodium bicarbonate solution (20 ml) was added, the mixture was extracted with chloroform (4x20 ml), the organic layer was dried over anhydrous magnesium sulfate, and the solvent was distilled off. The residue was purified by silica gel column chromatography (solvent: hexane-ethyl acetate) to obtain the title compound (1.56 g, 98%).
¹H-NMR(CDCl₃) δ: 0.88 (3H, t, J = 7.3Hz), 1.80 (2H, q, J = 7.4Hz), 2.00-2.20 (2H, m), 2.20-2.40 (2H, m), 2.44 (3H, s), 2.50-2.80 (4H, m), 3.41 (2H, s), 6.84 (1H, s), 7.20-7.50 (9H, m).

### Reference Example 34

### N-[1-Benzyl-4-(4-methylthiazol-2-yl)-4-piperidinyl]-N-(4-chlorophenyl)propionamide

According to the same manner as that of Reference Example 33, 1-benzyl-4-(4-chlorophenylamino)piperidine-4-carbonitrile was used in place of 1-benzyl-4-(3-chlorophenylamino)piperidine-4-carbonitrile, the title compound (0.40 g, 87%) was obtained as a white powder.
¹H-NMR(CDCl₃) δ: 0.87 (3H, t, J = 7.4Hz), 1.79 (2H, q, J = 7.4Hz), 2.00-2.20 (2H, m), 2.20-2.35 (2H, m), 2.43 (3H, s), 2.50-2.70 (4H, m), 3.41 (2H, s), 6.83 (1H, s), 7.20-7.40 (9H, m).

### Reference Example 35

### N-[1-Benzyl-4-(4-methylthiazol-2-yl)-4-piperidinyl]-N-(4-methylphenyl)propionamide

According to the same manner as that of Reference Example 33, 1-benzyl-4-(4-methylphenylamino)piperidine-4-carbonitrile was used in place of 1-benzyl-4-(3-chlorophenylamino)piperidine-4-carbonitrile, the title compound (0.37 g, 84%) was obtained as a white powder.
¹H-NMR(CDCl₃) δ: 0.86 (3H, t, J = 7.4Hz), 1.81 (2H, q, J = 7.4Hz), 2.00-2.20 (2H, m), 2.25-2.40 (2H, m), 2.39 (3H, s), 2.44 (3H, s), 2.55-2.70 (4H, m), 3.41 (2H, s), 6.82 (1H, s), 7.15-7.35 (9H, m).

### Reference Example 36

### N-[1-Benzyl-4-(4-methylthiazol-2-yl)-4-piperidinyl]-N-(3-methylphenyl)propionamide

According to the same manner as that of Reference Example 33, 1-benzyl-4-(3-methylphenylamino)piperidine-4-carbonitrile was used in place of 1-benzyl-4-(3-chlorophenylamino)piperidine-4-carbonitrile, the title compound (0.30 g, 69%) was obtained as a white powder.
¹H-NMR(CDCl₃) δ: 0.87 (3H, t, J = 7.4Hz), 1.81 (2H, q, J = 7.4Hz), 2.00-2.15 (2H, m), 2.25-2.40 (2H, m), 2.38 (3H, s), 2.44 (3H, s), 2.55-2.75 (4H, m), 3.41 (2H, s), 6.82 (1H, s), 7.10-7.35 (9H, m).

### Reference Example 37

### N-[1-Benzyl-4-(4-methylthiazol-2-yl)-4-piperidinyl]-N-(2-methylphenyl)propionamide

According to the same manner as that of Reference Example 33, 1-benzyl-4-(2-methylphenylamino)piperidine-4-carbonitrile was used in place of 1-benzyl-4-(3-chlorophenylamino)piperidine-4-carbonitrile, the title compound (0.42 g, 97%) was obtained as a white powder.
¹H-NMR(CDCl₃) δ: 0.87 (3H, t, J = 7.4Hz), 1.70 (2H, q, J = 7.4Hz), 1.98 (2H, t, J = 12.0Hz), 2.15-2.40 (2H, m), 2.36 (3H, s), 2.45 (3H, s), 2.55-2.75 (4H, m), 3.36 (2H, s), 6.87 (1H, s), 7.10-7.45 (9H, m).

### Reference Example 38

### N-[1-Benzyl-4-(4-methylthiazol-2-yl)-4-piperidinyl]-N-phenylpropionamide oxalate

To a solution of 317 mg (3.2 mmol) of 4-methylthiazole in 10 ml of THF was added 2.0 ml (3.2 mmol) of a 1.6 m n-butyllithium hexane solution under cooling at -78°C, and the mixture was stirred for 15 minutes. After a temperature was raised to -30°C, 466 mg (1.6 mmol) of 1-benzyl-4-(phenylamino)piperidine-4-carbonitrile was added thereto, the mixture was stirred for 30 minutes, and a temperature was raised to 0°C. To the reaction mixture were added ethyl acetate, and water, followed by extraction. The organic layer was washed with water, and concentrated under reduced pressure, and the residue was purified by silica gel column chromatography (eluting solvent: n-hexane/ethyl acetate = 4/6) to obtain 360 mg (yield 62%) of [1-benzyl-4-(4-methylthiazol-2-yl)-4-piperidinyl]-phenylamine as a colorless oil.

According to the same manner as that of Reference Example 23, from the resulting [1-benzyl-4-(4-methylthiazol-2-yl)-4-piperidinyl]phenylamine, 135 mg (yield 60%) of the title compound was obtained as a white powder.
¹H-NMR(DMSO-d₆) δ: 0.73 (3H, t, J = 7.4Hz), 1.74 (2H, q, J = 7.4Hz), 2.05-2.20 (2H, m), 2.30 (3H, s), 2.48-2.56 (2H, m), 2.82-3.05 (4H, m), 3.99 (2H, br s), 7.19 (1H, s), 7.30-7.40 (8H, m), 7.40-7.48 (2H, m).

### Reference Example 39

### N-[1-Benzyl-4-(4-methylthiazol-2-yl)-4-piperidinyl]-N-phenylacetamide oxalate

According to the same manner as that of Step 2 of Reference Example 23, from [1-benzyl-4-(4-methylthiazol-2-yl)-4-piperidinyl]phenylamine obtained in Reference Example 38 and acetyl chloride, 110 mg (yield 50%) of the title compound was obtained as a white powder.
¹H-NMR(DMSO-d₆) δ: 1.53 (3H, s), 2.05-2.20 (2H, m), 2.30 (3H, s), 2.43-2.55 (2H, m), 2.82-3.08 (4H, m), 4.00 (2H, br s), 7.20 (1H, s), 7.30-7.40 (8H, m), 7.40-7.49 (2H, m).

### Reference Example 40

### N-[1-Benzyl-4-(sec-butyl)-4-piperidinyl]-N-phenylpropionamide oxalate

To a solution of 466 mg (6 mmol) of 1-benzyl-4-(phenylamino)piperidine-4-carbonitrile obtained by the method described in Synthetic Communications, vol.27, pp.923-937 published in 1997 in 5 ml of THF was added 6.4 ml (6.4 mmol) of a 1.0 M sec-butyllithium hexane solution under ice-cooling, and the mixture was stirred at room temperature for 2 hours. To the reaction mixture were added ethyl acetate and water, followed by extraction. The organic layer was washed with water, and concentrated under reduced pressure, and the residue was purified by alumina column chromatography (eluting solvent: n-hexane/ethyl acetate = 98/2) to obtain 160 mg (yield 31%) of [1-benzyl-4-(sec-butyl)-4-piperidinyl]phenylamine as a colorless oil.

According to the same manner as that of Step 2 of Reference Example 23, from the resulting [1-benzyl-4-(sec-butyl)-4-piperidinyl]phenylamine, 71 mg (yield 30%) of the title compound was obtained as a white powder.
¹H-NMR(DMSO-d₆) δ: 0.79 (3H, t, J = 7.4Hz), 0.80-0.85 (3H, m), 0.88 (3H, t, J = 7.1Hz), 1.00-1.20 (1H, m), 1.20-1.38 (2H, m), 1.70 (2H, q, J = 7.4Hz), 2.00-2.25 (4H, m), 2.75-2.95 (4H, m), 4.05 (2H, s), 7.10-7.20 (2H, m), 7.30-7.42 (8H, m).

### Reference Example 41

### N-[1-Benzyl-4-(2-pyridinyl)-4-piperidinyl]-N-phenylpropionamide oxalate

According to the same manner as that of Reference Example 38, 2-bromopyridine was used in place of 4-methylthiazole, 31 mg (yield 32%) of the title compound was obtained as a white powder.
¹H-NMR(DMSO-d₆) δ: 0.65 (3H, t, J = 7.4Hz), 1.67 (2H, q, J = 7.4Hz), 1.95-2.15 (2H, m), 2.62-2.80 (4H, m), 2.85-3.04 (2H, m), 3.96 (2H, s), 7.23-7.27 (1H, m), 7.36 (5H, m), 7.38-7.50 (5H, m), 7.65-7.80 (2H, m), 8.54 (1H, d, J = 4.5Hz) .

### Reference Example 42

### N-[1-Benzyl-4-(2-thiazolyl)-4-piperidinyl]-N-phenylpropionamide maleate

According to the same manner as that of Reference Example 38, thiazole was used in place of 4-methylthiazole, and maleic acid was used in place of oxalic acid, 31 mg (yield 32%) of the title compound was obtained as a white powder.
¹H-NMR(DMSO-d₆) δ: 0.73 (3H, t, J = 7.3Hz), 1.76 (2H, q, J = 7.3Hz), 2.00-2.25 (2H, m), 2.50-2.68 (2H, m), 3.05-3.25 (4H, m), 4.18 (2H, s), 6.04 (2H, s), 7.32-7.52 (10H, m), 7.70-7.74 (2H, m).

### Reference Example 43

### N-[1-Benzyl-4-(4,5-dimetylthiazol-2-yl)-4-piperidinyl]-N-phenylpropionamide maleate

According to the same manner as that of Reference Example 38, 4,5-dimethylthiazole was used in place of 4-methylthiazole, and maleic acid was used in place of oxalic acid, 380 mg (yield 65%) of the title compound was obtained as a white powder.
¹H-NMR(DMSO-d₆) δ: 0.74 (3H, t, J = 7.4Hz), 1.75 (2H, q, J = 7.4Hz), 1.88-2.10 (2H, m), 2.17 (3H, s), 2.30 (3H, s), 2.47-2.63 (2H, m), 3.05-3.25 (4H, m), 4.18 (2H, s), 6.03 (2H, s), 7.35-7.52 (10H, m).

### Reference Example 44

### N-[1-Benzyl-4-(4-methylthiazol-2-yl)-4-piperidinyl]-N-phenylacetamide

According to the same manner as that of Reference Example 39, 5.35 g (yield 80%) of the title compound was obtained as a colorless oil.
¹H-NMR(CDCl₃) δ: 1.62 (3H, s), 2.05-2.20 (2H, m), 2.25-2.39 (2H, m), 2.45 (3H, s), 2.55-2.75 (4H, m), 3.41 (2H, s), 6.84 (1H, s), 7.20-7.32 (5H, m), 7.33-7.45 (5H, m).

### Reference Example 45

### N-[1-(4-Chlorobenzyl)-4-(4-methylthiazol-2-yl)-4-piperidinyl]-N-phenylacetamide

### Step 1

To a solution of 2.00 g (4.93 mmol) of N-[1-benzyl-4-(4-methylthiazol-2-yl)-4-piperidinyl]-N-phenylacetamide obtained in Reference Example 44 in 50 ml of chloroform was added 846 mg (5.92 mmol) of 1-chloroethyl chloroformate under ice-cooling, and the mixture was stirred for 15 minutes, and heated to reflux for 2 hours. After concentration under reduced pressure, to the residue was added 20 ml of methanol, and the mixture was refluxed for 4 hours. The reaction mixture was concentrated under reduced pressure, and to the residue were added ethyl acetate and a 2 N aqueous sodium hydroxide solution, followed by extraction. The organic layer was washed with water, dried over magnesium sulfate, and concentrated under reduced pressure to obtain 1.50 g (yield 96%) of N-[4-(4-methylthiazol-2-yl)-4-piperidinyl]-N-phenylacetamide as a colorless oil.

### Step 2

To a solution of 500 mg (1.58 mmol) of the resulting N-[4-(4-methyltihazol-2-yl)-4-piperidinyl]-N-phenylacetamide and 267 mg (1.90 mmol) of 4-chlorobenzaldehyde in a mixture of 8 ml of chloroform and 2 ml of acetic acid was added 403 mg (1.90 mmol) of sodium triacetoxyborohydride, and the mixture was stirred at room temperature for 16 hours. To the reaction mixture were added ethyl acetate and an aqueous sodium bicarbonate solution, followed by extraction. The organic layer was washed with water, and concentrated under reduced pressure, and the residue was purified by silica gel column chromatography (eluting solvent: n-hexane/ethyl acetate = 7/3) to obtain 420 mg (yield 60%) of the title compound as a colorless oil.
¹H-NMR(CDCl₃) δ: 1.63 (3H, s), 2.00-2.17 (2H, m), 2.25-2.37 (2H, m), 2.45 (3H, s), 2.52-2.61 (2H, m), 2.62-2.73 (2H, m), 3.37 (2H, s), 6.84 (1H, s), 7.15-7.28 (4H, m), 7.33-7.45 (5H, m).

### Reference Example 46

### N-[1-(3-Chlorobenzyl)-4-(4-methylthiazol-2-yl)-4-piperidinyl]-N-phenylacetamide

According to the same manner as that of Reference Example 45, 3-chlorobenzaldehyde was used in place of 4-chlorobenzaldehyde to obtain 430 mg (yield 62%) of the title compound as a colorless oil.
¹H-NMR(CDCl₃) δ: 1.63 (3H, s), 2.01-2.19 (2H, m), 2.25-2.36 (2H, m), 2.45 (3H, s), 2.52-2.61 (2H, m), 2.62-2.74 (2H, m), 3.37 (2H, s), 6.84 (1H, s), 7.09-7.28 (4H, m), 7.33-7.48 (5H, m).

### Reference Example 47

### N-[1-(2-Chlorobenzyl)-4-(4-methylthiazol-2-yl)-4-piperidinyl]-N-phenylacetamide

According to the same manner as that of Reference Example 45, 2-chlorobenzaldehyde was used in place of 4-chlorobenzaldehyde to obtain 510 mg (yield 73%) of the title compound as a colorless oil.
¹H-NMR(CDCl₃) δ: 1.63 (3H, s), 2.01-2.20 (2H, m), 2.25-2.36 (2H, m), 2.45 (3H, s), 2.54-2.63 (2H, m), 2.62-2.74 (2H, m), 3.42 (2H, s), 6.84 (1H, s), 7.08-7.30 (4H, m), 7.33-7.49 (5H, m).

### Reference Example 48

### N-[1-(2-Thienylmethyl)-4-(4-methylthiazol-2-yl)-4-piperidinyl]-N-phenylacetamide

According to the same manner as that of Reference Example 45, 2-thiophenaldehyde was used in place of 4-chlorobenzaldehyde to obtain 210 mg (yield 32%) of the title compound as a colorless oil.
¹H-NMR(CDCl₃) δ: 1.63 (3H, s), 2.02-2.18 (2H, m), 2.28-2.38 (2H, m), 2.44 (3H, s), 2.60-2.73 (4H, m), 3.63 (2H, s), 6.83 (1H, s), 6.83-6.84 (1H, m), 6.88-6.92 (1H, m), 7.19 (1H, d, J = 5.1Hz), 7.33-7.48 (5H, m).

### Reference Example 49

### N-[1-(2-Furylmethyl)-4-(4-methylthiazol-2-yl)-4-piperidinyl]-N-phenylacetamide

According to the same manner as that of Reference Example 45, 2-furaldehyde was used in place of 4-chlorobenzaldehyde to obtain 190 mg (yield 30%) of the title compound as a colorless oil.
¹H-NMR(CDCl₃) δ: 1.62 (3H, s), 2.03-2.18 (2H, m), 2.27-2.38 (2H, m), 2.44 (3H, s), 2.59-2.71 (4H, m), 3.44 (2H, s), 6.12-6.13 (1H, m), 6.26-6.27 (1H, m), 6.83 (1H, s), 7.32-7.33 (1H, m), 7.34-7.44 (5H, m).

### Reference Example 50

### N-[1-Benzyl-4-(1-methyl-1H-imidazol-2-yl)-4-piperidinyl]-N-phenylacetamide oxalate

### Step 1

A solution of 1-methylimidazole (0.56 g, 6.9 mmol) in tetrahydrofuran (5 ml) was cooled to -78°C. N-butyllithium (1.6 M, 4.4 ml) was added thereto, the mixture was stirred at -78°C for 20 minutes, and a solution of 1-benzyl-4-(phenylamino)piperidine-4-carbonitrile (1.0 g, 3.4 mmol) in tetrahydrofuran (5 ml)was added at once. After stirring at -78°C to 0°C for 3 hours, water (10 ml) was added to the reaction mixture, followed by extraction with ethyl acetate. The organic layer was washed successively with water and an aqueous saturated sodium chloride solution, and dried over anhydrous magnesium sulfate, and the solvent was distilled off. Precipitated crystals were washed with isopropyl ether to obtain [1-benzyl-4-(1-methyl-1H-imidazol-2-yl)-4-piperidinyl]-phenyl-amine (0.41 g, 34%).
¹H-NMR (CDCl₃) δ: 2.19 (2H, m), 2.31-2.52 (4H, m), 2.75 (2H, m), 3.54 (2H, s), 3.72 (3H, s), 3.98 (1H, br s), 6.26 (2H, d, J = 8.6Hz), 6.68 (1H, t, J = 7.3Hz), 6.76 (1H, s), 7.00-7.05 (3H, m), 7.24-7.36 (5H, m).

### Step 2

A solution of [1-benzyl-4-(1-methyl-1H-imidazol-2-yl)-4-piperidinyl]-phenyl-amine (0.20 g, 0.58 mmol) in acetic anhydride (6 ml) was stirred at 100°C for 48 hours, and the solvent was concentrated under reduced pressure. To the residue was added an aqueous saturated sodium bicarbonate solution, the mixture was extracted with ethyl acetate, the organic layer was washed with an aqueous saturated sodium chloride solution, and dried over anhydrous magnesium sulfate, and the solvent was distilled off. The residue was subjected to silica gel column chromatography, and fractions eluted with ethyl acetate-ethanol (10:1) were collected, and concentrated to obtain N-[1-benzyl-4-(1-methyl-1H-imidazol-2-yl)-4-piperidinyl]-N-phenylacetamide as a yellow oil.
¹H-NMR (CDCl₃) δ: 1.63 (3H, s), 2.02 (2H, m), 2.15-2.30 (4H, m), 2.79 (2H, m), 3.41 (2H, s), 3.98 (3H, s), 6.78 (1H, s), 7.02 (1H, s), 7.19-7.28 (7H, m), 7.36-7.43 (3H, m).

To a solution of the resulting N-[1-benzyl-4-(1-methyl-1H-imidazol-2-yl)-4-piperidinyl]-N-phenylacetamide in ethyl acetate (6 ml) was added a solution of oxalic acid (0.03 g, 0.33 mmol) in isopropanol (1 ml), and the mixture was stirred for 30 minutes. Precipitated crystals were recrystallized from ethyl acetate-isopropyl ether to obtain the title compound (0.11 g, 43%).

### Reference Example 51

### 1-Benzyl-4-[ethoxycarbonyl(phenyl)amino]piperidine-4-carboxylic acid ethyl ester

This compound was purchased from Ambinter Sarl (France).

### Reference Example 52

### N-[1-Benzyl-4-(2-pyrimidiylmethyl)-4-piperidinyl]-N-phenylacetamide oxalate

### Step 1

A solution of 2-methylpyridine (0.64 g, 6.9 mmol) in tetrahydrofuran (4 ml) was cooled to -78°C. N-butyllithium (1.6 m, 4.4 ml) was added thereto, the mixture was stirred at -78°C for 20 minutes, and a solution of 1-benzyl-4-(phenylamino)piperidine-4-carbonitrile (1.0 g, 3.4 mmol) in tetrahydrofuran (6 ml) was added at once. After stirring at -78°C to 0°C for 3 hours, to the reaction mixture was added water (10 ml), followed by extraction with ethyl acetate. The organic layer was washed successively with water and an aqueous saturated sodium chloride solution, and dried over anhydrous magnesium sulfate, and the solvent was distilled off. The residue was subjected to silica gel column chromatography, and fractions eluted with ethyl acetate-ethanol (10:1) were collected, and concentrated to obtain [1-benzyl-4-(2-pyrdinylmethyl)-4-piperidinyl]phenylamine (0.80 g, 65%).
¹H-NMR (CDCl₃) δ: 1.81 (2H, m), 1.99 (2H, m), 2.36 (2H, m), 2.59 (2H, m), 3.21 (2H, s), 3.49 (2H, s), 6.7-6.79 (3H, m), 7.00 (1H, d, J = 7.7Hz), 7.08 (1H, m), 7.17 (2H, t, J = 7.3Hz), 7.2-7.3 (5H, s), 7.47 (1H, m), 8.51 (1H, d, J = 4.9Hz).

### Step 2

A solution of [1-benzyl-4-(2-pyridinylmethyl)-4-piperidinyl]phenylamine (0.20 g, 0.58 mmol) in acetic anhydride (5 ml) was stirred at 100°C for 22 hours, and the solvent was concentrated under reduced pressure. To the residue was added an aqueous saturated sodium bicarbonate solution, this was extracted with ethyl acetate, the organic layer was washed with an aqueous saturated sodium chloride solution, and dried over anhydrous magnesium sulfate, and the solvent was distilled off. The residue was subjected to silica gel column chromatography, and fractions eluted with ethyl acetate-ethanol (10:1) were collected and concentrated to obtain N-[1-benzyl-4-(2-pyridinylmethyl)-4-piperidinyl]-N-phenylacetamide as a yellow oil.
¹H-NMR (CDCl₃) δ: 1.58 (3H, s), 1.83 (2H, m), 2.30-2.49 (4H, m), 2.74 (2H, m), 3.52 (2H, s), 3.78 (2H, s), 7.10-7.35 (12H, m), 7.65 (1H, t, J = 7.7Hz), 8.59 (1H, d, J = 4.9Hz).

To a solution of the resulting N-[1-benzyl-4-(2-pyridinylmethyl)-4-piperidinyl]-N-phenylacetamide in ethyl acetate (4 ml) was added a solution of oxalic acid (0.04 g, 0.42 mmol) in isopropanol (1 ml), and the mixture was stirred for 30 minutes. Precipitated crystals were recrystallized from ethyl acetate-isopropyl ether to obtain the title compound (0.17 g, 50%).

### Reference Example 53

### N-[1-Benzyl-4-(6-methyl-2-pyridinyl)-4-piperidinyl]-N-phenylacetamide oxalate

### Step 1

A solution of 2-bromo-6-methylpyridine (1.20 g, 7.0 mmol) in tetrahydrofuran (4 ml) was cooled to -78°C. N-butyllithium (1.6 m, 4.4 ml) was added thereto, the mixture was stirred at -78°C for 20 minutes, and a solution of 1-benzyl-4-(phenylamino)piperidine-4-carbonitrile (1.0 g, 3.4 mmol) in tetrahydrofuran (6 ml) was added at once. After stirring at -78°C to 0°C for 3 hours, water (10 ml) was added to the reaction mixture, followed by extraction with ethyl acetate. The organic layer was washed successively with water and an aqueous saturated sodium chloride solution, and dried over anhydrous magnesium sulfate, and the solvent was distilled off. The residue was subjected to silica gel column chromatography, and fractions eluted with hexane-ethyl acetate (1:1) were collected and concentrated to obtain [1-benzyl-4-(6-methyl-2-pyridinyl)-4-piperidinyl]-phenyl-amine (0.50 g, 40%).
¹H-NMR (CDCl₃) δ: 2.05 (2H, m), 2.33 (2H, m), 2.49 (2H, m), 2.55 (3H, s), 2.78 (2H, m), 3.53 (2H, s), 6.34 (2H, d, J = 7.7Hz), 6.61 (1H, t, J = 7.3Hz), 6.94-7.03 (3H, m), 7.20-7.35 (6H, s), 7.45 (1H, t, J = 7.7Hz).

### Step 2

A solution of [1-benzyl-4-(6-methyl-2-pyridinyl)-4-piperidinyl]-phenyl-amine (0.25 g, 0.70 mmol) in acetic anhydride (5 ml) was stirred at 100°C for 20 hours, and the solvent was concentrated under reduced pressure. To the residue was added an aqueous saturated sodium bicarbonate solution, the mixture was extracted with ethyl acetate, the organic layer was washed with an aqueous saturated sodium chloride solution, and dried over anhydrous magnesium sulfate, and the solvent was distilled off. The residue was subjected to silica gel column chromatography, and fractions eluted with ethyl acetate-ethanol (10:1) were collected and concentrated to obtain N-[1-benzyl-4-(6-methyl-2-pyridinyl)-4-piperidinyl]-N-phenylacetamide as a yellow oil.
¹H-NMR (CDCl₃) δ: 1.55 (3H, s), 1.93-2.10 (4H, m), 2.55 (3H, s), 2.62 (2H, m), 2.83 (2H, m), 3.35 (2H, s), 6.97 (1H, d, J = 7.1Hz), 7.10-7.42 (8H, m), 7.49-7.57 (4H, m).

To a solution of the resulting N-[1-benzyl-4-(6-methyl-2-pyridinyl)-4-piperidinyl]-N-phenylacetamide in ethyl acetate (6 ml) was added a solution of oxalic acid (0.04 g, 0.42 mmol) in isopropanol (1 ml), and the mixture was stirred for 30 minutes. Precipitated crystals were recrystallized from ethyl acetate-isopropyl ether to obtain the title compound (0.14 g, 44%).

### Reference Example 54

### N-[1-Benzyl-4-(6-methyl-2-pyridinyl)-4-piperidinyl]-2,2,2-trifluoro-N-phenylacetamide oxalate

To a solution of [1-benzyl-4-(6-methyl-2-pyridinyl)-4-piperidinyl]-phenyl-amine (0.23 g, 0.64 mmol) obtained in Step 1 of Reference Example 53 in tetrahydrofuran (3 ml) was added trifluoroacetic anhydride (1.35 g, 6.4 mmol), the mixture was stirred at room temperature for 16 hours, and the solvent was concentrated under reduced pressure. To the residue was added an aqueous saturated sodium bicarbonate solution, the mixture was extracted with ethyl acetate, the organic layer was washed with an aqueous saturated sodium chloride solution, and dried over anhydrous magnesium sulfate, and the solvent was distilled off. The residue was subjected to alumina column chromatography, and fractions eluted with hexane-ethyl acetate (5:1) to obtain N-[1-benzyl-4-(6-methyl-2-pyridinyl)-4-piperidinyl]-2,2,2-trifluoro-N-phenylacetamide as a yellow oil.
¹H-NMR (CDCl₃) δ: 1.88-2.08 (4H, m), 2.56 (3H, s), 2.66 (2H, m), 2.82 (2H, m), 3.54 (2H, s), 7.02 (1H, d, J = 7.4Hz), 7.18-7.30 (5H, m), 7.38-7.69 (4H, m), 7.57-7.61 (3H, m).

To a solution of the resulting N-[1-benzyl-4-(6-methyl-2-pyridinyl)-4-piperidinyl]-2,2,2-trifluoro-N-phenylacetamide in ethyl acetate (5 ml) was added a solution of oxalic acid (0.04 g, 0.42 mmol) in isopropanol (1 ml), and the mixture was stirred for 30 minutes. Precipitated crystals were recrystallized from ethyl acetate-hexane to obtain the title compound (0.21 g, 61%).

### Reference Example 55

### N-[1-Benzyl-4-(4-methyl-2-pyridinyl)-4-piperidinyl]-N-phenylacetamide oxalate

### Step 1

A solution of 2-bromo-4-methylpyridine (1.20 g, 7.0 mmol) in tetrahydrofuran (4 ml) was cooled to -78°C. N-butyllithium (1.6 M, 4.4 ml) was added thereto, the mixture was stirred at -78°C for 20 minutes, and a solution of 1-benzyl-4-(phenylamino)piperidine-4-carbonitrile (1.0 g, 3.4 mmol) in tetrahydrofuran (6 ml)was added at once. After stirring at -78°C to 0°C for 3 hours, water (10 ml) was added to the reaction mixture, followed by extraction with ethyl acetate. The organic layer was washed successively with water and an aqueous saturated sodium chloride solution, and dried over anhydrous magnesium sulfate, and the solvent was distilled off. The residue was subjected to silica gel column chromatography, and fractions eluted with hexane-ethyl acetate (1:1) were collected and concentrated to obtain [1-benzyl-4-(4-methyl-2-pyridinyl)-4-piperidinyl]-phenyl-amine (0.25 g, 20%)
¹H-NMR (CDCl₃) δ: 2.18 (2H, m), 2.31 (3H, s), 2.35-2.48 (4H, m), 2.75 (2H, m), 3.53 (2H, s), 6.34 (2H, d, J = 8.3Hz), 6.62 (1H, t, J = 7.3Hz), 6.94-7.04 (3H, m), 7.20-7.35 (5H, s), 7.40 (1H, s), 8.46 (1H, d, J = 4.9Hz).

### Step 2

A solution of [1-benzyl-4-(4-methyl-2-pyridinyl)-4-piperidinyl]-phenyl-amine (0.25 g, 0.70 mmol) in acetic anhydride (5 ml) was stirred at 100°C for 60 hours, and the solvent was concentrated under reduced pressure. To the residue was added an aqueous saturated sodium bicarbonate solution, the mixture was extracted with ethyl acetate, the organic layer was washed with an aqueous saturated sodium chloride solution, and dried over anhydrous magnesium sulfate, and the solvent was distilled off. The residue was subjected to silica gel column chromatography, and fractions eluted with ethyl acetate-ethanol (10:1) were colleted and concentrated to obtain N-[1-benzyl-4-(4-methyl-2-pyridinyl)-4-piperidinyl]-N-phenylacetamide as a yellow oil.
¹H-NMR (CDCl₃) δ: 1.55 (3H, s), 1.93-2.08 (4H, m), 2.40 (3H, s), 2.62 (2H, m), 2.83 (2H, m), 3.34 (2H, s), 6.96 (1H, d, J = 4.4Hz), 7.10-7.30 (5H, m), 7.34-7.53 (6H, m), 8.46 (1H, d, J = 4.9Hz).

To a solution of the resulting N-[1-benzyl-4-(4-methyl-2-pyridinyl)-4-piperidinyl]-N-phenylacetamide in ethyl acetate (6 ml) was added a solution of oxalic acid (0.04 g, 0.42 mmol) in isopropanol (1 ml), and the mixture was stirred for 30 minutes. Precipitated crystals were recrystallized from ethyl acetate-isopropyl ether to obtain the title compound (0.09 g, 26%).

### Reference Example 56

### N-1-Benzyl-4-(5-methyl-2-pyridinyl)-4-piperidinyl]-N-phenylacetamide oxalate

### Step 1

A solution of 2-bromo-5-methylpyridine (1.20 g, 7.0 mmol) in tetrahydrofuran (4 ml) was cooled to -78 °C. N-butyllithium (1.6 m, 4.4 ml) was added thereto, the mixture was stirred at -78°C for 20 minutes, and a solution of 1-benzyl-4-(phenylamino)piperidine-4-carbonitrile (1.0 g, 3.4 mmol) in tetrahydrofuran (6 ml) was added at once. After stirring at -78°C to 0°C for 3 hours, water (10 ml) was added to the reaction mixture, followed by extraction with ethyl acetate. The organic layer was washed successively with water and an aqueous saturated sodium chloride solution, and dried over anhydrous magnesium sulfate, and the solvent was distilled off. The residue was subjected to silica gel column chromatography, and fractions eluted with hexane-ethyl acetate (1:1) were collected and concentrated to obtain [1-benzyl-4-(5-methyl-2-pyridinyl)-4-piperidinyl]-phenyl-amine (0.58 g, 47%).
¹H-NMR (CDCl₃) δ: 2.07 (2H, m), 2.31 (3H, s), 2.27-2.48 (4H, m), 2.74 (2H, m), 3.53 (2H, s), 6.32 (2H, d, J = 8.9Hz), 6.61 (1H, m), 7.00 (2H, dd, J = 8.4, 7.4Hz), 7.20-7.45 (7H, s), 8.43 (1H, s).

### Step 2

A solution of [1-benzyl-4-(5-methyl-2-pyridinyl)-4-piperidinyl]-phenyl-amine (0.28 g, 0.78 mmol) in acetic anhydride (6 ml) was stirred at 100°C for 48 hours, and the solvent was concentrated under reduced pressure. To the residue was added an aqueous saturated sodium bicarbonate solution, the mixture was extracted with ethyl acetate, the organic layer was washed with an aqueous saturated sodium chloride solution, and dried over anhydrous magnesium sulfate, and the solvent was distilled off. The residue was subjected to silica gel column chromatography, and fractions eluted with ethyl acetate-ethanol (10:1) were collected and concentrated to obtain N-[1-benzyl-4-(5-methyl-2-pyridinyl)-4-piperidinyl]-N-phenylacetamide as a yellow oil.
¹H-NMR (CDCl₃) δ: 1.54 (3H, s), 1.91-2.05 (4H, m), 2.33 (3H, s), 2.64 (2H, m), 2.86 (2H, m), 3.33 (2H, s), 7.18-7.26 (5H, m), 7.37-7.49 (6H, m), 7.63 (1H, d, J = 8.1Hz), 8.44 (1H, s).

To a solution of the resulting N-[1-benzyl-4-(5-methyl-2-pyridinyl)-4-piperidinyl]-N-phenylacetamide in ethyl acetate (6 ml) was added a solution of oxalic acid (0.04 g, 0.42 mmol) in isopropanol (1 ml), and the mixture was stirred for 30 minutes. Precipitated crystals were recrystallized from ethyl acetate-isopropyl ether to obtain the title compound (0.17 g, 46%).

### Reference Example 57

### N-[1-Benzyl-4-(5-methyl-2-pyridinyl)-4-piperidinyl]-2,2,2-trifluoro-N-phenylacetamide oxalate

To a solution of [1-benzyl-4-(5-methyl-2-pyridinyl)-4-piperidinyl]-phenyl-amine (0.19 g, 0.53 ml) obtained in Step 1, Reference Example 56 in tetrahydrofuran (3 ml) was added trifluoroacetic anhydride (1.20 g, 5.7 mmol), the mixture was stirred at room temperature for 16 hours, and the solvent was concentrated under reduced pressure. To the residue was added an aqueous saturated sodium bicarbonate solution, the mixture was extracted with ethyl acetate, the organic layer was washed with an aqueous saturated sodium chloride solution, and dried over anhydrous magnesium sulfate, and the solvent was distilled off. The residue was subjected to alumina column chromatography, and fractions eluted with hexane-ethyl acetate (5:1) were collected and concentrated to obtain N-[1-benzyl-4-(5-methyl-2-pyridinyl)-4-piperidinyl]-2,2,2-trifluoro-N-phenylacetamide as a yellow oil.
¹H-NMR (CDCl₃) δ: 1.86-2.05 (4H, m), 2.35 (3H, s), 2.68 (2H, m), 2.86 (2H, m), 3.33 (2H, s), 7.18-7.28 (5H, m), 7.39-7.54 (6H, m), 7.62 (1H, d, J = 8.1Hz), 8.46 (1H, s).

To a solution of the resulting N-[1-benzyl-4-(5-methyl-2-pyridinyl)-2,2,2-trifluoro-4-piperidinyl]-N-phenylacetamide in ethyl acetate (6 ml) was added a solution of oxalic acid (0.04 g, 0.42 mmol) in isopropanol (1 ml), and the mixture was stirred for 30 minutes. Precipitated crystals were recrystallized from ethyl acetate-isopropyl ether to obtain the title compound (0.18 g, 63%).

### Reference Example 58

### N-[1-Benzyl-4-(6-bromo-2-pyridinyl)-4-piperidinyl]-N-phenylacetamide oxalate

### Step 1

A solution of 2,6-dibromopyridine (3.25 g, 13.7 mmol) in tetrahydrofuran (7 ml) was cooled to -78°C. N-butyllithium (1.6 M, 8.6 ml) was added thereto, the mixture was stirred at -78°C for 20 minutes, and a solution of 1-benzyl-4-(phenylamino)piperidine-4-carbonitrile (2.0 g, 6.9 mmol) in tetrahydrofuran (13 ml) was added at once. After stirring at -78°C to 0°C for 4 hours, water (10 ml) was added to the reaction mixture, followed by extraction with ethyl acetate. The organic layer was washed successively with water, and an aqueous saturated sodium chloride solution, and dried over anhydrous magnesium sulfate, and the solvent was distilled off. The residue was subjected to silica gel column chromatography, fractions eluted with hexane-ethyl acetate (1:2) were collected and concentrated, and the resulting crystals were washed with hexane, and dried to obtain [1-benzyl-4-(6-bromo-2-pyridinyl)-4-piperidinyl]phenylamine (1.46 g, 50%).
¹H-NMR (CDCl₃) δ: 2.02 (2H, m), 2.27 (2H, m), 2.46 (2H, m), 2.79 (2H, m), 3.52 (2H, s), 4.17 (1H, br s), 6.33 (2H, d, J = 8.6Hz), 6.65 (1H, t, J = 7.4Hz), 7.04 (2H, t, J = 7.4Hz), 7.20-7.33 (6H, s), 7.43 (1H, t, J = 7.7Hz), 7.52 (1H, m). Step 2

A solution of [1-benzyl-4-(6-bromo-2-pyridinyl)-4-piperidinyl]-phenyl-amine (0.25 g, 0.59 mmol) in acetic anhydride (5 ml) was stirred at 100°C for 48 hours, and the solvent was concentrated under reduced pressure. To the residue was added an aqueous saturated sodium bicarbonate solution, the mixture was extracted with ethyl acetate, the organic layer was washed with an aqueous saturated sodium chloride solution, and dried over anhydrous magnesium sulfate, and the solvent was distilled off. The residue was subjected to silica gel column chromatography, and fractions eluted with ethyl acetate-ethanol (10:1) were collected and concentrated to obtain N-[1-benzyl-4-(6-bromo-2-pyridinyl)-4-piperidinyl]-N-phenylacetamide as a yellow oil.
¹H-NMR (CDCl₃) δ: 1.57 (3H, s), 1.92-2.05 (4H, m), 2.63 (2H, m), 2.77 (2H, m), 3.35 (2H, s), 7.19-7.26 (5H, m), 7.31 (1H, d, J = 7.7Hz), 7.38-7.55 (6H, m), 7.65 (1H, d, J = 7.6Hz).

To a solution of the resulting N-1-bnzyl-4-(6-bromo-2-pyridinyl)-4-piperidinyl]-N-phenylacetamide in ethyl acetate (6 ml) was added a solution of oxalic acid (0.04 g, 0.42 mmol) in isopropanol (1 ml), and the mixture was stirred for 30 minutes. Precipitated crystals were recrystallized from ethyl acetate-isopropyl ether to obtain the title compound (0.19 g, 59%).

### Reference Example 59

### N-[1-Benzyl-4-(6-bromo-2-pyridinyl)-4-piperidinyl]-2,2,2-trifluoro-N-phenylacetamide

To a solution of [1-benzyl-4-(6-bromo-2-pyridinyl)-4-piperidinyl]-phenyl-amine (0.20 g, 0.47 mmol) obtained in Step 1 of Reference Example 58 in tetrahydrofuran (3 ml) was added trifluoroacetic anhydride (1.00 g, 4.7 mmol), the mixture was stirred at room temperature for 20 hours, and the solvent was concentrated under reduced pressure. To the residue was added an aqueous saturated sodium bicarbonate solution, the mixture was extracted with ethyl acetate, the organic layer was washed with an aqueous saturated sodium chloride solution, and dried over anhydrous magnesium sulfate, and the solvent was distilled off. The residue was subjected to alumina column chromatography, and fractions eluted with hexane-ethyl acetate (5:1) were collected and concentrated to obtain the title compound (0.20 g, 81%) as a yellow oil.
¹H-NMR (CDCl₃) δ: 1.89-2.05 (4H, m), 2.69 (2H, m), 2.77 (2H, m), 3.35 (2H, s), 7.19-7.26 (5H, m), 7.38 (1H, d, J = 7.6Hz), 7.41-7.64 (7H, m).

### Reference Example 60

### N-[1-Benzyl-4-(6-methylthio-2-pyridinyl)-4-piperidinyl]-N-phenylacetamide

### Step 1

A solution of [1-benzyl-4-(6-bromo-2-pyridinyl)-4-piperidinyl]-phenyl-amine (0.36 g, 0.85 mmol) obtained in Step 1 of Reference Example 58 in tetrahydrofuran (3 ml), was cooled to -78°C. N-butyllithium (1.6 M, 1.1 ml) was added thereto, the mixture was stirred at -78°C for 20 minutes, and a solution of dimethyl disulfide (0.14 g, 1.4 mmol) in tetrahydrofuran (1 ml) was added. After stirring at -78°C to 0°C for 4 hours, water (10 ml) was added to the reaction mixture, followed by extraction with ethyl acetate. The organic layer was washed successively with water and an aqueous saturated sodium chloride solution, and dried over anhydrous magnesium sulfate, and the solvent was distilled off. The residue was subjected to alumina column chromatography, and fractions eluted with hexane-ethyl acetate (10:1) were collected and concentrated to obtain [1-benzyl-4-(6-methylthio-2-pyridinyl)-4-piperidinyl]phenylamine (0.24 g, 72%).
¹H-NMR (CDCl₃) δ: 2.03 (2H, m), 2.30 (2H, m), 2.50 (2H, m), 2.61 (3H, s), 2.77 (2H, m), 3.54 (2H, s), 6.33 (2H, d, J = 8.6Hz), 6.63 (1H, t, J = 7.3Hz), 7.02 (3H, m), 7.21-7.38 (7H, s).

### Step 2

A solution of [1-benzyl-4-(6-methylthio-2-pyridinyl)-4-piperidinyl]phenylamine (0.25 g, 0.59 mmol) in acetic anhydride (5 ml) was stirred at 100°C for 45 hours, and the solvent was concentrated under reduced pressure. To the residue was added an aqueous saturated sodium bicarbonate solution, the mixture was extracted with ethyl acetate, the organic layer was washed with an aqueous saturated sodium chloride solution, and dried over anhydrous magnesium sulfate, and the solvent was distilled off. The residue was subjected to silica gel column chromatography, and fractions eluted with ethyl acetate-ethanol (10:1) were collected and concentrated to obtain the title compound (0.10 g, 65%) as a yellow oil.
¹H-NMR (CDCl₃) δ: 1.57 (3H, s), 1.92-2.05 (4H, m), 2.54 (3H, s), 2.64 (2H, m), 2.90 (2H, m), 3.33 (2H, s), 7.05 (1H, d, J = 7.7Hz), 7.18-7.26 (5H, m), 7.37-7.51 (7H, m).

### Reference Example 61

### N-[1-Benzyl-4-(6-methylthio-2-pyridinyl)-4-piperidinyl]-2,2,2-trifluoro-N-phenylacetamide

To a solution of [1-benzyl-4-(6-methylthio-2-pyridinyl)-4-piperidinyl]phenylamine (0.10 g, 0.26 mmol) obtained in Step 1 of Reference Example 60 was dissolved in tetrahydrofuran (3 ml) was added trifluoroacetic anhydride (0.58 g, 2.8 mmol), the mixture was stirred at room temperature for 20 hours, and the solvent was concentrated under reduced pressure. To the residue was added an aqueous saturated sodium bicarbonate solution, the mixture was extracted with ethyl acetate, the organic layer was washed with an aqueous saturated sodium chloride solution, and dried over anhydrous magnesium sulfate, and the solvent was distilled off. The residue was subjected to alumina column chromatography, and fractions eluted with hexane-ethyl acetate (5:1) were collected and concentrated to obtain the title compound (0.09 g, 69%) as a yellow oil.
¹H-NMR (CDCl₃) δ: 1.88-2.04 (4H, m), 2.54 (3H, s), 2. 68 (2H, m), 2.85 (2H, m), 3.34 (2H, s), 7.10 (1H, d, J = 7.7Hz), 7.19-7.26 (5H, m), 7.39-7.53 (7H, m).

### Reference Example 62

### N-[1-Benzyl-4-[6-(2-thienyl)-2-pyridinyl]-4-piperidinyl]-N-phenylacetamide

### Step 1

To a solution of [1-benzyl-4-(6-bromo-2-pyridinyl)-4-piperidinyl]-phenyl-amine (0.33 g, 0.78 mmol) obtained in Step 1 of Reference Example 58 in a mixed solvent of toluene (6 ml)-ethanol (1.5 ml)-water (1.5 ml) were added potassium carbonate (0.27 g, 1.95 mmol) and 2-thiopheneboronic acid (0.15 g, 1.17 mmol). After degassing for 15 minutes under reduced pressure, tetrakis(triphenylphosphine)palladium(0) (0.14 g, 0.12 mmol) was added in an argon atmosphere, and the mixture was heated under reflux for 20 hours. To the reaction mixture was added water (10 ml), the mixture was extracted with ethyl acetate, the organic layer was washed successively with water and an aqueous saturated sodium chloride solution, and dried over anhydrous magnesium sulfate, and the solvent was distilled off. The residue was subjected to alumina column chromatography, and fractions eluted with hexane-ethyl acetate (4:1) were collected and concentrated to obtain [1-benzyl-4-[6-(2-thienyl)-3-pyridinyl]-4-piperidinyl]phenylamine (0.12 g, 36%).
¹H-NMR (CDCl₃) δ: 2.08 (2H, m), 2.32 (2H, m), 2.46 (2H, m), 2.77 (2H, m), 3.54 (2H, s), 6.32 (2H, d, J = 8.6Hz), 6.62 (1H, t, J = 7.3Hz), 7.02 (2H, m), 7.13 (1H, m), 7.23-7.35 (7H, s), 7.58 (2H, m), 8.61 (1H, d, J = 4.7Hz).

### Step 2

A solution of [1-benzyl-4-[6-(2-thienyl)-2-pyridinyl]-4-piperidinyl]phenylamine (0.12 g, 0.27 mmol) in acetic anhydride (5 ml) was stirred at 100°C for 45 hours, and the solvent was concentrated under reduced pressure. To the residue was added an aqueous saturated sodium bicarbonate solution, the mixture was extracted with ethyl acetate, the organic layer was washed with an aqueous saturated sodium chloride solution, and dried over anhydrous magnesium sulfate, and the solvent was distilled off. The residue was subjected to silica gel column chromatography, and fractions eluted with ethyl acetate-ethanol (10:1) were collected and concentrated to obtain the title compound (0.05 g, 43%) as a yellow oil.
¹H-NMR (CDCl₃) δ: 1.55 (3H, s), 1.93-2.06 (4H, m), 2.62 (2H, m), 2.87 (2H, m), 3.34 (2H, s), 7.10-7.27 (7H, m), 7.37-7.45 (4H, m), 7.48 (2H, m), 7.66-7.73 (2H, m), 8.61 (1H, m). Reference Example 63A

### N-[4-(4-Methylthiazol-2-yl)-4-piperidinyl]-N-phenylacetamide

To a solution of 1.6 g (3.95 mmol) of N-[1-benzyl-4-(4-methylthiazol-2-yl)-4-piperidinyl]-N-phenylacetamide in 80 ml of methanol were added 2.5 g of 10% palladium carbon and 5.0 g (79.3 mmol) of ammonium formate, and the mixture was heated under reflux for 22 hours. The reaction mixture was filtered, and the filtrate was concentrated, and dried under reduced pressure to obtain 800 mg (yield 64%) of the title compound as a colorless oil.
¹H-NMR (CDCl₃) δ: 2.32 (2H, m), 2.43 (3H, s), 2.83 (2H, m), 3.23-3.38 (4H, m), 6.87 (1H, s), 7.31-7.34 (2H, m), 7.37-7.50 (3H, m), 9.40 (1H, br s).

### Reference Example 63B

### N-[1-(4-Acetylaminobenzyl)-4-(4-methylthiazol-2-yl)-4-piperidinyl]-N-phenylacetamide

To a solution of 250 mg (0.79 mmol) of N-[4-(4-methylthiazol-2-yl)-4-piperidinyl]-N-phenylacetamide and 155 mg (0.95 mmol) of 4-acetamidebenzaldehyde a mixture of 4 ml of chloroform and 1 ml of acetic acid was added 202 mg (0.95 mmol) of sodium triacetoxyborohydride, and the mixture was stirred at room temperature for 16 hours. To the reaction mixture were added ethyl acetate and an aqueous sodium bicarbonate solution, followed by extraction. The organic layer was washed with water, and concentrated under reduced pressure, the residue was subjected to alumina column chromatography, and fractions eluted with hexane-ethyl acetate (1:1) were collected and concentrated to obtain 10 mg (yield 3%) of the title compound as a colorless oil.
¹H-NMR (CDCl₃) δ: 1.63 (3H, s), 2.09 (2H, m), 2.15 (3H, s), 2.30 (2H, m), 2.45 (3H, s), 2.56-2.69 (4H, m), 3.36 (2H, s), 6.83 (1H, s), 7.13 (1H, br s), 7.19 (2H, m), 7.38-7.45 (7H, m) .

### Reference Example 64

### N-[4-(4-Methylthiazol-2-yl)-1-[4-(methylthio)benzyl]-[4-piperidinyl]-N-phenylacetamide

To a solution of 300 mg (0.95 mmol) of N-[4-(4-methylthiazol-2-yl)-4-piperidinyl]-N-phenylacetamide and 175 mg (1.15 mmol) of 4-methylthiobenzaldehyde in a mixture of 4 ml of chloroform and 1 ml of acetic acid was added 1.0 g (4.7 mmol) of sodium triacetoxyborohydride, and the mixture was stirred at room temperature for 40 hours. To the reaction mixture were added ethyl acetate and an aqueous sodium bicarbonate solution, followed by extraction. The organic layer was washed with water, and concentrated under reduced pressure, the residue was subjected to alumina column chromatography, and fractions eluted with hexane-ethyl acetate (5:1) were collected and concentrated to obtain 106 mg (yield 25%) of the title compound as a colorless oil.
¹H-NMR (CDCl₃) δ: 1.63 (3H, s), 2.09 (2H, m), 2.30 (2H, m), 2.46 (3H, s), 2.55-2.70 (4H, m), 3.36 (2H, s), 6.84 (1H, s), 7.13-7.19 (4H, m), 7.34-7.43 (5H, m).

### Reference Example 65

### N-[4-(4-Methylthiazol-2-yl)-1-(4-pyridinylmethyl)-4-piperidinyl]-N-phenylacetamide

To a solution of 250 mg (0.79 mmol) of N-[4-(4-methylthiazol-2-yl)-4-piperidinyl]-N-phenylacetamide and 102 mg (0.95 mmol) of 4-pyridinecarboaldehyde in a mixture of 4 ml of chloroform and 1 ml of acetic acid was added 202 mg (0.95 mmol) of sodium triacetoxyborohydride, and the mixture was stirred at room temperature for 16 hours. To the reaction mixture were added ethyl acetate and an aqueous sodium bicarbonate solution, followed by extraction. The organic layer was washed with water, and concentrated under reduced pressure, the residue was subjected to alumina column chromatography, and fractions eluted with hexane-ethyl acetate (1:1) were collected and concentrated to obtain 10 mg (yield 3%) of the title compound as a colorless oil.
¹H-NMR (CDCl₃) δ: 1.63 (3H, s), 2.09 (2H, m), 2.35 (2H, m), 2.45 (3H, s), 2.55-2.71 (4H, m), 3.41 (2H, s), 6.84 (1H, s), 7.19 (2H, d, J = 5.9Hz), 7.35-7.45 (5H, m), 8.49 (2H, d, J = 5.9Hz).

### Reference Example 66

### N-[4-(4-Methylthiazol-2-yl)-1-(3-pyridinylmethyl)-4-piperidinyl]-N-phenylacetamide

To a solution of 200 mg (0.63 mmol) of N-[4-(4-methylthiazol-2-yl)-4-piperidinyl]-N-phenylacetamide and 85 mg (0.79 mmol) of 3-pyridinecarboaldehyde in a mixture of 4 ml of chloroform and 1 ml of acetic acid was added 674 mg (3.18 mmol) of sodium triacetoxyborohydride, and the mixture was stirred at room temperature for 41 hours. To the reaction mixture were added ethyl acetate and an aqueous sodium bicarbonate solution, followed by extraction. The organic layer was washed with water, and concentrated under reduced pressure, the residue was subjected to alumina column chromatography, and fractions eluted with hexane-ethyl acetate (3:1) were collected and concentrated to obtain 72 mg (yield 28%) of the title compound as a colorless oil.
¹H-NMR (CDCl₃) δ: 1.63 (3H, s), 2.10 (2H, m), 2.35 (2H, m), 2.45 (3H, s), 2.55-2.72 (4H, m), 3.42 (2H, s), 6.84 (1H, s), 7.21 (1H, dd, J = 7.8, 4.8Hz), 7.35-7.45 (5H, m), 7.58 (1H, m), 8.47 (2H, m).

### Reference Example 67

### N-[1-(3,5-Difluorobenzyl)-4-(4-methylthiazol-2-yl)-4-piperidinyl]-N-phenylacetamide

To a solution of 200 mg (0.63 mmol) of N-[4-(4-methylthiazol-2-yl)-4-piperidinyl]-N-phenylacetamide and 110 mg (0.77 mmol) of 3, 5-difluorobenzaldehyde in a mixture of 4 ml of chloroform and 1 ml of acetic acid was added 674 mg (3.18 mmol) of sodium triacetoxyborohydride, and the mixture was stirred at room temperature for 41 hours. To the reaction mixture were added ethyl acetate and an aqueous sodium bicarbonate solution, followed by extraction. The organic layer was washed with water, and concentrated under reduced pressure, the residue was subjected to alumina column chromatography, and fractions eluted with hexane-ethyl acetate (5:1) were collected and concentrated to obtain 100 mg (yield 36%) of the title compound as a colorless oil.
¹H-NMR (CDCl₃) δ: 1.64 (3H, s), 2.08 (2H, m), 2.34 (2H, m), 2.45 (3H, s), 2.54-2.73 (4H, m), 3.37 (2H, s), 6.64 (1H, m), 6.80 (2H, m), 6.84 (1H, s), 7.36-7.46 (5H, m).

### Reference Example 68

### N-[4-(4-Methylthiazol-2-yl)-1-(2-pyridinylmethyl)-4-piperidinyl]-N-phenylacetamide

To a solution of 250 mg (0.79 mmol) of N-[4-(4-methylthiazol-2-yl)-4-piperidinyl]-N-phenylacetamide and 102 mg (0.95 mmol) of 2-pyridinecarboaldehyde in a mixture of 4 ml of chloroform and 1 ml of acetic acid was added 840 mg (3.96 mmol) of sodium triacetoxyborohydride, and the mixture was stirred at room temperature for 48 hours. To the reaction mixture were added ethyl acetate and an aqueous sodium bicarbonate solution, followed by extraction. The organic layer was washed with water, and concentrated under reduced pressure, the residue was subjected to silica gel column chromatography, and fractions eluted with ethyl acetate-ethanol (10:1) were collected and concentrated. The resulting residue was subjected to alumina column chromatography, and fractions eluted with hexane-ethyl acetate (4:1) were collected and concentrated to obtain 48 mg (yield 15%) of the title compound as a colorless oil.
¹H-NMR (CDCl₃) δ: 1.63 (3H, s), 2.15 (2H, m), 2.39-2.48 (2H, m), 2.45 (3H, s), 2.60-2.72 (4H, m), 3.57 (2H, s), 6.84 (1H, s), 7.13 (1H, m), 7.32 (1H, d, J = 7.8Hz), 7.35-7.42 (5H, m), 7.60 (1H, t, J = 7.8Hz), 8.52 (1H, d, J = 4.9Hz).

### Reference Example 69

### N-[1-[4-(1H-Imidazol-1-yl)benzyl]-4-(4-methylthiazol-2-yl)-4-piperidinyl]-N-phenylacetamide

To a solution of 250 mg (0.79 mmol) of N-[4-(4-methylthiazol-2-yl)-4-piperidinyl]-N-phenylacetamide and 171 mg (0.99 mmol) of 4-(1H-imidazol-1-yl)benzaldehyde in a mixture of 4 ml of chloroform and 1 ml of acetic acid was added 840 mg (3.96 mmol) of sodium triacetoxyborohydride, and the mixture was stirred at room temperature for 48 hours. To the reaction mixture were added ethyl acetate and an aqueous sodium bicarbonate solution, followed by extraction. The organic layer was washed with water, and concentrated under reduced pressure, the residue was subjected to silica gel column chromatography, and fractions eluted with ethyl acetate-ethanol (10:1) were collected and concentrated to obtain 36 mg (yield 10%) of the title compound as a colorless oil.
¹H-NMR (CDCl₃) δ: 1.64 (3H, s), 2.09 (2H, m), 2.36 (2H, m), 2.45 (3H, s), 2.60-2.74 (4H, m), 3.44 (2H, s), 6.85 (1H, s), 7.18 (1H, s), 7.24-7.45 (10H, m), 7.82 (1H, s).

### Reference Example 70

### N-[4-(4-Methylthiazol-2-yl)-1-[4-(trifluoromethoxy)benzyl]-4-piperidinyl]-N-phenylacetamide

To a solution of 250 mg (0.79 mmol) of N-[4-(4-methylthiazol-2-yl)-4-piperidinyl]-N-phenylacetamide and 188 mg (0.99 mmol) of 4-(trifluoromethoxy)benzaldehyde in a mixture of 4 ml of chloroform and 1 ml of acetic acid was added 840 mg (3.96 mmol) of sodium triacetoxyborohydride, and the mixture was stirred at room temperature for 48 hours. To the reaction mixture were added ethyl acetate and an aqueous sodium bicarbonate solution, followed by extraction. The organic layer was washed with water, and concentrated under reduced pressure, the residue was subjected to silica gel column chromatography, and fractions eluted with hexane-ethyl acetate (1:2) were collected and concentrated to obtain 35 mg (yield 9.0%) of the title compound as a colorless oil.
¹H-NMR (CDCl₃) δ: 1.63 (3H, s), 2.08 (2H, m), 2.32 (2H, m), 2.45 (3H, s), 2.55-2.72 (4H, m), 3.40 (2H, s), 6.84 (1H, s), 7.11 (2H, m), 7.24-7.28 (2H, m), 7.35-7.44 (5H, m).

### Reference Example 71

### N-[4-(4-Methylthiazol-2-yl)-1-(1-naphthylmethyl)-4-piperidinyl]-N-phenylacetamide

To a solution of 250 mg (0.79 mmol) of N-[4-(4-methylthiazol-2-yl)-4-piperidinyl]-N-phenylacetamide and 155 mg (0.99 mmol) of 1-naphthoaldehyde in a mixture of 4 ml of chloroform and 1 ml of acetic acid was added 840 mg (3.96 mmol) of sodium triacetoxyborohydride, and the mixture was stirred at room temperature for 48 hours. To the reaction mixture were added ethyl acetate and an aqueous sodium bicarbonate solution, followed by extraction. The organic layer was washed with water, and concentrated under reduced pressure, the residue was subjected to silica gel column chromatography, and fractions eluted with hexane-ethyl acetate (1:1) were collected and concentrated. Subsequently, the residue was subjected to alumina column chromatography, and fractions eluted with hexane-ethyl acetate (4:1) were collected and concentrated to obtain 17 mg (yield 5.0%) of the title compound as a colorless oil.
¹H-NMR (CDCl₃) δ: 1.62 (3H, s), 2.07 (2H, m), 2.41 (2H, m), 2.48 (3H, s), 2.67-2.70 (4H, m), 3.80 (2H, s), 6.85 (1H, s), 7.33-7.50 (9H, m), 7.73 (1H, m), 7.81 (1H, m), 8.19 (1H, m).

### Reference Example 72

### N-[1-Benzoyl-4-(4-methylthiazol-2-yl)-4-piperidinyl]-N-phenylacetamide

To a solution of 350 mg (1.11 mmol) of N-[4-(4-methylthiazol-2-yl)-4-piperidinyl]-N-phenylacetamide in a mixture of 4 ml of chloroform and 1.5 ml of pyridine was added 172 mg (1.22 mmol) of benzoyl chloride, and the mixture was stirred at room temperature for 14 hours. To the reaction mixture were added ethyl acetate and an aqueous sodium bicarbonate solution, followed by extraction. The organic layer was washed with water, and concentrated under reduced pressure, the residue was subjected to silica gel column chromatography, and fractions eluted with hexane-ethyl acetate (1:1) were collected and concentrated to obtain 229 mg (yield 49%) of the title compound as a colorless oil.
¹H-NMR (CDCl₃) δ: 1.67 (3H, s), 1.96 (2H, m), 2.45 (3H, s), 2.59 (1H, m), 2.78 (1H, m), 3.26 (1H, m), 3.55 (2H, m), 4.30 (1H, m), 6.86 (1H, s), 7.33-7.48 (10H, m).

### Reference Example 73

### N-[4-(4-Methylthiazol-2-yl)-1-(2-oxo-2-phenylethyl)-4-piperidinyl]-N-phenylacetamide oxalate

To a solution of 200 mg (0.63 mmol) of N-[4-(4-methylthiazol-2-yl)-4-piperidinyl]-N-phenylacetamide and 140 mg (0.70 mmol) of phenacyl bromide in 2 ml of acetone was added 131 mg (0.95 mmol) of potassium carbonate, and the mixture was stirred at 50°C for 5 hours. To the reaction mixture were added ethyl acetate and an aqueous sodium bicarbonate solution, followed by extraction. The organic layer was washed with water, and concentrated under reduced pressure, the residue was subjected to silica gel column chromatography, and fractions eluted with hexane-ethyl acetate (1:2) were collected and concentrated. Subsequently, the residue was subjected to alumina column chromatography, and fractions eluted with hexane-ethyl acetate (2:1) were collected and concentrated to obtain 80 mg (yield 29%) of N-[4-(4-methylthiazol-2-yl)-1-(2-oxo-2-phenylethyl)-4-piperidinyl]-N-phenylacetamide as a colorless oil.
¹H-NMR (CDCl₃) δ: 1.64 (3H, s), 2.19 (2H, m), 2.46 (2H, m), 2.48 (3H, s), 2.70-2.85 (4H, m), 3.75 (2H, s), 6.84 (1H, s), 7.36-7.56 (8H, m), 7.92 (2H, d, J = 7.1Hz).

The resulting N-[4-(4-methylthiazol-2-yl)-1-(2-oxo-2-phenylethyl)-4-piperidinyl]-N-phenylacetamide was dissolved in ethyl acetate (3 ml), a solution of oxalic acid (0.02 g, 0.17 mmol) in isopropanol (1 ml)was added, and the mixture was stirred for 30 minutes. Precipitated crystals were recrystallized from ethyl acetate-isopropyl ether to obtain the title compound (0.07 g, 70%).

### Reference Example 74

### N-[4-(4-Methylthiazol-2-yl)-1-phenylsulfonyl-4-piperidinyl]-N-phenylacetamide

To a solution of 200 mg (0.63 mmol) of N-[4-(4-methylthiazol-2-yl)-4-piperidinyl]-N-phenylacetamide and 100 mg (0.99 mmol) of triethylamine in 2 ml of tetrahydrofuran was added 123 mg (0.70 mmol) of benzenesulfonyl chloride, and the mixture was stirred at 50°C for 6 hours. To the reaction mixture were added ethyl acetate and an aqueous sodium bicarbonate solution, followed by extraction. The organic layer was washed with water, and concentrated under reduced pressure, the residue was subjected to silica gel column chromatography, and fractions eluted with hexane-ethyl acetate (1.1) were collected and concentrated to obtain 75 mg (yield 26%) of the title compound as a colorless oil.
¹H-NMR (CDCl₃) δ: 1.63 (3H, s), 2.07 (2H, m), 2.24 (3H, s), 2.60 (2H, m), 2.94 (2H, m), 3.37 (2H, m), 6.73 (1H, s), 7.24 (2H, m), 7.40 (3H, m), 7.52-7.62 (3H, m), 7.70 (2H, m).

### Reference Example 75

### N-[1-Cyclopropylmethyl-4-(4-methylthiazol-2-yl)-4-piperidinyl]-N-phenylacetamide oxalate

To a suspension of 200 mg (0.63 mmol) of N-[4-(4-methylthiazol-2-yl)-4-piperidinyl]-N-phenylacetamide and 132 mg (0.96 mmol) of potassium carbonate in 2 ml of N, N-dimethylformamide was added 103 mg (0.75 mmol) of cyclopropylmethyl bromide, and the mixture was stirred at 50°C for 8 hours. To the reaction mixture were added ethyl acetate and an aqueous sodium bicarbonate solution, followed by extraction. The organic layer was washed with water, and concentrated under reduced pressure, the residue was subjected to silica gel column chromatography, and fractions eluted with ethyl acetate/ethanol (5:1) were collected and concentrated. Subsequently, the residue was subjected to alumina column chromatography, and fractions eluted with hexane-ethyl acetate (3:1) were collected and concentrated to obtain 70 mg (yield 30%) of N-[1-cyclopropylmethyl-4-(4-methylthiazol-2-yl)-4-piperidinyl]-N-phenylacetamide as a colorless oil.
¹H-NMR (CDCl₃) δ: 0.33 (2H, m), 0.45 (2H, m), 0.80 (1H, m), 1.64 (3H, s), 2. 10 (2H, m), 2.15 (2H, d, J = 6.5Hz), 2.29 (2H, m), 2.45 (3H , s), 2.72-2.82 (4H, m), 6.84 (1H, s), 7.36-7.42 (5H, m).

The resulting N-[1-cyclopropylmethyl-4-(4-methylthiazol-2-yl)-4-piperidinyl]-N-phenylacetamide was dissolved in ethyl acetate (3 ml), a solution of oxalic acid (0.02 g, 0.17 mmol) in isopropanol (1 ml) was added, and the mixture was stirred for 30 minutes. Precipitated crystals were recrystallized from ethyl acetate-isopropyl ether to obtain the title compound (0.05 g, 58%).

### Reference Example 76

### Ethyl α-[4-(Acetylphenylamino)-4-(4-methylthiazol-2-yl)-1-piperidinyl]phenylacetate

To a suspension of 800 mg (2.54 mmol) of N-[4-(4-methylthiazol-2-yl)-4-piperidinyl]-N-phenylacetamide and 525 mg (3.80 mmol) of potassium carbonate in 5 ml of N,N-dimethylformamide was added 740 mg (3.04 mmol) of ethyl α-bromophenylacetate, and the mixture was stirred at 60°C for 17 hours. To the reaction mixture were added ethyl acetate and an aqueous sodium bicarbonate solution, followed by extraction. The organic layer was washed with water, and concentrated under reduced pressure, the residue was subjected to silica gel column chromatography, and fractions eluted with ethyl acetate were collected and concentrated. Subsequently, the residue was subjected to alumina column chromatography, and fractions eluted with hexane-ethyl acetate (3:1) were collected and concentrated to obtain 306 mg (yield 23%) of the title compound as a colorless oil.
¹H-NMR (CDCl₃) δ: 1.15 (3H, t, J = 7.1Hz), 1.62 (3H, s), 2.07 (1H, m), 2.18 (2H, m), 2.45 (3H, s), 2.48 (2H, m), 2.60 (1H, m), 2.75 (2H, m), 3.85 (2H, s), 4.09 (2H, q. J = 7.1Hz), 6.83 (1H, s), 7.25-7.40 (10H, m).

### Reference Example 77

### N-[1-(4-Fluorobenzyl)-4-(4-methylthiazol-2-yl)-4-piperidinyl]-N-phenylacetamide oxalate

To a suspension of 400 mg (1.27 mmol) of N-[4-(4-methylthiazol-2-yl)-4-piperidinyl]-N-phenylacetamide and 440 mg (3.18 mmol) of potassium carbonate in 2 ml of N,N-dimethylformamide was added 360 mg (1.95 mmol) of 4-fluorobenzyl bromide, and the mixture was stirred at 60°C for 4 days. To the reaction mixture were added ethyl acetate and an aqueous sodium bicarbonate solution, followed by extraction. The organic layer was washed with water, and concentrated under reduced pressure, the residue was subjected to silica gel column chromatography, and fractions eluted with ethyl acetate were collected and concentrated. Subsequently, the residue was subjected to alumina column chromatography, and fractions eluted with hexane-ethyl acetate (2:1) were collected and concentrated. The resulting N-[1-(4-fluorobenzyl)-4-(4-methylthiazol-2-yl)-4-piperidinyl]-N-phenylacetamide was dissolved in ethyl acetate (3 ml), a solution of oxalic acid (0.03 g, 0.38 mmol) in isopropanol (1 ml)was added, and the mixture was stirred for 30 minutes. Precipitated crystals were recrystallized from ethyl acetate-isopropyl ether to obtain the title compound (0.16 g, 29%).
¹H-NMR (DMSO-d₆) δ: 1.55 (3H, s), 1.99 (2H, m), 2.33 (3H, s), 2.53 (2H, m), 2.80-2.94 (4H, m), 3.94 (2H, m), 7.22 (2H, m), 7.37-7.53 (8H, m).

### Reference Example 78

### N-[1-(1-Methyl-1-phenylethyl)-4-(4-methylthiazol-2-yl)-4-piperidinyl]-N-phenylacetamide

### Step 1

To a solution of 4.67 g (34.5 mmol) of cumylamine in 27 ml of ethanol was added 480 mg (3.50 mmol) of potassium carbonate. A solution of 6.2 g (23.0 mmol) of 1-ethyl-1-methyl-4-oxopiperidinium iodide in water (12 ml) was added dropwise thereto with heating under refluxing, and the mixture was heated under reflux for 2 hours. To the reaction mixture was added water, followed by extraction with ethyl acetate. The organic layer was washed with water, and concentrated under reduced pressure, the residue was subjected to silica gel column chromatography, and fractions eluted with ethyl acetate were collected and concentrated. Subsequently, the residue was subjected to alumina column chromatography, and fractions eluted with hexane-ethyl acetate (2:1) were collected and concentrated to obtain 1-(1-methyl-1-phenylethyl)-4-piperidone (3.46 g, 69%).
¹H-NMR (CDCl₃) δ: 1.40 (6H, s), 2.40 (4H, t, J = 6.0Hz), 2.77 (4H, t, J = 6.0Hz), 7.23 (1H, m), 7.33 (2H, t, J = 7.1Hz), 7.58 (2H, d, J = 7.1Hz).

### Step 2

Trimethylsilylnitrile (1.08 ml, 8.1 mmol) was added dropwise to an acetic acid solution (10 mL) of 1-(1-methyl-1-phenylethyl)-4-piperidone (1.70 g, 7.8 mmol) and aniline (0.80 g, 8.6 mmol) under ice-cooling, and the mixture was stirred. The reaction mixture was poured into a cold aqueous ammonia solution (a mixture of 50 ml of an aqueous concentrated ammonium hydroxide solution and 50 g of crushed ice), and an aqueous concentrated hydroxide solution was slowly added until pH of the mixture became 10. Precipitated crystals were filtered, washed successively with water and isopropyl ether, and dried under reduced pressure to obtain 1-(1-methyl-1-phenylethyl)-4-(phenylamino)piperidine-4-carbonitrile (1.89 g, 76%).
¹H-NMR (CDCl₃) δ: 1.37 (6H, s), 1.87 (2H, m), 2.30 (2H, m), 2.54 (2H, m), 2.79 (2H, m), 3.62 (1H, br s), 6.91 (3H, m), 7.20-7.33 (5H, m), 7.51 (2H, d, J = 8.6Hz).

### Step 3

To a tetrahydrofuran solution (5 ml) of 4-methylthiazole (1.17 g, 11.8 mmol) was added dropwise n-butyllithium (1.6 M hexane solution, 7.4 ml, 11.8 mmol) at -78°C, and the mixture was stirred for 20 minutes. At the same temperature, a tetrahydrofuran solution (10 ml) of 1-(1-methyl-1-phenylethyl)-4-(phenylamino)piperidine-4-carbonitrile (1.88 g, 8.9 mmol) was added at once, and a temperature was slowly raised to 0°C over 30 minutes. To the reaction mixture was added water (20 ml), the mixture was extracted with ethyl acetate (3×40 ml), the organic layer was washed with water (40 ml), and dried with anhydrous magnesium sulfate, and the solvent was distilled off. The residue was subjected to silica gel column chromatography, and fractions eluted with hexane-ethyl acetate (3:1) were collected and concentrated to obtain [1-(1-methyl-1-phenylethyl)-4-(4-methylthiazol-2-yl)-4-piperidinyl]-phenyl-amine (2.05 g, 89%).
¹H-NMR (CDCl₃) δ: 1.33 (6H, s), 2.17 (2H, m), 2.31-2.44 (4H, m), 2.46 (3H, s), 2.70 (2H, m), 4.24 (1H, br s), 6.45 (2H, d, J = 7.6Hz), 6.69 (1H, t, J = 7.3Hz), 6.78 (1H, s), 7.06 (2H, m), 7.18 (1H, m), 7.28 (2H, m), 7.54 (2H, d, J = 7.2Hz).

### Step 4

[1-(1-Methyl-1-phenylethyl)-4-(4-methylthiazol-2-yl)-4-piperidinyl]-phenyl-amine (0.67 g, 1.71 mmol) was dissolved in chloroform (4 ml), acetyl chloride (2.5 ml, 35.2 mmol) was added thereto, the mixture was stirred at 40°C for 41 hours, and the solvent was concentrated under reduced pressure. To the residue was added an aqueous saturated sodium bicarbonate solution, this was extracted with ethyl acetate, the organic layer was washed with an aqueous saturated sodium chloride solution, and dried with anhydrous magnesium sulfate, and the solvent was distilled off. The residue was subjected to silica gel column chromatography, and fractions eluted with hexane-ethyl acetate (1:1) were collected and concentrated.
Subsequently, the residue was subjected to alumina column chromatography, and fractions eluted with hexane-ethyl acetate (5:1) were collected and concentrated to obtain the title compound (0.05 g, 43%) as a yellow oil.
¹H-NMR (CDCl₃) δ: 1.25 (6H, s), 1.62 (3H, s), 2.09 (2H, m), 2.39-2.52 (4H, m), 2.45 (3H, s), 2.59 (2H, m), 6.81 (1H, s), 7.16 (1H, m), 7.25 (2H, m), 7.37-7.48 (7H, m).

### Reference Example 79

### 2,2,2-Trifluoro-N-[1-(1-methyl-1-phenylethyl)-4-(4-methylthiazol-2-yl)-4-piperidinyl]-N-phenylacetamide

[1-(1-Methyl-1-phenylethyl-4-(4-methylthiazol-2-yl)-4-piperidinyl)-phenyl-amine (0.67 g, 1.71 mmol) obtained in Step 3 of Reference Example 78 was dissolved in tetrahydrofuran (10 ml), trifluoroacetic anhydride (3.60 g, 17.1 mmol) was added thereto, the mixture was stirred at room temperature for 24 hours, and the solvent was concentrated under reduced pressure. To the residue was added an aqueous saturated sodium bicarbonate solution, the mixture was extracted with ethyl acetate, the organic layer was washed with an aqueous saturated sodium chloride solution, and dried with anhydrous magnesium sulfate, and the solvent was distilled off. The residue was subjected to silica gel column chromatography, and fractions eluted with hexane-ethyl acetate (1:1) were collected and concentrated. Subsequently, the residue was subjected to alumina column chromatography, and fractions eluted with hexane-ethyl acetate (5:1) were collected and concentrated to obtain the title compound as a yellow oil.
¹H-NMR (CDCl₃) δ: 1.25 (6H, s), 2.04 (2H, m), 2.41 (2H, m), 2.45 (3H, s), 2.53-2.70 (4H, m), 6.86 (1H, s), 7.17 (1H, m), 7.25 (2H, m), 7.39-7.49 (7H, m).

### Reference Example 80A

### N-[1-Benzyl-4-(4-methylthiazol-2-yl)-4-piperidinyl]-N-(2-methylphenyl)acetamide

### Step 1

To an acetic acid solution (15 ml) of 1-benzyl-4-piperidone (3.00 g, 15.9 mmol) and 2-methylaniline (1.90 g, 17.5 mmol) was added dropwise trimethylsilylnitrile (2.10 ml, 15.9 mmol) under ice-cooling over 10 minutes with attention so that a temperature of the reaction system did not exceed 40°C, and the mixture was stirred for 1 hour. The reaction mixture was poured into a cold aqueous ammonia solution (a mixture of 25 ml of an aqueous concentrated ammonium hydroxide solution and 25 g of crushed ice), and an aqueous concentrated ammonium hydroxide solution was slowly added until pH of the mixture became 10. The mixture was extracted with chloroform (4×25 ml), the organic layer was dried with anhydrous sodium sulfate, and the solvent was distilled off. To the resulting oily substance were added diethyl ether and n-hexane, precipitated white crystals were washed with diethyl ether, and dried to obtain 1-benzyl-4-(2-methylphenylamino)piperidine-4-carbonitrile (4.31 g, 89%).
¹H-NMR (DMSO-d₆) δ: 1.94 (2H, m), 2.14 (3H, s), 2.25-2.40 (4H, m), 2.65-2.75(2H, m), 3.50 (2H, s), 4.86 (1H, s), 6.71 (1H, t, J = 7.3Hz), 6.96 (1H, d, J = 8.3Hz), 7.00-7.10 (2H, m), 7.20-7.34 (5H, m).

### Step 2

To a tetrahydrofuran solution (10 ml) of 4-methylthiazole (0.793 g, 8.00 mmol) was added dropwise n-butyllithium (1.6 M hexane solution, 5.00 ml, 8.00 mmol) at -78°C, and the mixture was stirred for 20 minutes. At the same temperature, a tetrahydrofuran solution (5 ml) of 1-benzyl-4-(2-methylphenylamino)piperidine-4-carbonitrile (1.22 g, 4.00 mmol) was added dropwise, and a temperature was slowly raised to 0°C over 30 minutes. Water (3 ml) was added to the reaction mixture, this was extracted with ethyl acetate, the organic layer was washed with water, and dried over anhydrous magnesium sulfate, and the solvent was distilled off. The residue was purified by silica gel column chromatography (hexane-ethyl acetate) to obtain [1-benzyl-4-(4-methylthiazol-2-yl)-4-piperidinyl](2-methylphenyl)amine (1.43 g, 95%).
¹H-NMR(CDCl₃) δ: 2.10-2.30 (4H, m), 2.25 (3H, s), 2.40-2.55 (2H, m), 2.46 (3H, s), 3.53 (2H, s), 4.14 (1H, s), 6.20 (1H, d, J = 8.0Hz), 6.63 (1H, t, J = 7.3Hz), 6.79 (1H, s), 6.87 (1H, t, J = 7.6Hz), 7.20-7.40 (6H, m).

### Step 3

Acetic anhydride (15 ml) was added to [1-benzyl-4-(4-methylthiazol-2-yl)-4-piperidinyl](2-methylphenyl)amine 0.380 g, 1.00 mmol) at room temperature, and the mixture was heated at 100°C for 48 hours. The reaction solvent was distilled off, an aqueous saturated sodium bicarbonate solution (30 ml) was added, this was extracted with chloroform (4×20 ml), the organic layer was dried with anhydrous magnesium sulfate, and the solvent was distilled off. The residue was purified by silica gel column chromatography (hexane-ethyl acetate) to obtain the title compound (0.029 g, 7%).
¹H-NMR(CDCl₃) δ: 1.56 (3H, s), 1.97 (1H, t, J = 11.9Hz), 2.20-2.50 (3H, m), 2.38 (3H, s), 2.45 (3H, s), 2.61 (1H, d, J = 12.1Hz), 2.82 (1H, d, J = 10.3Hz), 3.29-3.42 (3H, m), 6.88 (1H, s), 7.15-7.35 (8H, m), 7.42 (1H, d, J = 7.0Hz).

### Reference Example 80B

### N-[1-Benzyl-4-(4-methylthiazol-2-yl)-4-piperidinyl]-4-(2-methylphenyl)acetamide oxalate

To a 2-propanol solution (0.7 ml) of N-[1-benzyl-4-(4-methylthiazol-2-yl)-4-piperidinyl]-N-(2-methylphenyl)acetamide (0.029 g, 0.069 mmol) obtained in Reference Example 80A was added a 2-propanol solution (0.7 ml) of oxalic acid (0.009 g, 0.10 mmol), precipitated crystals were washed with an ethyl acetate/n-hexane mixed solvent, and dried to obtain the title compound (0.031 g, 88%).
IR (KBr) ν : 1663 cm⁻¹.

### Reference Example 81A

### N-[1-Benzyl-4-(4-methylthiazol-2-yl)-4-piperidinyl]-N-(3-methylphenyl)acetamide

According to the same manner as that of Reference Example 80A, 3-methylaniline was used in place of 2-methylaniline to obtain the title compound (0.068 g, 16%.
¹H-NMR(CDCl₃) δ: 1.63 (3H, s), 2.20-2.15 (2H, m), 2.26-2.40 (2H, m), 2.38 (3H, s), 2.45 (3H, s), 2.60-2.80 (4H, m), 3.42 (2H, s), 6.83 (1H, s), 7.15-7.32 (9H, m).

### Reference Example 81B

### N-[1-Benzyl-4-(4-methylthiazol-2-yl)-4-piperidinyl]-N-(3-methylphenyl)acetamide oxalate

According to the same manner as that of Reference Example 80B, from N-[1-benzyl-4-(4-methylthiazol-2-yl)-4-piperidinyl]-N-(3-methylphenyl)acetamide, the title compound (0.064 g, 77%) was obtained.
IR (KBr) ν : 1667 cm⁻¹.

### Reference Example 82A

### N-[1-Benzyl-4-(4-methylthiazol-2-yl)-4-piperidinyl]-N-(3-chlorophenyl)acetamide

According to the same manner as that of Reference Example 80A, 3-chloroaniline was used in place of 2-methylaniline to obtain the title compound (0.084 g, 19%).
¹H-NMR (CDCl₃) δ: 2.00-2.17 (2H, m), 2.05 (3H, m), 2.22-2.40 (2H, m), 2.45 (3H, s), 2.56-2.78 (4H, m), 3.42 (2H, s), 6.85 (1H, s), 7.20-7.50 (9H, m).

### Reference Example 82B

### N-[1-Benzyl-4-(4-methylthiazol-2-yl)-4-piperidinyl]-N-(3-chlorophenyl)acetamide oxalate

According to the same manner as that of Reference Example 80B, from N-[1-benzyl-4-(4-methylthiazol-2-yl)-4-piperidinyl]-N-(3-chlorophenyl)acetamide, the title compound (0.07 g, 72%) was obtained.
IR (KBr) ν : 1669 cm⁻¹.

### Reference Example 83A

### N-[1-Benzyl-4-(4-methylthiazol-2-yl)-4-piperidinyl]-N-(3-fluorophenyl)acetamide

According to the same manner as that of Reference Example 80A, 3-fluoroaniline was used in place of 2-methylaniline to obtain the title compound (0.080 g, 9%).
¹H-NMR (CDCl₃) δ: 1.65 (3H, s), 2.00-2.40 (4H, m), 2.05 (3H, s), 2.45 (3H, s), 2.50-2.80 (4H, m), 3.41 (2H, s), 6.84 (1H, d, J = 0.8Hz), 7.05-7.44 (9H, m).

### Reference Example 83B

### N-[1-Benzyl-4-(4-methylthiazol-2-yl)-4-piperidinyl]-N-(3-fluorophenyl)acetamide oxalate

According to the same manner as that of Reference Example 80B, from N-[1-benzyl-4-(4-methylthiazol-2-yl)-4-piperidinyl]-N-(3-fluorophenyl)acetamide, the title compound (0.072 g, 72%) was obtained.
IR (KBr) ν : 1680 cm⁻¹.

### Reference Example 84A

### N-[1-Benzyl-4-(4-methylthiazol-2-yl)-4-piperidinyl]-2,2,2-trifluoro-N-phenylacetamide

To a dichloromethane solution (15 ml) of 1-benzyl-4-(4-methylthiazol-2-yl)-4-(phenylamino)piperidine (0.600 g, 1.72 mmol) obtained in Reference Example 38, 4-dimethylaminopyridine (0.009 g, 0.074 mmol) and triethylamine (0.35 ml, 2.51 mmol) was added dropwise trifluoroacetic acid (0.960 ml, 12.0 mmol) at 0°C, the mixture was stirred at the same temperature for 15 minutes, and a temperature was gradually raised to room temperature, followed by stirring for 2 hours. The reaction solvent was distilled off, an aqueous saturated sodium bicarbonate solution (30 ml) was added, this was extracted with chloroform (3×30 ml), the organic layer was washed with an aqueous saturated sodium bicarbonate solution and water, and dried with anhydrous magnesium sulfate, and the solvent was distilled off. The residue was purified by silica gel column chromatography (hexane-ethyl acetate) to obtain the title compound (0.508 g, 77%).
¹H-NMR(CDCl₃) δ: 1.99-2.17 (2H, m), 2.23-2.38 (2H, m), 2.46 (3H, s), 2.63-2.76 (4H, m), 3.40 (2H, s), 6.89 (1H, d, J = 0.7Hz), 7.17-7.34 (5H, m), 7.35-7.53 (5H, m).

### Reference Example 84B

### N-[1-Benzyl-4-(4-methylthiazol-2-yl)-4-piperidinyl]-2,2,2-trifluoro-N-phenylacetamide oxalate

To a 2-propanol solution (2 ml) of N-[1-benzyl-4-(4-methylthiazol-2-yl)-4-piperidinyl]-2,2,2-trifluoro-N-phenylaceatamide (0.508 g, 1.11 mmol) obtained in Reference Example 84A was added oxalic acid (0.120 g, 1.33 mmol) to completely dissolve the material, a small amount of n-hexane was added to precipitate crystals, which was washed with an ethyl acetate/n-hexane mixed solvent, and dried to obtain the title compound (0.453 g, 74%).
IR (KBr) ν : 1703 cm⁻¹.

### Reference Example 85A

### N-[1-(2,6-Dimethylbenzyl)-4-(4-methylthiazol-2-yl)-4-piperidinyl]-N-phenylacetamide

N-[4-(4-Methylthiazol-2-yl)-4-piperidinyl]-N-phenylacetamide (0.300 g, 0.951 mmol) obtained in Reference Example 63A, 2,6-dimethylbenzaldehyde (0.128 g, 0.954 mmol) and titanium (IV) isopropoxide (0.341 g, 1.20 mmol) were mixed, the mixture was stirred at room temperature for 2 hours, a THF solution (5 ml) of poly(methylhydrosiloxane) (0.114 g, 1.90 mmol) was added, and the mixture was stirred for 6 hours. The reaction mixture was ice-cooled, a 3N aqueous sodium hydroxide solution (10 ml) was added dropwise, and the mixture was stirred at the same temperature for 20 minutes, and extracted with ethyl acetate. The organic layer was washed with an aqueous saturated sodium chloride solution, and dried with anhydrous sodium sulfate, and the solvent was distilled off. The residue was purified by silica gel column chromatography (hexane-ethyl acetate) to obtain the title compound (0.049 g, 12%) .
¹H-NMR (CDCl₃) δ: 1.62 (3H, s), 1.90-2.05 (2H, m), 2.30-2.40 (2H, m), 2.31 (6H, m), 2.47 (3H, s), 2.50-2.70 (4H, m), 3.36 (2H, s), 6.85 (1H, s), 6.90-7.40 (8H, m).

### Reference Example 85B

### N-[1-(2,6-Dimethylbenzyl)-4-(4-methylthiazol-2-yl)-4-piperidinyl]-N-phenylacetamide oxalate

To a 2-propanol solution (2 ml) of N-[1-(2,6-dimethylbenzyl)-4-(4-methylthiazol-2-yl)-piperidinyl]-N-phenylacetamide (0.049 g, 0.113 mmol) obtained in Reference Example 85A was added oxalic acid (0.012 g, 0.132 mmol) to completely dissolve the material, a small amount of n-hexane was added to precipitate crystals, which was washed with an ethyl acetate/n-hexane mixed solvent, and dried to obtain the title compound (0.039 g, 66%).
IR (KBr) ν : 1665 cm⁻¹.

### Reference Example 86A

### N-[1-(2,6-Dichlorobenzyl)-4-(4-methylthiazol-2-yl)-4-piperidinyl]-N-phenylacetamide

According to the same manner as that of Reference Example 85A, 2,6-dichlorobenzaldehyde was used in place of 2,6-dimethylbenzaldehyde to obtain the title compound (0.100 g, 22%).
¹H-NMR (CDCl₃) δ: 1.62 (3H, s), 1.95-2.10 (2H, m), 2.47 (3H, s), 2.51 (2H, t, J = 11.6Hz), 2.67 (4H, d, J = 8.7Hz), 3.64 (2H, s), 6.85 (1H, s), 6.05-7.45 (8H, m).

### Reference Example 86B

### N-[1-(2,6-Dichlorobenzyl)-4-(4-methylthiazol-2-yl)-4-piperidinyl]-N-phenylacetamide oxalate

According to the same manner as that of Reference Example 85B, from N-[1-(2,6-dichlorobenzyl)-4-(4-methylthiazol-2-yl)-4-piperidinyl]-N-phenylacetamide, the title compound (0.054 g, 45%) was obtained.
IR (KBr) ν : 1663 cm⁻¹.

### Reference Example 87A

### N-[1-(2,6-Difluorobenzyl)-4-(4-methylthiazol-2-yl)-4-piperidinyl]-N-phenylacetamide

A chloroform solution (10 ml) of N-[4-(4-methylthiazol-2-yl)-4-piperidinyl]-N-phenylacetamide (0.300 g, 1.20 mmol), 2,6-difluorobenzaldehyde (0.171 g, 1.20 mmol), acetic acid (2.5 ml), and sodium triacetoxyborohydride (0.254 g, 1.20 mmol) was stirred at room temperature for 16 hours. To the reaction mixture was added an aqueous saturated sodium bicarbonate solution (30 ml), this was extracted with chloroform (3×30 ml), the organic layer was washed with water, and dried with anhydrous magnesium sulfate, and the solvent was distilled off. The residue was purified by silica gel column chromatography (hexane-ethyl acetate) to obtain the title compound (0.191 g, 46%).
¹H-NMR (CDCl₃) δ: 1.61 (3H, s), 2.00-2.15 (2H, m), 2.41 (3H, s), 2.40-2.50 (2H, m), 2.62 (4H, m), 3.59 (2H, s), 6.80-6.90 (3H, m), 7.15-7.30 (1H, m), 7.36 (5H, s).

### Reference Example 87B

### N-[1-(2,6-Difluorobenzyl)-4-(4-methylthiazol-2-yl)-4-piperidinyl]-N-phenylacetamide oxalate

According to the same manner as that of Reference Example 85B, from N-[1-(2,6-difluorobenzyl)-4-(4-methylthiazol-2-yl)-4-piperidinyl]-N-phenylacetamide, the title compound (0.123 g, 53%) was obtained.
IR (KBr) ν : 1667 cm⁻¹.

### Reference Example 88

### N-[1-(2-Cyanobenzyl)-4-(4-methylthiazol-2-yl)-4-piperidinyl]-N-phenylacetamide

According to the same manner as that of Reference Example 87A, 2-formylbenzonitrile was used in place of 2,6-difluorobenzaldehyde to obtain the title compound (0.010 g, 2%).
¹H-NMR (CDCl₃) δ: 1.63 (3H, s), 2.00-2.20 (2H, m), 2.35-2.50 (2H, m), 2.46 (3H, s), 2.55-2.75 (4H, m), 3.60 (2H, s), 6.85 (1H, s), 7.25-7.65 (9H, m).

### Reference Example 89

### N-[1-(4-Cyanobenzyl)-4-(4-methylthiazol-2-yl)-4-piperidinyl]-N-phenylacetamide

According to the same manner as that of Reference Example 87A, 4-formylbenzonitrile was used in place of 2,6-difluorobenzaldehyde to obtain the title compound (0.024 g, 6%).
¹H-NMR (CDCl₃) δ: 1.64 (3H, s), 2.00-2.15 (2H, m), 2.34 (2H, t, J = 9.6Hz), 2.45 (3H, s), 2.50-2.65 (2H, m), 2.70 (2H, d, J = 12.7Hz), 3.44 (2H, s), 6.85 (1H, s), 7.35-7.60 (9H, m).

### Reference Example 90A

### N-[4-(4-Methylthiazol-2-yl)-1-(2-trifluoromethylbenzyl)-4-piperidinyl]-N-phenylacetamide

According to the same manner as that of Reference Example 87A, 2-trifluoromethylbenzaldehyde was used in place of 2,6-difluorobenzaldehyde to obtain the title compound (0.085 g, 19%).
¹H-NMR (CDCl₃) δ: 1.65 (3H, s), 2.10-2.20 (2H, m), 2.30-2.45 (2H, m), 2.46 (3H, s), 2.50-2.65 (2H, m), 2.70 (2H, d, J = 14.7Hz), 3.56 (3H, s), 6.85 (1H, s), 7.25-7.50 (7H, m), 7.57 (1H, d, J = 7.6Hz), 7.72 (1H, d, J = 9.2Hz).

### Reference Example 90B

### N-[4-(4-Methylthiazol-2-yl)-1-(2-trifluoromethylbenzyl)-4-piperidinyl]-N-phenylacetamide oxalate

According to the same manner as that of Reference Example 85B, from N-[4-(4-methylthiazol-2-yl)-1-(2-trifluoromethylbenzyl)-4-piperidinyl]-N-phenylacetamide, the title compound (0.068 g, 67%) was obtained.
IR (KBr) ν : 1667 cm⁻¹.

### Reference Example 91A

### N-[4-(4-Methylthiazol-2-yl)-1-(3-trifluoromethylbenzyl)-4-piperidinyl]-N-phenylacetamide

According to the same manner as that of Reference Example 87A, 3-trifluoromethylbenzaldehyde was used in place of 2,6-difluorobenzaldehyde to obtain the title compound (0.084 g, 19%).
¹H-NMR (CDCl₃) δ: 1.64 (3H, s), 2.00-2.15 (2H, m), 2.25-2.40 (2H, m), 2.45 (3H, s), 2.55-2.65 (2H, m), 2.70 (2H, d, J = 13.7Hz), 3.45 (2H, s), 6.84 (1H, s), 7.35-7.65 (9H, m).

### Reference Example 91B

### N-[4-(4-Methylthiazol-2-yl)-1-(3-trifluoromethylbenzyl)-4-piperidinyl]-N-phenylacetamide oxalate

According to the same manner as that of Reference Example 85B, from N-[4-(4-methylthiazol-2-yl)-1-(3-trifluoromethylbenzyl)-4-piperidinyl]-N-phenylacetamide, the title compound (0.081 g, 81 %) was obtained.
IR (KBr) ν : 1667 cm⁻¹.

### Reference Example 92

### N-[1-Benzyl-2-methyl-4-(4-methylthiazol-2-yl)-4-piperidinyl]-N-phenylacetamide (isomer A' and isomer B') Step 1

To an ethyl acetate (15 ml) solution of 7-methyl-1,4-dioxa-8-azaspiro[4.5]decane-8-carboxylic acid tert-butyl ester (3.31 g) obtained by the method described in Tetrahedron Letters, vol.30, pp.1197-1200 published in 1989 was added a 4N hydrochloric acid-ethyl acetate solution (15 ml), and the mixture was stirred at room temperature for 5 hours. The reaction mixture was concentrated under reduced pressure, and potassium carbonate (6.63 g), benzyl bromide (2.14 ml) and 1-methyl-2-pyrrolidone (30 ml) were added to the residue, and the mixture was stirred at 130°C for 15 hours. The reaction mixture was cooled to room temperature, water (100 ml) was added, and this was extracted with ethyl acetate (50 ml × 3). The extract was washed with an aqueous saturated sodium chloride solution (100 ml), and dried with anhydrous magnesium sulfate, and the solvent was distilled off. The residue was purified by silica gel column chromatography (ethyl acetate/n-hexane = 15/85) to obtain colorless oily 8-benzyl-7-methyl-1,4-dioxa-8-azaspiro[4.5]decane (2.47 g, 83%).
¹H-NMR (CDCl₃) δ: 1.20 (3H, d, J = 6.2Hz), 1.56-1.78 (4H, m), 2.11-2.44 (1H, m), 2.45-2.62 (1H, m), 2.71-2.80 (1H, m), 3.14 (1H, d, J = 13.2Hz), 3.91-4.05 (4H, m), 4.08 (1H, d, J = 13.2Hz), 7.22-7.36 (5H, m).

### Step 2

To a solution of 8-benzyl-7-methyl-1,4-dioxa-8-azaspiro[4.5]decane (2.47 g) in benzene (45 ml) was added a 4 N hydrochloric acid (15 ml), and the mixture was heated to reflux at room temperature for 24 hours. The reaction mixture was cooled to room temperature, and benzene was distilled off under reduced pressure. To the residue was added water (50 ml), this was neutralized with sodium bicarbonate, and extracted with ethyl acetate (50 ml × 3). The extract was washed with an aqueous saturated sodium chloride solution (100 ml), and dried with anhydrous magnesium sulfate, and the solvent was distilled off to obtain pale yellow oily 1-benzyl-2-methylpiperidine-4-one (2.01 g, 99%).
¹H-NMR (CDCl₃) δ: 1.18 (3H, d, J = 6.6Hz), 2.23-2.41 (3H, m), 2.49-2.61 (2H, m), 2.49-3.08 (2H, m), 3.45 (1H, d, J = 13.6Hz), 3.97 (1H, d, J = 13.6Hz), 7.23-7.40 (5H, m).

### Step 3

To a solution of 1-benzyl-2-methyl-piperidine-4-one (2.01 g) and aniline (1.02 g) in acetic acid (10 ml) was added dropwise 96% trimethylsilylnitrile (1.02 g) under ice-cooling for 10 minutes, and the mixture was stirred at 0°C for 30 minutes. The reaction mixture was poured into ice (50 g)-28% aqueous ammonia (50 g), 28% aqueous ammonia was added until a pH became 10, and this was extracted with chloroform (50 ml × 3). The extract was washed with an aqueous saturated sodium chloride solution (100 ml), and dried with anhydrous magnesium sulfate, and the solvent was distilled off. The residue was purified by silica gel column chromatography (ethyl acetate/n-hexane = 1/2), and crystallized from diethyl ether to obtain 1-benzyl-2-methyl-4-(phenylamino)piperidine-4-carbonitrile (1.77 g, 59%) as colorless crystals.
¹H-NMR (CDCl₃) δ: 1.27 (3H, d, J = 5.8Hz), 1.56-1.78 (2H, m), 2.23-2.43 (3H, m), 2.65-2.75 (1H, m), 2.82-2.92 (1H, m), 3.14 (1H, d, J = 13.4Hz), 3.63 (1H, br s), 4.18 (1H, d, J = 13.4Hz), 6.88-6.96 (3H, m), 7.21-7.33 (7H, m).

### Step 4

A 1.6 M n-butyllithium hexane solution (6.9 ml) was added dropwise to a tetrahydrofuran (20 ml) solution of 4-methylthiazole (1.09 g) cooled to -78°C, and the mixture was stirred for 15 minutes. After a temperature was raised to -30°C, a solution of 1-benzyl-2-methyl-4-(phenylamino)piperidine-4-carbonitrile (1.67 g) in tetrahydrofuran (10 ml) was added, and the mixture was stirred for 30 minutes, and further stirred at 0°C for 15 minutes. To the reaction mixture were added water (50 ml) and ethyl acetate (50 ml), and the layers were separated. The organic layer was washed with an aqueous saturated sodium chloride solution (50 ml), and dried with anhydrous magnesium sulfate, and the solvent was distilled off. The residue was purified by silica gel column chromatography (ethyl acetate/n-hexane = 1/9), and crystallized from hexane to obtain colorless crystalline [1-benzyl-2-methyl-4-(4-methylthiazol-2-yl)-4-piperidinyl]-phenyl-amine as two kinds of diastereomers (isomer A and isomer B). Isomer A (low polarity): 0.43 g(21%), colorless crystal
¹H-NMR (CDCl₃) δ: 1.19 (3H, d, J = 6.2Hz), 2.08-2.24 (4H, m), 2.33 (1H, dd, J = 13.4 and 3.4Hz), 2.45 (3H, d, J = 1.0Hz), 2.49-2.73 (2H, m), 3.06 (1H, d, J = 13.6Hz), 4.16 (1H, d, J = 13.6Hz), 4.29 (1H, br s), 6.42-6.46 (2H, m), 6.66-6.73 (1H, m), 6.79 (1H, d, J = 1.0Hz), 7.03-7.11 (2H, m), 7.21-7.35 (5H, m).
Isomer B (high polarity): 1.35 g(65%), colorless crystal ¹H-NMR (CDCl₃) δ: 1.20 (3H, d, J = 6.4Hz), 1.76-2.01 (2H, m), 2.28-2.42 (2H, m), 2.44 (3H, d, J = 1.0Hz), 2.64-2.78 (2H, m), 2.87-2.96 (1H, m), 3.15 (1H, d, J = 13.2Hz), 4.05 (1H, d, J = 13.2Hz), 4.09 (1H, br s), 6.44-6.48 (2H, m), 6.66-6.74 (1H, m), 6.80 (1H, d, J = 1.0Hz), 7.00-7.08 (2H, m), 7.20-7.34 (5H, m).

### Step 5

To a solution of [1-benzyl-2-methyl-4-(4-methylthiazol-2-yl)-4-piperidinyl]-phenyl-amine (isomer A, 151 mg) in chloroform (5 ml) was added acetyl chloride (157 mg), and the mixture was heated to reflux for 36 hours. The reaction mixture was cooled to room temperature, and concentrated under reduced pressure, to the residue was added an aqueous saturated sodium bicarbonate solution (10 ml), and this was extracted with ethyl acetate (10 ml × 3). The extract was washed with an aqueous saturated sodium chloride solution (20 ml), and dried with anhydrous magnesium sulfate, and the solvent was distilled off. The residue was purified by basic silica gel column chromatography (ethyl acetate/n-hexane = 1/4) to obtain the colorless oily title compound (isomer A', 166 mg, 99%).
¹H-NMR (CDCl₃) δ: 1.00 (3H, d, J = 5.8Hz), 1.64 (3H, s), 1.81 (1H, dd, J = 14.2 and 11.4Hz), 2.07-2.33 (3H, m), 2.42 (3H, d, J = 1.0Hz), 2.52-2.68 (2H, m), 2.84-2.90 (1H, m), 3.36 (1H, d, J = 13.6Hz), 3.93 (1H, d, J = 13.6Hz), 6.75 (1H, d, J = 1.0Hz), 7.21-7.37 (9H, m), 7.65-7.68 (1H, m).

Using [1-benzyl-2-methyl-4-(4-methylthiazol-2-yl)-4-piperidinyl]-phenyl-amine (isomer B, 302 mg), the similar procedure afforded the colorless oily title compound (isomer B', 204 mg, 61%).
¹H-NMR (CDCl₃) δ: 1.17 (3H, d, J = 5.8Hz), 1.61 (3H, s), 1.59-1.82 (2H, m), 1.94-2.08 (1H, m), 2.29-2.40 (1H, m), 2.43 (3H, d, J = 1.0Hz), 2.55 (1H, dt, J = 11.8 and 3.6Hz), 2.70-2.86 (1H, m), 2.92 (1H, d, J = 13.2Hz), 3.16 (1H, dt, J = 13.2 and 3.0Hz), 4.04 (1H, d, J = 13.2Hz), 6.83 (1H, d, J = 1.0Hz), 7.12-7.43 (10H, m).

### Reference Example 93

### N-[1-Benzyl-2-methyl-4-(4-methylthiazol-2-yl)-4-piperidinyl]-N-phenylacetamide fumarate (isomer A' and isomer B' fumarate)

To a solution of N-[1-benzyl-2-methyl-4-(4-methylthiazol-2-yl)-4-piperidinyl]-N-phenylacetamide (isomer A', 166 mg) in ethanol (4 ml) was added fumaric acid (46 mg), the mixture was dissolved by warming, and stirred at room temperature for 30 minutes. The reaction mixture was concentrated under reduced pressure, and the residue was crystallized from diisopropyl ether to obtain the colorless crystalline title compound (isomer A' fumarate, 156 mg, 73%).
¹H-NMR (DMSO-d₆) δ: 0.89 (3H, d, J = 5.8Hz), 1.55 (3H, s), 1.60-1.73 (1H, m), 2.00-2.80 (6H, m), 2.35 (3H, d, J = 1.0Hz), 3.23 (1H, d, J = 13.8Hz), 3.91 (1H, d, J = 13.8Hz), 6.62 (2H, s), 7.13 (1H, d, J = 1.0Hz), 7.23-7.50 (9H, m), 7.65-7.70 (1H, m), 12.40-13.60 (2H, br).

Using N-[1-benzyl-2-methyl-4-(4-methylthiazol-2-yl)-4-piperidinyl]-N-phenylacetamide (isomer B', 204 mg), the similar procedure afforded the colorless crystalline title compound (isomer B' fumarate, 218 mg, 83%).
¹H-NMR (DMSO-d₆) δ: 1.10 (3H, d, J = 6.0Hz), 1.50 (3H, s), 1.56-1.73 (2H, m), 2.02-2.22 (2H, m), 2.33 (3H, d, J = 1.0Hz), 2.40-2.60 (1H, m), 2.75-2.92 (2H, m), 3.01 (1H, d, J = 13.6Hz), 3.99 (1H, d, J = 13.6Hz), 6.61 (2H, s), 7.17 (1H, d, J = 1.0Hz), 7.18-7.30 (5H, m), 7.38-7.51 (5H, m), 12.50-13.50 (2H, br).

### Reference Example 94

### N-[1-Benzyl-2-methyl-4-(4-methylthiazol-2-yl)-4-piperidinyl]-2,2,2-trifluoro-N-phenylaectamide

To a solution of [1-benzyl-2-methyl-4-(4-methylthiazol-2-yl)-4-piperidinyl]-phenyl-amine (isomer B, 302 mg) in tetrahydrofuran (10 ml) was added trifluoroacetic anhydride (1.68 g), and the mixture was stirred at room temperature for 15 hours. The reaction mixture was concentrated under reduce pressure, to the residue was added an aqueous saturated sodium bicarbonate solution (10 ml), and the mixture was extracted with ethyl acetate (10 ml × 3). The extract was washed with an aqueous saturated sodium chloride solution (20 ml), and dried with anhydrous magnesium sulfate, and the solvent was distilled off. The residue was purified by basic silica gel column chromatography (ethyl acetate/n-hexane = 1/4) to obtain the pale yellow oily title compound (228 mg, 65%). ¹H-NMR (CDCl₃) δ: 1.19 (3H, d, J = 5.8Hz), 1.61-1.76 (1H, m), 1.78 (1H, dd, J = 13.6 and 11.8Hz), 1.93-2.06 (1H, m), 2.28-2.38 (1H, m), 2.45 (3H, d, J = 0.8Hz), 2.53-2.63 (1H, m), 2.70-2.80 (1H, m), 2.92 (1H, d, J = 13.2Hz), 3.02-3.12 (1H, m), 4.05 (1H, d, J = 13.2Hz), 6.89 (1H, d, J = 0.8Hz), 7.16-7.50 (10H, m).

### Reference Example 95

### N-[1-Benzyl-2-methyl-4-(4-methylthiazol-2-yl)-4-piperidinyl]-2,2,2-trifluoro-N-phenylacetamide fumarate

A solution of N-[1-benzyl-2-methyl-4-(4-methylthiazol-2-yl)-4-piperidinyl]-2,2,2-trifluoro-N-phenylacetamide (218 mg) in ethanol (3 ml) was added fumaric acid (58 mg), the mixture was dissolved by warming and stirred at room temperature for 30 minutes. The reaction mixture was concentrated under reduced pressure, and the residue was crystallized from diisopropyl ether to obtain the colorless crystalline title compound (206 mg, 70%).
¹H-NMR (DMSO-d₆) δ: 1.12 (3H, d, J = 5.6Hz), 1.58-1.83 (2H, m), 1.96-2.28 (2H, m), 2.37 (3H, s), 2.40-2.60 (1H, m), 2.68-2.85 (2H, m), 2.97 (1H, d, J = 13.2Hz), 3.98 (1H, d, J = 13.2Hz), 6.62 (2H, s), 7.19-7.26 (5H, m), 7.30 (1H, m), 7.52 (5H, s), 12.60-13.60 (2H, br).

### Reference Example 96

### N-[1-Benzyl-4-(4-methylthiazol-2-yl)-4-piperidinyl]-N-(2-methylphenyl)propionamide oxalate

To a solution of N-[1-benzyl-4-(4-methylthiazol-2-yl)-4-piperidinyl]-N-(2-methylphenyl)propionamide (0.419 g, 0.966 mmol) in a mixture of ethyl acetate (1 ml)/2-propanol (2 ml) was added oxalic acid (0.104 g, 1.16 mmol). After complete dissolution, a small amount of n-hexane was added to precipitate crystals, which was washed with an ethyl acetate/n-hexane mixed solvent, and dried to obtain the title compound (0.331 g, 66%).
IR (KBr) ν : 1665 cm⁻¹.

### Reference Example 97

### N-[1-Benzyl-4-(4-methylthiazol-2-yl)-4-piperidinyl]-N-(3-methylphenyl)propionamide oxalate

According to the same manner as that of Reference Example 96, from N-[1-benzyl-4-(4-methylthiazol-2-yl)-4-piperidinyl]-N-(3-methylphenyl)propionamide, the title compound (0.242 g, 67%) was obtained.
IR (KBr) ν : 1667 cm⁻¹.

### Reference Example 98

### N-[1-Benzyl-4-(4-methylthiazol-2-yl)-4-piperidinyl]-N-4-methylphenyl)propionamide oxalate

According to the same manner as that of Reference Example 96, from N-[1-benzyl-4-(4-methylthiazol-2-yl)-4-piperidinyl]-N-(4-methylphenyl)propionamide, the title compound (0.351 g, 79%) was obtained.
IR (KBr) ν : 1667 cm⁻¹.

### Reference Example 99

### N-[1-Benzyl-4-(4-methylthiazol-2-yl)piperidine-4-yl]-N-(3-chlorophenyl)propionamide oxalate

According to the same manner as that of Reference Example 96, from N-[1-benzyl-4-(4-methylthiazol-2-yl)-4-piperidinyl]-N-(3-chlorophenyl)propionamide, the title compound (0.335 g, 88%) was obtained.
IR (KBr) ν : 1672 cm⁻¹.

### Reference Example 100

### N-[1-Benzyl-4-(4-methylthiazol-2-yl)piperidin-4-yl]-N-(4-chlorophenyl)propionamide oxalate

According to the same manner as that of Reference Example 96, from N-[1-benzyl-4-(4-methyl-thiazol-2-yl)-4-piperidinyl]-N-(4-chlorophenyl)propionamide, the title compound (0.405 g, 85%) was obtained.
IR (KBr) ν : 1669 cm⁻¹.

### Reference Example 101

### N-[1-Benzyl-4-(4-methylthiazol-2-yl)-4-piperidinyl]-N-phenylacetamide fumarate

N-[1-benzyl-4-(4-methylthiazol-2-yl)-4-piperidinyl]-N-phenylacetamide (3.0 g, 7.40 mmol) was dissolved in isopropanol (5 ml), and a solution of fumaric acid (0.87 g, 7.50 mmol) in isopropanol (5 ml) was added. The mixture was concentrated under reduced pressure, ethyl ether was added, seed crystals were added, and this was stirred at room temperature for 2 hours. Precipitated crystals were washed with ethyl ether-isopropanol, and dried to obtain the title compound (2.5 g, 65%).
m.p. 146.4-146.5°C
¹H-NMR (CDCl₃) δ: 1.52 (3H, s), 2.01 (2H, m), 2.33-2.58 (6H, m), 2.36 (3H, s), 3.44 (2H, s), 6.61 (2H, s), 7.18 (1H, s), 7.23-7.32 (5H, m), 7.43-7.52 (5H, m).

### Reference Example 102

### 4-(Benzoyl-phenylamino)-1-benzylpiperidine-4-carboxylic acid benzyl ester oxalate

To a suspension of 1-benzyl-4-(phenylamino)piperidine-4-carboxylic acid sodium salt (1.00 g, 3.01 mmol) and benzoic anhydride (4.76 g, 21.06 mmol) in ethyl acetate (30 mL) was added triethylamine (0.90 g, 8.89 mmol), and the mixture was heated to reflux for 2 hours. To this mixture was added benzyl alcohol (4.0 mL, 38.65 mmol) at 70°C, and the mixture was heated to reflux for 15 hours. The resulting reaction mixture was returned to room temperature, diluted with water, and extracted with ethyl acetate. The extract was washed successively with water and an aqueous saturated sodium chloride solution, dried with magnesium sulfate, and concentrated under reduced pressure. The residue was purified by silica gel chromatography eluting with ethyl acetate-hexane (3:7 to 1:2), to obtain 4-(benzoyl-phenylamino)-1-benzylpiperidine-4-carboxylic acid benzyl ester (0.82 g, 54%) as a colorless amorphous substance.
¹H-NMR (CDCl₃) δ: 1.85 (2H, t, J = 11.9Hz), 2.40 (2H, d, J = 13.0Hz), 2.51 (2H, t, J = 11.7Hz), 2.65 (2H, d, J = 11.8Hz), 3.45 (2H, s), 5.27 (2H, s), 7.01-7.44 (20H, m).

To a solution of the resulting 4-(benzoyl-phenylamino)-1-benzylpiperidine-4-carboxylic acid benzyl ester (400 mg, 0.79 mmol) in isopropanol (2 mL) was added a solution of oxalic acid (99 mg, 0.79 mmol) in isopropanol (2 mL), and the mixture was concentrated. To the residue was added diethyl ether, and the resulting solid was filtered to obtain the title compound (382 mg) as a white powder.
¹H-NMR (DMSO-d₆) δ: 1.92 (2H, t, J = 10.2Hz), 2.33 (2H, d, J = 13.6Hz), 2.75-2.95 (2H, br), 2.95-3.10 (2H, br), 3.92 (2H, s), 5.23 (2H, s), 7.00 (2H, d, J = 8.1Hz), 7.07-7.20 (8H, m), 7.38-7.47 (10H, m).

| Example 1 | |
|---|---|
| (1) Compound of Reference Example 39 | 10.0 mg |
| (2) Lactose | 60.0 mg |
| (3) Corn starch | 35.0 mg |
| (4) Gelatin | 3.0 mg |
| (5) Magnesium stearate | 2.0 mg |

Using 0.03 ml of a 10% aqueous gelatin solution (3.0 mg as gelatin), a mixture of 10.0 mg of the compound of Reference Example 39, 60.0 mg of lactose and 35.0 mg of corn starch is granulated by passing through a 1 mm mesh sieve, and the resulting granules are dried at 40°C and passed through a sieve again. The thus obtained granules are mixed with 2.0 mg of magnesium stearate, and are compressed. The resulting core tables are coated with a sugar film formed from an aqueous suspension of sucrose, titanium dioxide, talc and gum arabic. The coated tablets are polished with beewax to obtain coated tablets.

| Example 2 | |
|---|---|
| (1) Compound of Reference Example 39 | 10.0 mg |
| (2) Lactose | 70.0 mg |
| (3) Corn starch | 50.0 mg |
| (4) Soluble starch | 7.0 mg |
| (5) Magnesium stearate | 3.0 mg |

Using 0.07 ml of an aqueous soluble starch solution (7.0 mg as soluble starch), 10.0 mg of the compound of Reference Example 39 and 3.0 mg of magnesium stearate are granulated, dried, and mixed with 70.0 mg of lactose and 50.0 mg of corn starch. The mixture is compressed to obtain tablets.

| Example 3 | |
|---|---|
| (1) Compound of Reference Example 39 | 5.0 mg |
| (2) Salt | 20.0 mg |
| (3) Distilled water | ad. 2 ml |

A solution of 5.0 mg of the compound of Reference Example 39 and 20.0 mg of salt in distilled water is prepared, and water is added thereto to make up the total amount to 2.0 ml. The solution is filtered, and filled in a 2 ml ample under aseptic conditions. The ample is sterilized, and sealed to obtain an injectable solution.

### Test Example 1

### Preparation of cell membrane fraction expressing human FM-3

To 1×10⁸ CHO/human FM3 cells were added 10 ml of a homogenizing buffer (50 mM Tris hydrochloride buffer, pH 7.5, 5 mM EDTA, 0.5 mM PMSF, 0.1 µg/ml PepstatinA, 4 µg/ml E-64, 20 µg/ml Leupeptin), and the mixture was ground using Polytron (12,000 rpm, 15 seconds × three times).

The cell ground mixture was centrifuged (1,000 g, 10 minutes) to obtain a supernatant. Then, the supernatant was ultracentrifuged (Beckman type 30 rotor, 30,000 rpm, 1 hour), and the resulting precipitate was used as a CHO cell fraction expressing human FM3.

### Test Example 2

### Preparation of cell membrane fraction expressing human TGR-1

To 1×10⁸ CHO/human TGR-1 cells were added 10 ml of a homogenizing buffer (50 mM Tris hydrochloride buffer, pH 7.5, 5 mM EDTA, 0.5 mM PMSF, 0.1 µg/ml PepstatinA, 4 µg/ml E-64, 20 µg/ml Leupeptin), and the mixture was ground using Polytron (12,000 rpm, 15 seconds × three times).

The cell ground mixture was centrifuged (1,000 g, 10 minutes) to obtain a supernatant. Then, the supernatant was ultracentrifuged (Beckman type 30 rotor, 30,000 rpm, 1 hour), the resulting precipitate was used as a CHO cell fraction expressing human TGR-1.

### Test Example 3

### Preparation of Neuromedin U-8 labeled with isotope

Neuromedin U-8 labeled with an isotope for using in a binding inhibiting experiment was prepared as follows: To a solution of 10 µl of 100 µM Neuromedin U-8 (Buchem) in distilled water was added 0.01 mg/ml of lactoperoxygenase (Sigma). After mixing, [¹²⁵I]NaI 37 MBq (Amersham Biosciences) was added thereto. Further, 10 µl of 0.005% H₂O₂ was added thereto and the reaction was performed for 10 minutes. After addition of 600 µL of 0.1%TFA, purification was performed by HPLC using TSKgel ODS-80Ts (100 mm × 4.6 mm I.D, Tosoh Corporation) to obtain labeled Neuromedin U-8.

### Test Example 4

### Binding inhibiting experiment of test compound using human FM-3 expressing cell membrane fraction and isotope-labeled Neuromedin U-8

Human FM-3 expressing CHO cell membrane fraction was diluted with a membrane diluting buffer (50 mM Tris hydrochloride buffer, pH 7.5, 5 mM EDTA, 0.5 mM PMSF, 0.1 µg/ml Pepstatin, 20 µg/ml Leupeptin, 4 µg/ml E-64) to prepare a cell membrane fraction solution for an assay having a protein concentration of 20 µg/ml. Each 25 µl of a membrane fraction solution for an assay was dispensed in a 96-well microtiter plate, and 25 µl of a membrane diluting buffer containing 400 pM [¹²⁵I]-labeled Neuromedin U-8 and 50 µl of a solution in which dimethyl sulfoxide was diluted 100 volume-fold with a membrane diluting buffer for investigating total binding, 25 µl of a membrane diluting buffer containing 400 pM [¹²⁵I]-labeled Neuromedin U-8 and 50 µl of a 10% dimethyl sulfoxide-containing membrane diluting buffer containing 20 µM non-isotope-labeled Neuromedin U-8 for investigating non-specific binding, and 50 µl of a solution in which a solution of a test compound in dimethyl sulfoxide was diluted 100 volume-fold with a membrane diluting buffer and 25 µl of a membrane diluting buffer containing 400 pM [¹²⁵I]-labeled Neuromedin U-8 for investigating binding inhibiting activity of a test compound were added, respectively, to react them at 25°C for 1.5 hours. The mixture was filtered with a filter plate (GF/C, Whatmann) and the filter was washed with a washing buffer (50 mM Tris hydrochloride buffer, pH 7.5) six times, 20 µl of Microscinti 20 (Perkin Elmer Lifescience) was added, and radioactivity was measured with Topcount (Perkin Elmer Lifescience). Specific binding is a value obtained by subtracting non-specific binding from total binding. Human FM-3 binding inhibiting activity of a test compound is indicated by a ratio of a value obtained by subtracting radioactivity of a cell membrane fraction with a test compound added from total binding relative to specific binding.

### Test Example 5

### Binding inhibiting experiment of test compound using human TGR-1 expressing cell membrane fraction and isotope-labeled Neuromedin U-8

Human TGR-1 expressing CHO cell membrane fraction was diluted with a membrane diluting buffer (50 mM Tris hydrochloride buffer, pH 7.5, 5 mM EDTA, 0.5 mM PMSF, 0.1 µg/ml Pepstatin, 20 µg/ml Leupeptin, 4 µg/ml E-64) to prepare a cell membrane fraction solution for an assay having a protein concentration of 20 µg/ml. Each 25 µl of a membrane fraction solution for an assay was dispensed in a 96-well microtiter plate, and 25 µl of a membrane diluting buffer containing 400 pM [¹²⁵I]-labeled Neuromedin U-8 and 50 µl of a solution in which dimethyl sulfoxide was diluted 100 volume-fold with a membrane diluting buffer for investigating total binding, 25 µl of a membrane diluting buffer containing 400 pM [¹²⁵I]-labeled Neuromedin U-8 and 50 µl of a 1% dimethyl sulfoxide-containing membrane diluting buffer containing 20 µM non-isotope-labeled Neuromedin U-8 for investigating non-specific binding, and 50 µl of a solution in which a solution of a test compound in dimethyl sulfoxodie was diluted 100 volume-fold with a membrane diluting buffer and 25 µl of a membrane diluting buffer containing 400 pM [¹²⁵I]-labeled Neuromedin U-8 for investigating binding inhibiting activity of a test compound were added, respectively, to react them at 25°C for 1.5 hours. The mixture was filtered with a filter plate (GF/C, Whatmann) and the filter was washed with a washing buffer (50 mM Tris hydrochloride buffer, pH 7.5) six times, 20 µl of Microscinti 20 (Perkin Elmer Lifescience) was added, and radioactivity was measured with Topcount (Perkin Elmer Lifescience). Specific binding is a value obtained by subtracting non-specific binding from total binding. Human TGR-1 binding inhibiting activity of a test compound is indicated by a ratio of a value obtained by subtracting radioactivity of a cell membrane fraction with a test compound added from total binding relative to specific binding.

### Teat Example 6

### Change in intracellular calcium concentration of test compound on human FM-3 expressing CHO cell

FM-3 expressing CHO cell was seeded in a 96-well plate at 1×10⁴ cell/well, and cultured for 48 hours and, then, 50 µl of an assay buffer containing 4 µM Fluo3, 0.04% Pluronic acid, 2.5 mM probenicid (calcium screening kit, typeC, Dojin Chemical Institute) was added to react them at 37°C for 1 hour. When agonist action was measured, 25 µl of a solution in which a solution of a test compound in dimethyl sulfoxide was diluted 250 volume-fold with an assay buffer was further added and, when antagonist action was measured, 25 µl of 4 nM Neuromedin U-8 was further added, and a change in an intracelullar calcium concentration was measured with FLIPR (Nippon Molecular Device).

### Test Example 7

### Change in intracellular calcium concentration of test compound on human TGR-1 expressing CHO cell

TGR-1 expressing CHO cell was seeded in a 96-well plate at 1×10⁴ cell/well, and cultured for 48 hours and, then, 50 µl of an assay buffer containing 4 µM Fluo3, 0.04% Pluronic acid, 2.5 mM probenicid (calcium screening kit, typeC, Dojin Chemical Institute) was added to react them at 37°C for 1 hour. When agonist action was measured, 25 µl of a solution in which a solution of a test compound in dimethyl sulfoxide was diluted 250 volume-fold with an assay buffer was further added and, when antagonist action was measured, 25 µl of 4nM Neuromedin U-8 was further added, and a change in an intracelullar calcium concentration was measured with FLIPR (Nippon Molecular Device).

### Test Results

Table 1 shows action of a test compound of inhibiting binding to a human neuromedin U receptor FM-3 using Neuromedin U-8.

**Table 1**

| Test compound (Reference Example No.) | FM-3 IC₅₀ (µM) |
|---|---|
| 2 | 1.2 |
| 5 | 0.12 |
| 17 | 0.082 |
| 18 | 0.52 |
| 24 | 0.44 |
| 37 | 0.041 |
| 39 | 0.024 |
| 41 | 0.15 |
| 45 | 0.067 |
| 48 | 0.077 |
| 51 | 0.52 |

Thereby, it has been found that the compound of the present invention or a salt thereof or a prodrug thereof has excellent human neuromedin U receptor FM-3 binding inhibiting activity.

In addition, the compound of Reference Example 39 inhibited increase in an intracellular calcium concentration due to Neuromedin U-8 in FM-3 expressing CHO cell. Thereby, it has been found that the compound of Reference Example 39 has human neuromedin U receptor FM-3 antagonist activity.

### Industrial Applicability

The compound of the present invention has excellent neuromedin U receptor function regulating action, and can be used as a preventive or therapeutic agent for hypertension, cardiac infarct, acute renal dysfunction, stress disease (e.g. (i) cardiovascular disease (angina, cardiac infarct, arrhythmia etc.), (ii) respiratory disease (bronchial asthma, hyperpnea syndrome etc.), (iii) muscle skeleton disease (rheumatoid arthritis, lumbago, migraine, tonic headache etc.), (iv) others (diabetes, climacteric disorder, immune activity reduction etc.), digestive disease (stomach ulcer, ulcerative colitis et.).

## Claims

1. A composition for regulatingneuromedin U receptor, which comprises a compound having a partial structure represented by the formula: 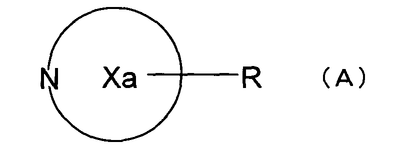 wherein ring Xa represents a nitrogen-containingnitrogen-containing ring, and R represents an optionally substituted amino group, or a salt thereof.

2. The composition according to claim 1, which comprises a compound having an aminopiperidine skeleton or a salt thereof.

3. The composition according to claim 1, which comprises a compound represented by the formula: 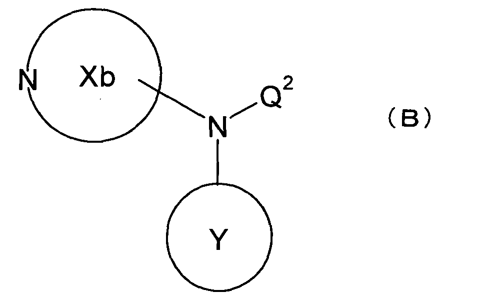 wherein ring Xb represents an optionally further substituted 5 to 8-membered nitrogen-containing ring, Y represents an optionally substituted ring group, and Q² represents an acyl group, or a salt thereof or a prodrug thereof.

4. The composition according to claim 1, which comprises a compound represented by the formula: 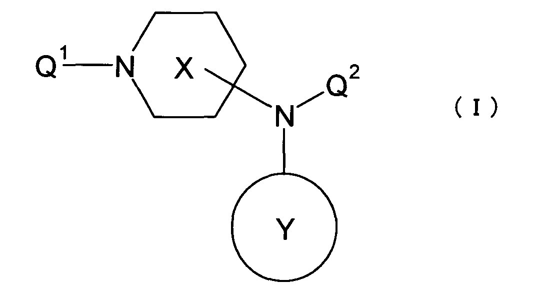 wherein ring X represents an optionally further substituted piperidine ring, Y represents an optionally substituted ring group, Q¹ represents a hydrogen atom or a substituent, and Q² represents an acyl group, or a salt thereof or a prodrug thereof.

5. The composition according to claim 1, which comprises a compound represented by the formula: 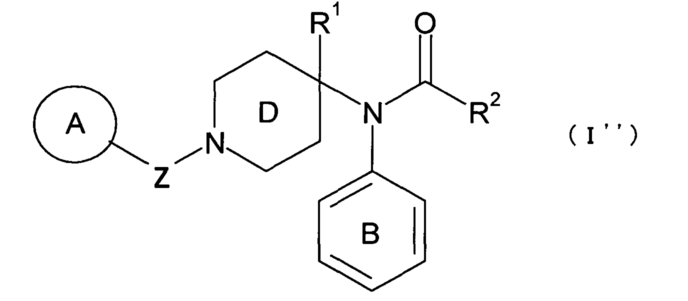 wherein A represents an optionally substituted ring group, B represents an optionally substituted phenyl group, ring D represents an optionally further substituted piperidine ring, Z represents an optionally substituted methylene group, -COCH₂-, -CH₂CO- or -SO₂-, R¹ represents a hydrogen atom, a cyano group, an optionally substituted lower alkyl group, an optionally substituted phenyl group, an optionally substituted aromatic heterocyclic group, an optionally esterified carboxyl group or an optionally substituted carbamoyl group, and R² represents an optionally substituted lower alkyl group, an optionally substituted lower alkenyl group, an optionally substituted lower alkoxy group, an optionally substituted aralkyloxy group or an optionally substituted phenyl group, or a salt thereof or a prodrug thereof.

6. The composition according to claim 1, which is an antagonist of a neuromedin U receptor FM-3.

7. The composition according to claim 1, which is a composition for regulating physiological function in which neuromedin U is involved, or a preventive/therapeutic agent for morbid state or disease in which neuromedin U in involved.

8. The composition according to claim 1, which is a preventive/therapeutic agent for hypertension, cardiac infarct, acute renal dysfunction, angina, cardiac infarct, arrhythmia, bronchial asthma, hyperpnea syndrome, rheumatoid arthritis, diabetes, climacteric disorder, immune activity reduction, stomach ulcer or ulcerative colitis, or a composition for regulating an appetite.

9. A method of regulating function of a neuromedin U receptor, which comprises administering an effective amount of a compound having a partial structure represented by the formula: 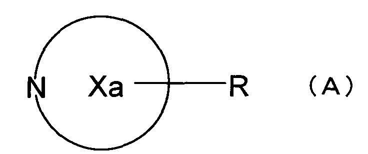 wherein ring Xa represents a nitrogen-containing ring, and R represents an optionally substituted amino group, or a salt thereof to a mammal.

10. Use of a compound having a partial structure represented by the formula: 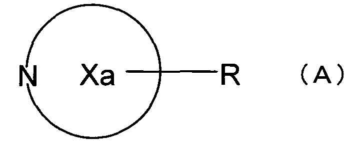 wherein ring Xa represents a nitrogen-containing ring, and R represents an optionally substituted amino group, or a salt thereof for preparing a composition for regulatingneuromedin U receptor.

11. A compound represented by the formula: 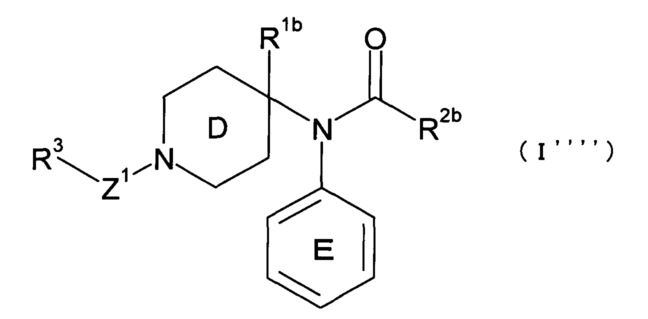 wherein ring D represents an optionally further substituted piperidine ring, E represents an optionally substituted phenyl group, Z¹ represents a methylene group optionally substituted with a substituent selected from the group consisting of lower alkyl, lower alkoxycarbonyl, oxo and phenyl, -COCH₂-, -CH₂CO- or -SO₂-, R^{1b} represents an optionally substituted 2-thiazolyl group, an optionally substituted 2-imidazolyl group or an optionally substituted 2-pyridyl group, R^{2b} represents an optionally halogenated lower alkyl group, and R³ represents an optionally substituted phenyl group, an optionally substituted aromatic heterocyclic group or an optionally substituted cycloalkyl group, provided that N-[1-benzyl-4-(4-methylthiazol-2-yl)-4-piperidinyl]-N-phenylpropionamide and N-[1-benzyl-4-(2-pyridinyl)-4-piperidinyl]-N-phenylpropionamide are excluded, or a salt thereof.

12. The compound according to claim 11, wherein R³ is an optionally substituted phenyl group or an optionally substituted thienyl group.

13. The compound according to claim 11, wherein R³ is a phenyl group.

14. The compound according to claim 11, wherein E is a phenyl group optionally having a substituent at an ortho position or a meta position.

15. The compound according to claim 11, wherein E is an unsubstituted phenyl group.

16. The compound according to claim 11, wherein R^{1b} is a 2-thiazolyl group optionally substituted with a lower alkyl group.

17. The compound according to claim 11, wherein R^{1b} is a 4-methyl-2-thiazolyl group.

18. The compound according to claim 11, wherein R^{1b} is a 2-pyridyl group optionally substituted with a substituent selected from the group consisting of a lower alkyl group, a lower alkylthio group, a halogen atom, a C₆₋₁₄ aryl group and an aromatic heterocyclic group.

19. The compound according to claim 11, wherein R^{1b} is a 6-methyl-2-pyridyl group.

20. The compound according to claim 11, wherein Z¹ is a methylene group optionally substituted with a lower alkyl group.

21. The compound according to claim 11, wherein Z¹ is a methylene group.

22. The compound according to claim 11, wherein R^{2b} is an optionally halogenated methyl group or ethyl group.

23. The compound according to claim 11, wherein R^{2b} is a methyl group or a trifluoromethyl group.

24. The compound according to claim 11, wherein ring D is a piperidine ring optionally further substituted with a lower alkyl.

25. The compound according to claim 11, wherein ring D is a piperidine ring optionally further substituted with C₁₋₆ alkyl, E is a phenyl group optionally substituted with a substituent selected from the group consisting of a halogen atom and C₁₋₆ alkyl, Z¹ is a methylene group optionally substituted with a substituent selected from the group consisting of C₁₋₆ alkyl, C₁₋₆ alkoxycarbonyl, oxo and phenyl, -COCH₂- or -SO₂-, R^{1b} is (i) a 2-thiazolyl group optionally substituted with C₁₋₆ alkyl, (ii) a 2-imidazolyl group optionally substituted with C₁₋₆ alkyl, or (iii) a 2-pyridyl group optionally substituted with a substituent selected from the group consisting of C₁₋₆ alkyl, a halogen atom, C₁₋₆ alkylthio, phenyl and thienyl, R^{2b} is an optionally halogenated C₁₋₆ alkyl group, R³ is (i) a C₃₋₈ cycloalkyl group, (ii) a phenyl group or (iii) a 5- to 10-membered aromatic heterocyclic group containing one or two kinds of 1 to 4 hetero atoms selected from a nitrogen atom, a sulfur atom and an oxygen atom in addition to carbon atoms, which may be substituted with a substituent selected from the group consisting of a halogen atom, cyano, C₁₋₆ alkyl optionally substituted with a halogen atom, C₁₋₆ alkoxy optionally substituted with a halogen atom, C₁₋₆ alkyl-carbonylamino, a 5- or 6-membered aromatic heterocyclic group and C₁₋₆ alkylthio.

26. N-[1-benzyl-4-(4-methylthiazol-2-yl)-4-piperidinyl]-N-phenylacetamide, N-[1-benzyl-4-(4-methylthiazol-2-yl)-4-piperidinyl]-2,2,2-trifluoro-N-phenylacetamide, N-[1-benzyl-4-(6-methyl-2-pyridinyl)-9-piperidinyl]-N-phenylacetamide, N-[1-benzyl-4-(6-methyl-2-pyridinyl)-4-piperidinyl]-2,2,2-trifluoro-N-phenylacetamide, N-[1-(4-fluorobenzyl)-4-(4-methylthiazol-2-yl)-4-piperidinyl]-N-phenylacetamide, N-[1-benzyl-4-(4-methylthiazol-2-yl)-4-piperidinyl]-N-(2-methylphenyl)acetamide, N-[1-benzyl-4-(4-methylthiazol-2-yl)-4-piperidinyl]-N-(3-chlorophenyl)acetamide, N-[4-(4-methylthiazol-1-(2-thienylmethyl)-2-yl)-4-piperidinyl]-N-phenylacetamide, N-[1-benzyl-4-(1-methyl-1H-imidazol-2-yl)-4-piperidinyl]-N-phenylacetamide, or a salt thereof.

27. A prodrug of the compound according to claim 11 or 26 or a salt thereof.

28. A medicine comprising the compound according to claim 11 or 26 or a salt thereof or a prodrug thereof.
